(19) Europäisches Patentamt
European Patent Office
Office européen des brevets

(11) EP 4 019 023 A1

(12) **EUROPEAN PATENT APPLICATION**

(43) Date of publication:
**29.06.2022 Bulletin 2022/26**

(21) Application number: **22156237.4**

(22) Date of filing: **03.08.2017**

(51) International Patent Classification (IPC):
***A61K 31/675*** *(2006.01)* ***A61K 31/5377*** *(2006.01)*
***A61K 38/38*** *(2006.01)* ***A61K 47/42*** *(2017.01)*
*A61K 9/08* *(2006.01)* *A61K 9/19* *(2006.01)*

(52) Cooperative Patent Classification (CPC):
(C-Sets available)
**A61K 38/385; A61K 9/0019; A61K 9/08; A61K 9/19; A61K 31/5377; A61K 31/675; A61K 47/42** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2016 US 201662370453 P**
**24.10.2016 US 201662412085 P**

(62) Document number(s) of the earlier application(s) in accordance with Art. 76 EPC:
**17837680.2 / 3 493 815**

(71) Applicant: **Zhuhai Beihai Biotech Co., Ltd.**
**Zhuhai (CN)**

(72) Inventor: **SUN, Qun**
**Princeton, 08540 (US)**

(74) Representative: **Fish & Richardson P.C.**
**Highlight Business Towers**
**Mies-van-der-Rohe-Straße 8**
**80807 München (DE)**

Remarks:
This application was filed on 11-02-2022 as a divisional application to the application mentioned under INID code 62.

# (54) FORMULATIONS OF FOSAPREPITANT AND APREPITANT

(57) This document relates to a composition comprising fosaprepitant, or a pharmaceutically acceptable salt thereof, and human serum albumin, wherein the fosaprepitant, or a pharmaceutically acceptable salt thereof, and the human serum albumin in the composition have a ratio by weight from about 1:0.1 to about 1:500. This document also relates to a composition comprising aprepitant and human serum albumin, wherein the aprepitant and the human serum albumin in the composition have a ratio by weight from about 1:80 to about 1:1000. This document also relates to a composition comprising fosaprepitant, or a pharmaceutically acceptable salt thereof, aprepitant and human serum albumin.

EP 4 019 023 A1

(52) Cooperative Patent Classification (CPC): (Cont.)

C-Sets
**A61K 31/5377, A61K 2300/00;**
**A61K 31/675, A61K 2300/00;**
**A61K 38/385, A61K 2300/00**

## Description

### CLAIM OF PRIORITY

This application claims the benefit of U.S. provisional application No. 62/370,453 filed August 3, 2016 and U.S. provisional application No. 62/412,085 filed October 24, 2016. The entire contents of the foregoing are hereby incorporated by reference.

### TECHNICAL FIELD

This document relates to compositions and formulations for the treatment and prevention of chemotherapy-induced nausea and vomiting, and more particularly to compositions comprising fosaprepitant, or a pharmaceutically acceptable salt thereof, compositions comprising aprepitant, and compositions comprising fosaprepitant, or a pharmaceutically acceptable salt thereof, and aprepitant.

### BACKGROUND

Nausea and vomiting are common complications of cancer chemotherapy. Patients consistently report that chemotherapy-induced nausea and vomiting (CINV) is an aspect of treatment they find most unpleasant and distressing. This syndrome has a significant impact on patients' functional status and quality of life and patients may delay scheduled chemotherapy or even on occasion refuse potentially curative therapy because of CINV.

Aprepitant is a selective high-affinity antagonist at human substance P neurokinin 1 (NK1) receptors used for the prevention of CINV. Despite the demonstrated benefits of oral aprepitant, there is still a medical need for treatment options (such as intravenous administration) to prevent CINV in patients who cannot easily tolerate orally administered medication prior to initiating chemotherapy. Parenteral administration is also an important treatment option for oncologists for whom it is frequently more convenient and easier to administer compounds intravenously prior to the administration of chemotherapy (which is also commonly given intravenously).

Fosaprepitant is a water soluble phosphoryl prodrug to aprepitant. Following intravenous administration, fosaprepitant is rapidly converted to aprepitant *in vivo* via ubiquitous phosphatases. The antiemetic effects of fosaprepitant are attributed to aprepitant.

Aprepitant is insoluble in water whereas fosaprepitant is soluble in water. Fosaprepitant easily degrades to aprepitant unless stored at low temperature. Degradation is enhanced by the presence of water. To solubilise possibly present aprepitant, current fosaprepitant formulation contains polysorbate 80. Polysorbate 80 may cause hypersensitivity reactions, including flushing, itching or shortness of breath, and has the potential to cause severe anaphylaxis reactions, and cause infusion site reactions (Leal AD et al., Support Care Cancer 2014; 22:1313-1317).

There is a need for the fosaprepitant or aprepitant formulations containing no polysorbate 80 that are suitable for parenteral administration.

### SUMMARY

Provided herein is a composition comprising fosaprepitant, or a pharmaceutically acceptable salt thereof, and human serum albumin, wherein the fosaprepitant, or a pharmaceutically acceptable salt thereof, and the human serum albumin in the composition have a ratio by weight from about 1:0.1 to about 1:500.

In some embodiments, the fosaprepitant, or a pharmaceutically acceptable salt thereof, and the human serum albumin in the composition have a ratio by weight from about 1:0.5 to about 1:300, from about 1:0.8 to about 1:200, from about 1:1 to about 1:150, from about 1:2 to about 1:100, from about 1:2 to about 1:80, from about 1:2 to about 1:50, from about 1:2 to about 1:40, from about 1:2.5 to about 1:30, or from about 1:3 to about 1:20.. In some embodiments, the fosaprepitant, or a pharmaceutically acceptable salt thereof, and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the fosaprepitant can be fosaprepitant dimeglumine, which is a dimeglumine salt of fosaprepitant.

In some embodiments, the human serum albumin is a native human serum albumin. In some embodiments, the human serum albumin is a recombinant human serum albumin. In some embodiments, the human serum albumin is essentially fatty acid free.

In some embodiments, the composition comprises at least one stabilizer for the human serum albumin. In some embodiments, the composition comprises two stabilizers for the human serum albumin. In some embodiments, the

stabilizers are N-acetyltryptophanate and caprylic acid or sodium salt thereof. In some embodiments, the stabilizer is N-acetyltryptophanate or sodium salt thereof. In some embodiments, the stabilizer is caprylic acid or sodium salt thereof.

In some embodiments, the composition is a solid formulation. For example, the solid formulation can be produced in a uniform manner by lyophilization. A skilled artisan would recognize other methods, such as rotary evaporation, that can also produce solid formulations.

In some embodiments, the composition is an aqueous formulation. In some embodiments, the aqueous formulation is substantially free of solvent other than water. In some embodiments, the aqueous formulation is free of solvent other than water.

In some embodiments, the aqueous formulation is a clear aqueous solution. In some embodiments, the solution remains clear for at least about 1 hours, 2 hours, 3 hours, 4 hours, 5 hours, 6 hours, 8 hours, 12 hours, or 24 hours.

Also, provided herein is a pharmaceutical composition comprising the composition comprising the fosaprepitant, or a pharmaceutically acceptable salt thereof, and the human serum albumin as described herein, and a pharmaceutically acceptable carrier.

In some embodiments, the pharmaceutical composition is free of a surfactant, such as CREMOPHOR® surfactants and Polysorbate 80.

Also, provided herein is a method for the prevention of chemotherapy-induced nausea and vomiting, the method comprising the step of administering to a subject in need thereof of a therapeutically effective amount of a pharmaceutical composition comprising the composition comprising the fosaprepitant, or a pharmaceutically acceptable salt thereof, and the human serum albumin as described herein, and a pharmaceutically acceptable carrier.

In some embodiments, the methods described herein are for the prevention of acute and delayed nausea and vomiting associated with initial and repeat courses of highly emetogenic cancer chemotherapy (HEC) including high-dose cisplatin. In some embodiments, the methods described herein are for the prevention of delayed nausea and vomiting associated with initial and repeat courses of moderately emetogenic cancer chemotherapy (MEC).

Also, provided herein is a kit comprising a solid composition comprising the fosaprepitant, or a pharmaceutically acceptable salt thereof, and an aqueous solution of human serum albumin.

Also, provided herein is a composition comprising aprepitant and human serum albumin, wherein the aprepitant and the human serum albumin in the composition have a ratio by weight from about 1:80 to about 1:1000.

In some embodiments, the aprepitant and the human serum albumin in the composition have a ratio by weight from about 1:100 to about 1:800. In some embodiments, the aprepitant and the human serum albumin in the composition have a ratio by weight from about 1:120 to about 1:600. In some embodiments, the aprepitant and the human serum albumin in the composition have a ratio by weight from about 1:130 to about 1:400. In some embodiments, the aprepitant and the human serum albumin in the composition have a ratio by weight from about 1:140 to about 1:300. In some embodiments, the aprepitant and the human serum albumin in the composition have a ratio by weight from about 1:150 to about 1:280. In some embodiments, the aprepitant and the human serum albumin in the composition have a ratio by weight from about 1:160 to about 1:260. In some embodiments, the aprepitant and the human serum albumin in the composition have a ratio by weight from about 1:180 to about 1:250. In some embodiments, the aprepitant and the human serum albumin in the composition have a ratio by weight from about 1:200 to about 1:250. In some embodiments, the aprepitant and the human serum albumin in the composition have a ratio by weight of about 1:120, about 1:130, about 1:140, about 1:150, about 1:160, about 1:170, about 1:180, about 1:190, about 1:200, about 1:210, about 1:220, about 1:230, about 1:240, about 1:250, about 1:260, about 1:270, about 1:280, about 1:290, about 1:300, about 1:310, about 1:320, about 1:330, about 1:340, about 350, .or about 1:400

In some embodiments, the human serum albumin is a native human serum albumin.

In some embodiments, the human serum albumin is a recombinant human serum albumin. In some embodiments, the human serum albumin is essentially fatty acid free.

In some embodiments, the composition is a solid formulation. For example, the solid formulation can be produced in a uniform manner by lyophilization. A skilled artisan would recognize other methods, such as rotary evaporation, that can also produce solid formulations.

In some embodiments, the composition is an aqueous formulation. In some embodiments, the aqueous formulation is substantially free of solvent other than water. In some embodiments, the aqueous formulation is free of solvent other than water.

In some embodiments, the aqueous formulation is a clear aqueous solution. In some embodiments, the aqueous formulation is a clear aqueous solution for at least 1 hour. In some embodiments, the aqueous formulation is a clear aqueous solution for at least 2 hours. In some embodiments, the aqueous formulation is a clear aqueous solution for at least 3 hours. In some embodiments, the aqueous formulation is a clear aqueous solution for at least 4 hours. In some embodiments, the aqueous formulation is a clear aqueous solution for at least 5 hours. In some embodiments, the aqueous formulation is a clear aqueous solution for at least 6 hours. In some embodiments, the aqueous formulation is a clear aqueous solution for at least 8 hours. In some embodiments, the aqueous formulation is a clear aqueous solution for at least 24 hours.

Also, provided herein is a pharmaceutical composition comprising the composition comprising the aprepitant and the human serum albumin as described herein, and a pharmaceutically acceptable carrier.

In some embodiments, the pharmaceutical composition is free of a surfactant, such as CREMOPHOR® surfactants and Polysorbate 80.

Also, provided herein is a method for the prevention of chemotherapy-induced nausea and vomiting, the method comprising the step of administering to a subject in need thereof of a therapeutically effective amount of a pharmaceutical composition comprising the composition comprising the aprepitant and the human serum albumin as described herein, and a pharmaceutically acceptable carrier.

Also, provided herein is a composition comprising fosaprepitant, or a pharmaceutically acceptable salt thereof, aprepitant and human serum albumin. In some embodiments, the fosaprepitant, or a pharmaceutically acceptable salt thereof, and the human serum albumin in the composition have a ratio by weight from about 1:0.1 to about 1:500.

In some embodiments, the aprepitant and the fosaprepitant, or a pharmaceutically acceptable salt thereof, in the composition have a ratio by weight no more than 1:10, no more than 1:20, no more than 1:25, no more than 3:100, no more than 1:50, no more than 1:100, no more than 1:200, no more than 1:500, or no more than 1:1000. In some embodiments, the weight ratio of fosaprepitant, or a pharmaceutically acceptable salt thereof, to aprepitant in the composition is from about 1000:1 to about 20:1, from about 1000:1 to about 50:1, from about 1000:1 to about 70:1, or from about 1000:1 to about 100:1.

In some embodiments, the molar ratio of fosaprepitant, or a pharmaceutically acceptable salt thereof, to aprepitant in the composition is from about 1000:1 to about 10:1, from about 1000 to about 20:1, from about 1000:1 to about 50:1, or from about 1000:1 to about 100:1.

In some embodiments, the fosaprepitant, or the pharmaceutically acceptable salt thereof, and the human serum albumin in the composition have a ratio by weight from about 1:0.5 to about 1:300, from about 1:0.8 to about 1:200, from about 1:1 to about 1:150, from about 1:2 to about 1:100, from about 1:2 to about 1:80, from about 1:2 to about 1:50, from about 1:2.5 to about 1:30, from about 1:3 to about 1:20, or from about 2:1 to about 1:20. In some embodiments, the fosaprepitant, or a pharmaceutically acceptable salt thereof, and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the fosaprepitant can be fosaprepitant dimeglumine, which is a dimeglumine salt of fosaprepitant.

In some embodiments, the human serum albumin is a native human serum albumin.

In some embodiments, the human serum albumin is a recombinant human serum albumin. In some embodiments, the human serum albumin is essentially fatty acid free.

In some embodiments, the composition comprises at least one stabilizer for the human serum albumin. In some embodiments, the composition comprises two stabilizers for the human serum albumin. In some embodiments, the stabilizers are N-acetyltryptophanate and caprylic acid or sodium salt thereof. In some embodiments, the stabilizer is N-acetyltryptophanate or sodium salt thereof. In some embodiments, the stabilizer is caprylic acid or sodium salt thereof.

In some embodiments, the composition is a solid formulation. For example, the solid formulation can be produced in a uniform manner by lyophilization. A skilled artisan would recognize other methods, such as rotary evaporation, that can also produce solid formulations.

In some embodiments, the composition is an aqueous formulation. In some embodiments, the aqueous formulation is substantially free of solvent other than water. In some embodiments, the aqueous formulation is free of solvent other than water.

In some embodiments, the aqueous formulation is a clear aqueous solution. In some embodiments, the solution remains clear for at least about 1 hours, 2 hours, 3 hours, 4 hours, 5 hours, 6 hours, 8 hours, 12 hours, or 24 hours.

Also, provided herein is a pharmaceutical composition comprising the composition comprising the fosaprepitant, or the pharmaceutically acceptable salt thereof, the aprepitant and the human serum albumin as described herein, and a pharmaceutically acceptable carrier.

In some embodiments, the pharmaceutical composition is free of a surfactant, such as CREMOPHOR® surfactants and Polysorbate 80.

Also, provided herein is a method for the prevention of chemotherapy-induced nausea and vomiting, the method comprising the step of administering to a subject in need thereof of a therapeutically effective amount of a pharmaceutical composition comprising the composition comprising the fosaprepitant, or the pharmaceutically acceptable salt thereof, the aprepitant and the human serum albumin as described herein, and a pharmaceutically acceptable carrier.

In some embodiments, the methods described herein are for the prevention of acute and delayed nausea and vomiting associated with initial and repeat courses of highly emetogenic cancer chemotherapy (HEC) including high-dose cisplatin. In some embodiments, the methods described herein are for the prevention of delayed nausea and vomiting associated with initial and repeat courses of moderately emetogenic cancer chemotherapy (MEC).

Also, provided herein is a liquid pharmaceutical composition comprising the composition comprising the fosaprepitant, or the pharmaceutically acceptable salt thereof, the aprepitant and the human serum albumin as described herein, and a pharmaceutically acceptable carrier.

In some embodiments, the liquid pharmaceutical composition is a reconstituted solution, reconstituted from the solid composition comprising the fosaprepitant, or the pharmaceutically acceptable salt thereof, the aprepitant and the human serum albumin as described herein.

In some embodiments, the liquid pharmaceutical composition is an aqueous solution. In some embodiments, the liquid pharmaceutical composition is an aqueous solution substantially free of solvent other than water. In some embodiments, the liquid pharmaceutical composition is an aqueous solution free of solvent other than water.

In some embodiments, the liquid pharmaceutical composition is an aqueous reconstituted solution, reconstituted in a parenterally acceptable aqueous pharmaceutical diluent. In some embodiments, the liquid pharmaceutical composition is an aqueous reconstituted solution, reconstituted in an aqueous infusion fluid.

In some embodiments, the liquid pharmaceutical composition is an aqueous reconstituted solution, prepared by reconstituting a solid composition comprising the fosaprepitant, or the pharmaceutically acceptable salt thereof, and the aprepitant in a parenterally acceptable aqueous pharmaceutical diluents comprising human serum albumin.

In some embodiments, the liquid pharmaceutical composition is an aqueous reconstituted solution, prepared by reconstituting a solid composition comprising the fosaprepitant, or the pharmaceutically acceptable salt thereof, and the aprepitant in a parenterally acceptable aqueous pharmaceutical diluents comprising a commercially available solution of human serum albumin USP for infusion.

In some embodiments, the liquid pharmaceutical composition is an aqueous reconstituted solution, prepared by reconstituting a solid composition comprising the fosaprepitant, or the pharmaceutically acceptable salt thereof, and the aprepitant in an aqueous solution comprising a commercially available solution of human serum albumin USP for infusion.

In some embodiments, the liquid pharmaceutical composition is an aqueous reconstituted solution, prepared by reconstituting a solid composition comprising the fosaprepitant, or the pharmaceutically acceptable salt thereof, and the aprepitant in an aqueous solution prepared from diluting a commercially available solution of human serum albumin USP for infusion with water or saline.

In some embodiments, the liquid pharmaceutical composition is an aqueous reconstituted solution, prepared by reconstituting a solid composition comprising the fosaprepitant, or the pharmaceutically acceptable salt thereof, and the aprepitant in a commercially available solution of human serum albumin USP for infusion.

In some embodiments, the liquid pharmaceutical composition is an aqueous reconstituted solution, prepared by reconstituting a solid composition comprising the fosaprepitant, or the pharmaceutically acceptable salt thereof, and the aprepitant in an aqueous solution of human serum albumin, in which the concentration of human serum albumin in the solution is in the range from 0.1% to 25% (w/v).

In some embodiments, the fosaprepitant can be fosaprepitant dimeglumine, which is a dimeglumine salt of fosaprepitant.

In some embodiments, the liquid pharmaceutical composition is a clear aqueous solution. In some embodiments, the liquid pharmaceutical composition is a clear aqueous solution for at least 1 hour. In some embodiments, the liquid pharmaceutical composition is a clear aqueous solution for at least 2 hours. In some embodiments, the liquid pharmaceutical composition is a clear aqueous solution for at least 3 hours. In some embodiments, the liquid pharmaceutical composition is a clear aqueous solution for at least 4 hours. In some embodiments, the liquid pharmaceutical composition is a clear aqueous solution for at least 5 hours. In some embodiments, the liquid pharmaceutical composition is a clear aqueous solution for at least 6 hours. In some embodiments, the liquid pharmaceutical composition is a clear aqueous solution for at least 24 hours.

In some embodiments, the liquid pharmaceutical composition is an injectable pharmaceutical formulation.

In some embodiments, the injectable pharmaceutical formulation is free of solvent other than water. In some embodiments, the injectable pharmaceutical formulation is substantially free of solvent other than water.

In some embodiments, the injectable pharmaceutical formulation is a reconstituted solution, reconstituted from the composition comprising the fosaprepitant, or the pharmaceutically acceptable salt thereof, the aprepitant and the human serum albumin as described herein. In some embodiments, the injectable pharmaceutical formulation is a reconstituted solution, reconstituted in an aqueous infusion fluid. In some embodiments, the aqueous infusion fluid is normal saline. In some embodiments, the aqueous infusion fluid is a dextrose solution.

In some embodiments, the injectable pharmaceutical formulation is a clear aqueous solution. In some embodiments, the injectable pharmaceutical formulation is a clear aqueous solution for at least 1 hour. In some embodiments, the injectable pharmaceutical formulation is a clear aqueous solution for at least 2 hours. In some embodiments, the injectable pharmaceutical formulation is a clear aqueous solution for at least 3 hours. In some embodiments, the injectable pharmaceutical formulation is a clear aqueous solution for at least 4 hours. In some embodiments, the injectable pharmaceutical formulation is a clear aqueous solution for at least 5 hours. In some embodiments, the injectable pharmaceutical formulation is a clear aqueous solution for at least 6 hours.

In some embodiments, the injectable pharmaceutical formulation is a clear aqueous solution for at least 1 hour at a temperature from about 0 °C to about 10 °C. In some embodiments, the injectable pharmaceutical formulation is a clear aqueous solution for at least 2 hours at a temperature from about 0 °C to about 10 °C. In some embodiments, the injectable pharmaceutical formulation is a clear aqueous solution for at least 3 hours at a temperature from about 0 °C to about 10 °C. In some embodiments, the injectable pharmaceutical formulation is a clear aqueous solution for at least 6 hours at a temperature from about 0 °C to about 10 °C. In some embodiments, the injectable pharmaceutical formulation is a clear aqueous solution for at least 8 hours at a temperature from about 0 °C to about 10 °C. In some embodiments, the injectable pharmaceutical formulation is a clear aqueous solution for at least 24 hours at a temperature from about 0 °C to about 10 °C.

Also, provided herein is a kit comprising a solid composition comprising the fosaprepitant, or the pharmaceutically acceptable salt thereof, and the aprepitant and an aqueous solution of human serum albumin.

## DETAILED DESCRIPTION

### Compositions comprising fosaprepitant, or a pharmaceutically acceptable salt thereof, and human serum albumin.

Provided herein is a composition comprising fosaprepitant, or a pharmaceutically acceptable salt thereof, and human serum albumin, wherein the fosaprepitant, or a pharmaceutically acceptable salt thereof, and the human serum albumin in the composition have a ratio by weight from about 1:0.1 to about 1:500.

In some embodiments, the composition comprises a non-covalently bound complex comprising fosaprepitant and human serum albumin. In some aspects of these embodiments, the molar ratio of fosaprepitant to human serum albumin in the complex is from about 200:1 to about 1:1, from about 150:1 to about 1:1, from about 100:1 to about 1:1, from about 80:1 to about 1:1, from about 60:1 to about 1:1, from about 60:1 to about 3:1, from about 55:1 to about 1:1, from about 55:1 to about 3:1, from about 50:1 to about 1:1, from about 50:1 to about 3:1, from about 40:1 to about 1:1, from about 30:1 to about 1:1, from about 30:1 to about 3:1, from about 20:1 to about 1:1, from about 20:1 to about 3:1, from about 10:1 to about 1:1, from about 5:1 to about 1:1, from about 5:1 to about 3:1, or from about 3:1 to about 1:1. In other aspects of these embodiments, the molar ratio of fosaprepitant to human serum albumin in the complex is about 1:1, about 2:1, about 3:1, about 4:1, about 5:1, about 6;1, about 7:1, about 8;1, about 9:1, about 10:1, about 15;1, about 20:1, about 30:1, about 40:1.

In some embodiments, the fosaprepitant, or a pharmaceutically acceptable salt thereof, and the human serum albumin in the composition have a ratio by weight from about 1:0.5 to about 1:300, from about 1:0.8 to about 1:200, from about 1:1 to about 1:150, from about 1:2 to about 1:100, from about 1:2 to about 1:80, from about 1:2 to about 1:50, from about 1:2 to about 1:40, from about 1:2.5 to about 1:30, or from about 1:3 to about 1:20.. In some embodiments, the fosaprepitant, or a pharmaceutically acceptable salt thereof, and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the fosaprepitant, or a pharmaceutically acceptable salt thereof, and the human serum albumin in the composition have a molar ratio from about 200:1 to about 1:1, from about 150:1 to about 1:1, from about 100:1 to about 1:1, from about 80:1 to about 1:1, from about 60:1 to about 1:1, from about 60:1 to about 3:1, from about 55:1 to about 1:1, from about 55:1 to about 3:1, from about 50:1 to about 1:1, from about 50:1 to about 3:1, from about 40:1 to about 1:1, from about 30:1 to about 1:1, from about 30:1 to about 3:1, from about 20:1 to about 1:1, from about 20:1 to about 3:1, from about 10:1 to about 1:1, from about 5:1 to about 1:1, from about 5:1 to about 3:1, or from about 3:1 to about 1:1. In some embodiments, the fosaprepitant, or a pharmaceutically acceptable salt thereof, and the human serum albumin in the composition have a molar ratio of about 1:1, about 3:1, about 5:1, about 7:1, about 10:1, about 15;1, about 20:1, about 25;1, about 30:1, about 40:1, about 45;1, about 50:1, about 55:1, about 60:1, about 80:1, or about 100:1.

As used herein, the term "human serum albumin" refers to native and recombinant human serum albumin. Native human serum albumin and other plasma proteins can be precipitated from human plasma by varying the pH and adding ethanol, in what is known as the Cohn fractionation process (Cohn EJ et al., J. Am. Chem. Soc. 1946; 68:459-475). By controlling the pH and ethanol content, semi-purified fractions of plasma proteins can be produced. One of the last proteins to precipitate in the Cohn process is native human serum albumin. After precipitation, a wet paste of crude native human serum albumin is obtained. Subsequent bioprocessing steps (purification, filtration, pasteurization, etc.) can be used to produce a purified, stabilized form of native human serum albumin for commercial use (Lin JJ et al., Pharmaceutical Research 2000; 17:391-6). Recombinant human serum albumin is a highly purified animal-, virus-, and prion-free product as alternative to native human serum albumin, to which it is structurally equivalent (Bosse D et al., J.

Clin. Pharmacol. 2005; 45:57-67). Recombinant human serum albumin has been produced by various hosts, both prokaryotic and eukaryotic (Chen Z et al., Biochimica etBiophysica Acta 2013; 1830:5515-5525). A fatty acid free human serum albumin can be prepared by treatment of human serum albumin with charcoal at low pH. Likewise, treatment of human serum albumin with charcoal at low pH can be used to remove fatty acids from human serum albumin (Chen RF, J. Biol. Chem. 1967; 242:173-181).

Human serum albumin (HSA) is a highly soluble globular protein of $M_r$ 65K and consists of 585 amino acids. HSA is the most abundant protein in the plasma and accounts for 70-80% of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (*see, e.g.,* Tullis, JAMA, 237, 355-360, 460-463, (1977) and Houser et al., Surgery, Gynecology and Obstetrics, 150, 811-816 (1980)) and in conjunction with exchange transfusion in the treatment of neonatal hyperbilirubinemia (*see, e.g.,* Finlayson, Seminars in Thrombosis and Hemostasis, 6, 85-120, (1980)).

Human serum albumin (HSA) has multiple hydrophobic binding sites (a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (*see, e.g.,* Fehske et al., Biochem. Pharmcol., 30, 687-92 (1981), Vorum, Dan. Med. Bull., 46, 379-99 (1999), Kragh-Hansen, Dan. Med Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (1992), and Carter et al., Adv. Protein. Chem., 45, 153-203 (1994)).

In some embodiments, the human serum albumin is a native human serum albumin. In some embodiments, the human serum albumin is a recombinant human serum albumin. In some embodiments, the human serum albumin is a fatty acid free human serum albumin. In some embodiments, the human serum albumin is essentially fatty acid free.

As used herein, the term "essentially fatty acid free" refers to proteins (e.g. serum albumin) that contain less than about 0.02% fatty acid by weight. For example, human serum albumin that is essentially fatty acid free can contain less than 0.02% fatty acid by weight. In some embodiments, the human serum albumin contains no more than 2, 1, 0.5, 0.1, 0.05, 0.01, 0.001, 0.0005, or 0.0001 moles of fatty acids bound to one mole of human serum albumin.

As used herein, the term "fatty acids" refers to non-esterified fatty acids (e.g. linoleic acid, $\alpha$-linoleic acid, $\gamma$-linoleic acid).

Solutions of human serum albumin for infusion are commercially available. Those solutions must be supplemented with stabilizers to allow pasteurization and storage, to avoid the spontaneous polymerization of the albumin. Usually, N-acetyltryptophan and caprylic acid or their sodium salts are used in alone or in combination.

In some embodiments, the human serum albumin is a commercially available solution of human serum albumin USP for infusion. In some embodiments, the solution of human serum albumin USP for infusion is 5% solution of human serum albumin USP (w/v). In some embodiments, the solution of human serum albumin USP for infusion is 20% solution of human serum albumin USP (w/v). In some embodiments, the solution of human serum albumin USP for infusion is 25% solution of human serum albumin USP (w/v). In some embodiments, the human serum albumin is an aqueous solution prepared by diluting a commercially available solution of human serum albumin USP for infusion with saline. In some embodiments, the human serum albumin is an aqueous solution prepared by diluting a commercially available solution of human serum albumin USP for infusion with water.

In some embodiments, the composition comprises at least one stabilizer for the human serum albumin. In some embodiments, the composition comprises two stabilizers for the human serum albumin. In some embodiments, the stabilizers are N-acetyltryptophan and caprylic acid or sodium salt thereof. In some embodiments, the stabilizer is N-acetyltryptophan or sodium salt thereof. In some embodiments, the stabilizer is caprylic acid or sodium salt thereof.

As used herein the term "fosaprepitant" is a compound that has the CAS No. 172673-20-0 and the following chemical structure:

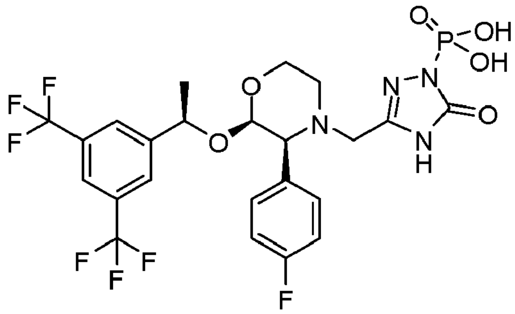

Fosaprepitant (Emend for Injection (US), Ivemend (EU)) is an antiemetic drug, administered intravenously. It is a prodrug of aprepitant.

In some embodiments, the fosaprepitant can be a pharmaceutically acceptable salt of fosaprepitant.

As used herein, the term "pharmaceutically acceptable salts" refers to salts that retain the desired biological activity of the subject compound and exhibit minimal undesired toxicological effects. These pharmaceutically acceptable salts may be prepared in situ during the final isolation and purification of the compound, or by separately reacting the purified compound in its free acid or free base form with a suitable base or acid, respectively. In some embodiments, pharmaceutically acceptable salts may be preferred over the respective free base or free acid because such salts impart greater stability or solubility to the molecule thereby facilitating formulation into a dosage form. Basic compounds are generally capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate),napthalene-2-sulfonate, ethanedisulfonate, hydrogen bisulfide, bitartrate, gluconate, glucuronate, para-bromophenylsulfonate, carbonate, pyrosulfate, sulfite, bisulfite, monohydrogen phosphate, dihydrogen phosphate, metaphosphate, pyrophosphate, chloride, bromide, iodide, decanoate, caprylate, caprate, propiolate, suberate, sebacate, butyne-1,4-dioate, hexyne-1,6-dioate, terephthalate, sulfonate, xylenesulfonate, phenylpropionate, phenylbutyrate, β-hydroxybutyrate, glycolate, propanesulfonate, naphthalene-1-sulfonate, naphthalene-2-sulfonate and 2,5-dihydroxybenzoate. Suitable bases include pharmaceutically acceptable inorganic bases and pharmaceutically acceptable organic bases. Representative pharmaceutically acceptable base addition salts include hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-($C_1$-$C_6$)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; aminosugars such as meglumine; and amino acids such as arginine, lysine, and the like.

In some embodiments, the fosaprepitant can be fosaprepitant dimeglumine, which is a dimeglumine salt of fosaprepitant.

As used herein the term "fosaprepitant dimeglumine" is a compound that has the CAS No. 265121-04-8 and the following chemical structure:

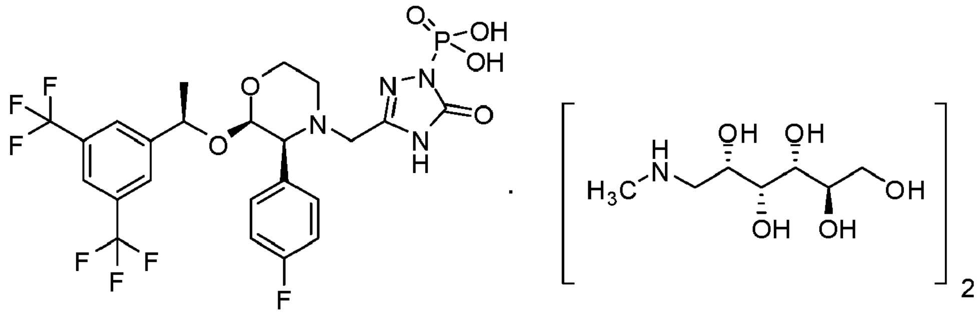

In some embodiments, the fosaprepitant dimeglumine and the human serum albumin in the composition have a ratio by weight from about 1:0.2 to about 1:300, from about 1:0.5 to about 1:150, from about 1:0.8 to about 1:120, from about 1:1 to about 1:100, from about 1:1.2 to about 1:70, from about 1:1.5 to about 1:50, from about 1:1.8 to about 1:20, from about 1:1 to about 1:50, from about 1:2 to about 1:40, from about 1:2 to about 1:20, or from about 1:2 to about 1:10. In some embodiments, the fosaprepitant dimeglumine and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 70.

In some embodiments, the fosaprepitant dimeglumine and the human serum albumin in the composition have a molar ratio from about 200:1 to about 1:1, from about 150:1 to about 1:1, from about 100:1 to about 1:1, from about 80:1 to about 1:1, from about 60:1 to about 1:1, from about 60:1 to about 3:1, from about 55:1 to about 1:1, from about 55:1 to about 3:1, from about 50:1 to about 1:1, from about 50:1 to about 3:1, from about 40:1 to about 1:1, from about 30:1 to about 1:1, from about 30:1 to about 3:1, from about 20:1 to about 1:1, from about 20:1 to about 3:1, from about 10:1 to about 1:1, from about 5:1 to about 1:1, from about 5:1 to about 3:1, or from about 3:1 to about 1:1. In some embodiments, the fosaprepitant dimeglumine and the human serum albumin in the composition have a molar ratio of about 1:1, about 3:1, about 5:1, about 7:1, about 10:1, about 15;1, about 20:1, about 25; 1, about 30:1, about 40:1, about 45;1, about 50:1, about 55:1, about 60:1, about 80:1, or about 100:1.

In some embodiments, the composition is a solid formulation. For example, the solid formulation can be produced in a uniform manner by lyophilization. A skilled artisan would recognize other methods, such as rotary evaporation, that can also produce solid formulations.

In some embodiments, the fosaprepitant and the human serum albumin in the solid formulation are bound non-covalently. In some embodiments, the solid formulation comprises a non-covalently bond complex comprising fosaprepitant and the human serum albumin.

As used herein, the term "non-covalent" refers to an interaction between two or more components, wherein the bonds between the components are non-covalent bonds (i.e., no atom of one component shares a pair of electrons with an atom of another component; e.g., weak bonds such as hydrogen bonds, electrostatic effects, $\pi$-effects, hydrophobic effects and Van der Waals forces). Further, human serum albumin (HSA) has multiple hydrophobic binding sites (a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Additionally, after the drug molecule binds to HSA, the drug molecule and HSA form a non-covalently bound drug and protein complex through the binding sites of HSA. This concept is commonly understood by one of ordinary skill in the art to which this disclosure belongs. One example of a non-covalently bound complex is a non-covalently bound complex of HSA and fatty acids, in which the fatty acids bind to HSA through HSA's multiple binding sites.

In some embodiments, the non-covalent interaction between fosaprepitant and human serum albumin comprises hydrogen bonding. In some embodiments, the non-covalent interaction between fosaprepitant and human serum albumin comprises electrostatic interaction. In some embodiments, the non-covalent interaction between fosaprepitant and human serum albumin comprises hydrophobic interaction. In some embodiments, the non-covalent interaction between fosaprepitant and human serum albumin comprises Van der Waals forces. In some embodiments, the non-covalent interaction between fosaprepitant and human serum albumin comprises hydrogen bonding, electrostatic interaction, hydrophobic interaction, and Van der Waals forces.

In some embodiments, the composition is an aqueous formulation. In some embodiments, the aqueous formulation is substantially free of solvent other than water. In some embodiments, the aqueous formulation is free of solvent other than water.

As used herein, "substantially free of solvent," in reference to an aqueous solution, refers to an aqueous solution that contains less than 0.5 %, by weight, of any non-water solvent. In some embodiments, the aqueous solution contains

less than 0.1%, by weight, of any non-water solvent.

In some embodiments, the aqueous formulation is a clear aqueous solution reconstituted from the solid formulation (e.g. the sterile lyophilized powder) in water. In some embodiments, the aqueous formulation is a clear aqueous solution reconstituted from the solid formulation (e.g. the sterile lyophilized powder) in 0.9% saline. In some embodiments, the aqueous formulation is a clear aqueous solution reconstituted from the solid formulation (e.g. the sterile lyophilized powder) in 5% Dextrose solution.

In some embodiments, the aqueous formulation is a clear aqueous solution reconstituted from the solid formulation (e.g. the sterile lyophilized powder) in water, wherein the aqueous formulation has pH value from about 5 to about 8. In some embodiments, the aqueous formulation is a clear aqueous solution reconstituted from the solid formulation (e.g. the sterile lyophilized powder) in 0.9% saline, wherein the aqueous formulation has pH value from about 5 to about 8. In some embodiments, the aqueous formulation is a clear aqueous solution reconstituted from the solid formulation (e.g. the sterile lyophilized powder) in 5% Dextrose solution, wherein the aqueous formulation has pH value from about 5 to about 8.

In some embodiments, the aqueous formulation is a clear aqueous solution reconstituted from the solid formulation (e.g. the sterile lyophilized powder) in water, wherein the aqueous formulation has pH value from about 6 to about 7.5. In some embodiments, the aqueous formulation is a clear aqueous solution reconstituted from the solid formulation (e.g. the sterile lyophilized powder) in 0.9% saline, wherein the aqueous formulation has pH value from about 6 to about 7.5. In some embodiments, the aqueous formulation is a clear aqueous solution reconstituted from the solid formulation (e.g. the sterile lyophilized powder) in 5% Dextrose solution, wherein the aqueous formulation has pH value from about 6 to about 7.5.

In some embodiments, the aqueous formulation has pH value from about 5 to about 8. In some embodiments, the aqueous formulation has pH value from about 5.5 to about 7.8. In some embodiments, the aqueous formulation has pH value from about 6 to about 7.5. In some embodiments, the aqueous formulation has pH value from about 6.5 to about 7.5. In some embodiments, the aqueous formulation has pH value from about 6 to about 6.5. In some embodiments, the aqueous formulation has pH value from about 6.5 to about 7. In some embodiments, the aqueous formulation has pH value from about 7 to about 7.5. In some embodiments, the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the aqueous formulation is substantially free of solvent other than water. In some embodiments, the aqueous formulation is free of solvent other than water.

In some embodiments, the aqueous formulation is a clear aqueous solution, wherein the aqueous formulation has pH value from about 5 to about 8, and wherein the aqueous formulation is substantially free of solvent other than water. In some embodiments, the aqueous formulation is a clear aqueous solution, wherein the aqueous formulation has pH value from about 5 to about 8, and wherein the aqueous formulation is free of solvent other than water. In some embodiments, the aqueous formulation is a clear aqueous solution, wherein the aqueous formulation has pH value from about 5.5 to about 7.8, and wherein the aqueous formulation is substantially free of solvent other than water. In some embodiments, the aqueous formulation is a clear aqueous solution, wherein the aqueous formulation has pH value from about 5.5 to about 7.8, and wherein the aqueous formulation is free of solvent other than water. In some embodiments, the aqueous formulation is a clear aqueous solution, wherein the aqueous formulation has pH value from about 6 to about 7.5, and wherein the aqueous formulation is substantially free of solvent other than water. In some embodiments, the aqueous formulation is a clear aqueous solution, wherein the aqueous formulation has pH value from about 6 to about 7.5, and wherein the aqueous formulation is free of solvent other than water. In some embodiments, the aqueous formulation is a clear aqueous solution, wherein the aqueous formulation has pH value from about 6.5 to about 7.5, and wherein the aqueous formulation is substantially free of solvent other than water. In some embodiments, the aqueous formulation is a clear aqueous solution, wherein the aqueous formulation has pH value from about 6.5 to about 7.5, and wherein the aqueous formulation is free of solvent other than water. In some embodiments, the aqueous formulation is a clear aqueous solution, wherein the aqueous formulation has pH value from about 6 to about 6.5, and wherein the aqueous formulation is substantially free of solvent other than water. In some embodiments, the aqueous formulation is a clear aqueous solution, wherein the aqueous formulation has pH value from about 6 to about 6.5, and wherein the aqueous formulation is free of solvent other than water. In some embodiments, the aqueous formulation is a clear aqueous solution, wherein the aqueous formulation has pH value from about 6.5 to about 7, and wherein the aqueous formulation is substantially free of solvent other than water. In some embodiments, the aqueous formulation is a clear aqueous solution, wherein the aqueous formulation has pH value from about 6.5 to about 7, and wherein the aqueous formulation is free of solvent other than water. In some embodiments, the aqueous formulation is a clear aqueous solution, wherein the aqueous formulation has pH value from about 7 to about 7.5, and wherein the aqueous formulation is substantially free of solvent other than water. In some embodiments, the aqueous formulation is a clear aqueous solution, wherein the aqueous formulation has pH value from about 7 to about 7.5, and wherein the aqueous formulation is free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about

6.7, about 6.8, about 6.9, about 7.0, about 7.1, about 7.2, about 7.3, about 7.4, or about 7.5, and wherein the aqueous formulation is substantially free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5, and wherein the aqueous formulation is free of solvent other than water.

In some embodiments, the aqueous formulation is a clear aqueous solution. In some embodiments, the solution remains clear for at least about 1 hours, 2 hours, 3 hours, 4 hours, 5 hours, 6 hours, 8 hours, 12 hours, or 24 hours.

As used herein, the term "clear aqueous solution" refers to an aqueous solution that is transparent and free of visible particles or precipitation upon visual observation.

When visually observed, for example, the term "clear aqueous solution" excludes a milky aqueous solution. Further, the term "clear aqueous solution" excludes a cloudy or hazy aqueous solution.

In some embodiments, the aqueous solution is a clear aqueous solution for at least 1 hour. In some embodiments, the aqueous formulation is a clear aqueous solution for at least 2 hours. In some embodiments, the aqueous formulation is a clear aqueous solution for at least 3 hours. In some embodiments, the aqueous formulation is a clear aqueous solution for at least 6 hours. In some embodiments, the aqueous formulation is a clear aqueous solution for at least 8 hours. In some embodiments, the aqueous formulation is a clear aqueous solution for a period of time selected from 1 hour. 2 hours, 3 hours, 4 hours, 5 hours, 6 hours, 7 hours, 8 hours, 12 hours, and 24 hours. In some embodiments, the aqueous formulation is a clear aqueous solution for at least 24 hours.

Also, provided herein is a pharmaceutical composition comprising the composition comprising the fosaprepitant, or a pharmaceutically acceptable salt thereof, and the human serum albumin as described herein, and a pharmaceutically acceptable carrier. In some embodiments, the pharmaceutical composition comprising the composition comprising the fosaprepitant, or a pharmaceutically acceptable salt thereof, and the human serum albumin as described herein, and a pharmaceutically acceptable carrier, is a liquid pharmaceutical composition. In some embodiments, the liquid pharmaceutical composition is an aqueous solution. In some embodiments, the liquid pharmaceutical composition is free of solvent other than water. In some embodiments, the liquid pharmaceutical composition is substantially free of solvent other than water.

In some embodiments, the liquid pharmaceutical composition is an injectable pharmaceutical formulation. In some embodiments, the liquid pharmaceutical composition is a formulation for infusion (e.g., intravenous infusion).

As used herein, the term "aqueous solution" refers to a solution, wherein at least one solvent is water and the weight % of water in the mixture of solvents is at least 50%, at least 60%, at least 70%, at least 90%, at least 95%, or at least 99%. In some embodiments, aqueous solution is a solution in which water is the only solvent. As used herein, the term "aqueous solvent" refer to a liquid comprising at least 50%, at least 60%, at least 70%, at least 90% or at least 95% water. In some embodiments, aqueous solvent is water. In some embodiments, aqueous solvent is 0.9% saline.

In some embodiments, the pharmaceutically acceptable carrier is any carrier useful to solubilize and deliver an agent to a subject. A desirable pharmaceutically acceptable carrier is saline.

Other pharmaceutically acceptable carrier and their formulation are known to one skilled in the art and described, for example, in Remington's Pharmaceutical Sciences. (20th edition), ed. A. Gennaro, 2003, Lippincon Williams & Wilkins. In some embodiments, the carrier may contain components such as, for example, dextrose, glucose, serum proteins (other than HSA), buffer substances such as phosphates, glycine, sorbic acid, potassium sorbate, salts or electrolytes, such as protamine sulfate, disodium hydrogen phosphate, potassium hydrogen phosphate, sodium chloride, zinc salts, and cellulose-based substances. In some embodiments, the carrier may contain components such as contain anti-oxidants, buffers, bacteriostats and solutes which render the formulation isotonic with the blood of the intended recipient.

In some embodiments, the pharmaceutically acceptable excipient is selected from lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The pharmaceutical compositions may additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The pharmaceutical composition may be formulated so as to provide quick, sustained or delayed release of the active ingredient after administration to the patient by employing procedures known in the art.

In some embodiments, the pharmaceutical composition of the present disclosure may be administered by a syringe or a catheter, or any other means generally known in the art for the delivery of a pharmaceutical agent by injection to the subject in need thereof. The delivery means will vary, as recognized by those skilled in the art, depending on the diseases and conditions treated, the severity of the disease, the sex, age and general health condition of the subject, excipient usage, the possibility of co-usage with other therapeutic treatments such as use of other agents as described herein and the judgment of the treating physician.

The pharmaceutical composition comprising the composition comprising the fosaprepitant, or a pharmaceutically acceptable salt thereof, and the human serum albumin as described herein can be administered to a subject via

various routes, such as parenterally, including intravenous, intra-arterial, intraperitoneal, intrapulmonary, oral, inhalation, intravascular, intramuscular, intra-tracheal, subcutaneous, intraocular, intrathecal, or transdermal. For example, the composition can be administered by inhalation to treat conditions of the respiratory tract. In some embodiments, the pharmaceutical composition is administrated intravenously (e.g., as an infusion).

In some embodiments, the pharmaceutical composition is free of a surfactant, such as CREMOPHOR® surfactants and Polysorbate 80. In some embodiments, the pharmaceutical composition is substantially free of a surfactant, such as CREMOPHOR® surfactants and Polysorbate 80. As used herein, the term "substantially free of surfactant" refers to a formulation containing less than 0.0005%, less than 0.0003%, or less than 0.0001% of surfactants and/or less than 0.0005%, less than 0.0003%, or less than 0.0001% of surfactant.

Also, provided herein is a method of treating chemotherapy-induced nausea and vomiting, the method comprising the step of administering to a subject in need thereof of a therapeutically effective amount of a pharmaceutical composition comprising the composition comprising the fosaprepitant, or a pharmaceutically acceptable salt thereof, and the human serum albumin as described herein, and a pharmaceutically acceptable carrier.

Also, provided herein is a method for the prevention of chemotherapy-induced nausea and vomiting, the method comprising the step of administering to a subject in need thereof of a therapeutically effective amount of a pharmaceutical composition comprising the composition comprising the fosaprepitant, or a pharmaceutically acceptable salt thereof, and the human serum albumin as described herein, and a pharmaceutically acceptable carrier.

In some aspects of these embodiments, the chemotherapy-induced nausea and vomiting is acute and delayed nausea and vomiting associated with initial and repeat courses of highly emetogenic cancer chemotherapy (HEC) including high-dose cisplatin. In other aspects of these embodiments, the chemotherapy-induced nausea and vomiting delayed nausea and vomiting associated with initial and repeat courses of moderately emetogenic cancer chemotherapy (MEC).

As used herein, the terms "individual", "patient", or "subject" are used interchangeably and refer to any animal, including mammals, preferably mice, rats, other rodents, rabbits, dogs, cats, swine, cattle, sheep, horses, or primates, and most preferably humans.

As used herein the term "treating" or "treatment" refers to 1) inhibiting the disease; for example, inhibiting a disease, condition or disorder in an individual who is experiencing or displaying the pathology or symptomatology of the disease, condition or disorder (i.e., arresting further development of the pathology and/or symptomatology), or 2) ameliorating the disease; for example, ameliorating a disease, condition or disorder in an individual who is experiencing or displaying the pathology or symptomatology of the disease, condition or disorder (i.e., reversing the pathology and/or symptomatology).

As used herein, an "effective amount," "therapeutically effective amount," or a "pharmaceutically-effective amount" in reference to the compounds or compositions of the instant invention refers to the amount sufficient to induce a desired biological, pharmacological, or therapeutic outcome in a subject. That result can be reduction, prevention, mitigation, delay, shortening the time to resolution of, alleviation of the signs or symptoms of, or exert a medically-beneficial effect upon the underlying pathophysiology or pathogenesis of an expected or observed side-effect, toxicity, disorder or condition, or any other desired alteration of a biological system.

As used herein, the term "preventing" (or "prevention") means to completely or almost completely stop an disease or condition (e.g., cancer, metastatic cancer) from occurring, for example when the patient or subject is predisposed to an condition or is at risk of a disease or condition. Preventing can also include inhibiting, i.e., arresting the development, of a condition.

In some embodiments, the methods described herein are for the prevention of acute and delayed nausea and vomiting associated with initial and repeat courses of highly emetogenic cancer chemotherapy (HEC) including high-dose cisplatin. In some embodiments, the methods described herein are for the prevention of delayed nausea and vomiting associated with initial and repeat courses of moderately emetogenic cancer chemotherapy (MEC).

In some embodiments, the methods described herein are performed in combination with at least one other antiemetic agent. In some embodiments, the methods described herein are performed in combination with dexamethasone and a 5-$HT_3$ antagonist (e.g., tropisetron, palonosetron, ramosetron, granisetron, ondansetron, dolasetron, or metoclopramide). In some embodiments, the antiemetic agent is selected from tropisetron, palonosetron, ramosetron, granisetron, ondansetron, dolasetron, metoclopramide, domperidone, olanzapine, droperidol, haloperidol, chlorpromazine, prochlorperazine, alizapride, prochlorperazine, metoclopramide, casopitant, aprepitant, cyclizine, diphenhydramine, dimenhydrinate, doxylamine, meclizine, promethazine, hydroxyzine, dronabinol, sativex, midazolam, lorazepam, hyoscine, trimethobenzamide, emetrol, propofol and muscimol.

In some embodiments, provided herein is a method for treating chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC)), the method comprising administering to a subject in need thereof a therapeutically effective amount a pharmaceutical composition comprising the composition comprising

the fosaprepitant, or a pharmaceutically acceptable salt thereof, and the human serum albumin as described herein, and a therapeutically effective amount of at least one antiemetic agent as described herein.

In some embodiments, provided herein is a method for preventing chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC)), the method comprising administering to a subject in need thereof a therapeutically effective amount a pharmaceutical composition comprising the composition comprising the fosaprepitant, or a pharmaceutically acceptable salt thereof, and the human serum albumin as described herein, and a therapeutically effective amount of at least one antiemetic agent as described herein.

In some embodiments, a pharmaceutical composition comprising the composition comprising the fosaprepitant, or a pharmaceutically acceptable salt thereof, and the human serum albumin as described herein and an antiemetic agent are administered simultaneously.

In some embodiments, a pharmaceutical composition comprising the composition comprising the fosaprepitant, or a pharmaceutically acceptable salt thereof, and the human serum albumin as described herein and an antiemetic agent are administered consecutively.

Also, provided herein is a pharmaceutical composition comprising the composition comprising the fosaprepitant, or a pharmaceutically acceptable salt thereof, and the human serum albumin as described herein, at least one antiemetic agent as described herein, and a pharmaceutically acceptable carrier.

In some embodiments, provided herein is a pharmaceutical composition comprising the composition comprising the fosaprepitant and the human serum albumin as described herein, a 5-$HT_3$ antagonist (e.g., tropisetron, palonosetron, ramosetron, granisetron, ondansetron, dolasetron, or metoclopramide), and a pharmaceutically acceptable carrier.

In some embodiments, provided herein is a pharmaceutical composition comprising the composition comprising the fosaprepitant and the human serum albumin as described herein, palonosetron, and a pharmaceutically acceptable carrier.

In some embodiments, provided herein is a pharmaceutical composition comprising the composition comprising the fosaprepitant and the human serum albumin as described herein, 0.5mg of palonosetron, and a pharmaceutically acceptable carrier.

In some embodiments, provided herein is a pharmaceutical composition comprising the composition comprising the fosaprepitant and the human serum albumin as described herein, 0.25mg of palonosetron, and a pharmaceutically acceptable carrier.

In some embodiments, provided herein is a pharmaceutical composition comprising the composition comprising the fosaprepitant and the human serum albumin as described herein, 0.28mg of palonosetron hydrochloride, and a pharmaceutically acceptable carrier.

In some embodiments, provided herein is a pharmaceutical composition comprising the composition comprising the fosaprepitant and the human serum albumin as described herein, about 0.25mg of palonosetron, and a pharmaceutically acceptable carrier.

In some embodiments, provided herein is a pharmaceutical composition comprising the composition comprising the fosaprepitant and the human serum albumin as described herein, about 0.28mg of palonosetron hydrochloride, and a pharmaceutically acceptable carrier.

In some embodiments, provided herein is a pharmaceutical composition comprising 150mg of fosaprepitant, the human serum albumin, 0.25mg of palonosetron, and a pharmaceutically acceptable carrier.

In some embodiments, provided herein is a pharmaceutical composition comprising 150mg of fosaprepitant, the human serum albumin, 0.28mg of palonosetron hydrochloride, and a pharmaceutically acceptable carrier.

In some embodiments, provided herein is a pharmaceutical composition comprising 245.3mg of fosaprepitant dimeglumine, the human serum albumin, 0.25mg of palonosetron, and a pharmaceutically acceptable carrier.

In some embodiments, provided herein is a pharmaceutical composition comprising 245.3mg of fosaprepitant dimeglumine, the human serum albumin, 0.28mg of palonosetron hydrochloride, and a pharmaceutically acceptable carrier.

In some embodiments, provided herein is a liquid pharmaceutical formulation for injection comprising the composition comprising the fosaprepitant and the human serum albumin as described herein, and a pharmaceutically acceptable carrier.

In some embodiments, provided herein is a liquid pharmaceutical formulation for injection comprising the composition comprising the fosaprepitant and the human serum albumin as described herein, 0.25mg of palonosetron, and a pharmaceutically acceptable carrier.

In some embodiments, provided herein is a liquid pharmaceutical formulation for injection comprising the composition comprising the fosaprepitant and the human serum albumin as described herein, 0.28mg of palonosetron hydrochloride, and a pharmaceutically acceptable carrier.

In some embodiments, provided herein is a liquid pharmaceutical formulation for injection comprising 150mg

of fosaprepitant, the human serum albumin, 0.25mg of palonosetron, and a pharmaceutically acceptable carrier.

In some embodiments, provided herein is a liquid pharmaceutical formulation for injection comprising 150mg of fosaprepitant, the human serum albumin, 0.28mg of palonosetron hydrochloride, and a pharmaceutically acceptable carrier.

In some embodiments, provided herein is a liquid pharmaceutical formulation for injection comprising 245.3mg of fosaprepitant dimeglumine, the human serum albumin, 0.25mg of palonosetron, and a pharmaceutically acceptable carrier.

In some embodiments, provided herein is a liquid pharmaceutical formulation for injection comprising 245.3mg of fosaprepitant dimeglumine, the human serum albumin, 0.28mg of palonosetron hydrochloride, and a pharmaceutically acceptable carrier.

In some embodiments, the liquid pharmaceutical formulation for injection is an aqueous solution. In some embodiments, the liquid pharmaceutical formulation for injection is free of solvent other than water. In some embodiments, the liquid pharmaceutical formulation for injection is substantially free of solvent other than water. In some embodiments, the liquid pharmaceutical formulation for injection is a formulation for infusion (e.g., intravenous infusion).

In some embodiments, the liquid pharmaceutical formulation for injection has pH value from about 5 to about 8. In some embodiments, the liquid pharmaceutical formulation for injection has pH value from about 5.5 to about 7.8. In some embodiments, the liquid pharmaceutical formulation for injection has pH value from about 6 to about 7.5. In some embodiments, the liquid pharmaceutical formulation for injection has pH value from about 6.5 to about 7.5. In some embodiments, the liquid pharmaceutical formulation for injection has pH value from about 6 to about 6.5. In some embodiments, the liquid pharmaceutical formulation for injection has pH value from about 6.5 to about 7. In some embodiments, the liquid pharmaceutical formulation for injection has pH value from about 7 to about 7.5. In some embodiments, the liquid pharmaceutical formulation for injection has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the liquid pharmaceutical formulation for injection is substantially free of solvent other than water. In some embodiments, the liquid pharmaceutical formulation for injection is free of solvent other than water.

As used herein the term "palonosetron" is a compound that has the CAS No. 135729-61-2 and the following chemical structure:

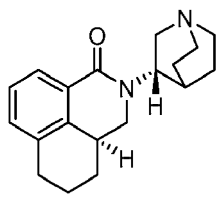

In some embodiments, the palonosetron can be a pharmaceutically acceptable salt of palonosetron. In some embodiments, the palonosetron can be palonosetron hydrochloride.

As used herein the term "palonosetron hydrochloride" is a compound that has the CAS No.135729-62-3 and the following chemical structure:

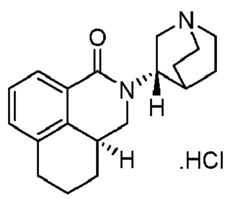

In some embodiments, provided herein is a pharmaceutical composition comprising the composition comprising the fosaprepitant and the human serum albumin as described herein, granisetron, and a pharmaceutically acceptable carrier.

In some embodiments, provided herein is a pharmaceutical composition comprising the composition comprising the fosaprepitant and the human serum albumin as described herein, ondansetron, and a pharmaceutically acceptable carrier.

The methods described herein may be performed alone or in conjunction with another therapy, such as surgery, radiation, chemotherapy, immunotherapy, gene therapy, and the like.

In some embodiments, the amount of fosaprepitant, or a pharmaceutically acceptable salt thereof, that is administered to a subject in need thereof with any one of pharmaceutical compositions described herein is from about 50 mg to about 250 mg, from about 60 mg to about 240 mg, from about 70 mg to 230 mg, from about 80 mg to about 220 mg, from about 90 mg to about 210 mg, from about 100 mg to about 200 mg, or from about 125 mg to about 175 mg. In some embodiments, the amount of fosaprepitant, or a pharmaceutically acceptable salt thereof, that is administered to a subject in need thereof with any one of pharmaceutical compositions described herein is about 100 mg, about 125 mg, about 150 mg, about 175 mg, or about 200 mg. In some embodiments, the amount of fosaprepitant, or a pharmaceutically acceptable salt thereof, that is administered to a subject in need thereof with any one of pharmaceutical compositions described herein is about 150 mg. In some embodiments, the amount of fosaprepitant, or a pharmaceutically acceptable salt thereof, is administered as an intravenous infusion over 20 to 30 minutes approximately 30 minutes prior to chemotherapy.

As will be understood by those of ordinary skill in the art, the appropriate doses of fosaprepitant will be approximately those already employed in clinical therapies wherein fosaprepitant is administered alone or in combination with other therapeutic agents. Variation in dosage will likely occur depending on the condition being treated. Appropriate effective doses will also vary, as recognized by those skilled in the art, depending on the severity of the disease, the route of administration, the sex, age and general health condition of the subject, excipient usage, the possibility of co-usage with other therapeutic treatments such as use of other agents, and the judgment of the treating physician. For example, guidance for selecting an effective dose can be determined by reference to the prescribing information for fosaprepitant.

Also, provided herein is a kit comprising a solid composition comprising fosaprepitant, or a pharmaceutically acceptable salt thereof, and an aqueous solution of human serum albumin.

In some embodiments, provided herein is a kit comprising a solid composition comprising palonosetron and fosaprepitant, or a pharmaceutically acceptable salt thereof, and an aqueous solution of human serum albumin.

In some embodiments, provided herein is a kit comprising a solid composition comprising 0.25mg of palonosetron and fosaprepitant, or a pharmaceutically acceptable salt thereof, and an aqueous solution of human serum albumin.

In some embodiments, provided herein is a kit comprising a solid composition comprising 0.28mg of palonosetron hydrochloride and fosaprepitant, or a pharmaceutically acceptable salt thereof, and an aqueous solution of human serum albumin.

In some embodiments, the fosaprepitant, or a pharmaceutically acceptable salt thereof, and the human serum albumin in the kit have a ratio by weight from about 1:0.5 to about 1:300. In some embodiments, the fosaprepitant, or a pharmaceutically acceptable salt thereof, and the human serum albumin in the kit have a ratio by weight from about 1:0.8 to about 1:200. In some embodiments, the fosaprepitant, or a pharmaceutically acceptable salt thereof, and the human serum albumin in the kit have a ratio by weight from about 1:1 to about 1:150. In some embodiments, the fosaprepitant, or a pharmaceutically acceptable salt thereof, and the human serum albumin in the kit have a ratio by weight from about 1:2 to about 1:100. In some embodiments, the fosaprepitant, or a pharmaceutically acceptable salt thereof, and the human serum albumin in the kit have a ratio by weight from about 1:2 to about 1:80. In some embodiments, the fosaprepitant, or a pharmaceutically acceptable salt thereof, and the human serum albumin in the kit have a ratio by weight from about 1:2 to about 1:50. In some embodiments, the fosaprepitant, or a pharmaceutically acceptable salt thereof, and the human serum albumin in the kit have a ratio by weight from about 1:2.5 to about 1:30. In some embodiments, the fosaprepitant, or a pharmaceutically acceptable salt thereof, and the human serum albumin in the kit have a ratio by weight from about 1:3 to about 1:20. In some embodiments, the fosaprepitant, or a pharmaceutically acceptable salt thereof, and the human serum albumin in the kit have a ratio by weight from about 1:3.5 to about 1:15. In some embodiments, the fosaprepitant, or a pharmaceutically acceptable salt thereof, and the human serum albumin in the kit have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the solid composition in the kit comprises the fosaprepitant dimeglumine.

In some embodiments, the solid composition in the kit comprises the fosaprepitant dimeglumine and palonosetron.

In some embodiments, the solid composition in the kit comprises the fosaprepitant dimeglumine and palonosetron hydrochloride.

In some embodiments, provided herein is a kit comprising a solid composition comprising fosaprepitant dimeglumine, and an aqueous solution of human serum albumin.

In some embodiments, provided herein is a kit comprising a solid composition comprising palonosetron and fosaprepitant dimeglumine, and an aqueous solution of human serum albumin.

In some embodiments, provided herein is a kit comprising a solid composition comprising fosaprepitant dimeglumine and 0.25mg of palonosetron, and an aqueous solution of human serum albumin.

In some embodiments, provided herein is a kit comprising a solid composition comprising fosaprepitant dimeglumine and 0.28mg of palonosetron hydrochloride , and an aqueous solution of human serum albumin.

In some embodiments, the fosaprepitant dimeglumine and the human serum albumin in the solid composition have a ratio by weight from about 1:0.2 to about 1:300. In some embodiments, the fosaprepitant dimeglumine and the human serum albumin in the solid composition have a ratio by weight from about 1:0.5 to about 1:150. In some embodiments, the fosaprepitant dimeglumine and the human serum albumin in the solid composition have a ratio by weight from about 1:0.8 to about 1:120. In some embodiments, the fosaprepitant dimeglumine and the human serum albumin in the solid composition have a ratio by weight from about 1:1 to about 1:100. In some embodiments, the fosaprepitant dimeglumine and the human serum albumin in the solid composition have a ratio by weight from about 1:1.2 to about 1:70. In some embodiments, the fosaprepitant dimeglumine and the human serum albumin in the solid composition have a ratio by weight from about 1:1.5 to about 1:50. In some embodiments, the fosaprepitant dimeglumine and the human serum albumin in the solid composition have a ratio by weight from about 1:1.8 to about 1:20. In some embodiments, the fosaprepitant dimeglumine and the human serum albumin in the solid composition have a ratio by weight from about 1:2 to about 1:10. In some embodiments, the fosaprepitant dimeglumine and the human serum albumin in the solid composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the aqueous solution of human serum albumin in the kit comprises a commercially available solution of human serum albumin USP for infusion.

In some embodiments, the aqueous solution of human serum albumin in the kit has concentration of human serum albumin in the range from 0.1% to 25% (w/v).

In some embodiments, the solid composition in the kit comprises 150 mg of fosaprepitant.

In some embodiments, the solid composition in the kit comprises 245.3 mg of fosaprepitant dimeglumine equivalent to 150 mg of fosaprepitant free acid.

In some embodiments, the solid composition in the kit comprises 0.25mg of palonosetron.

In some embodiments, the solid composition in the kit comprises 0.28mg of palonosetron hydrochloride.

In some embodiments, the solid composition in the kit comprises 245.3 mg of fosaprepitant dimeglumine and 0.25mg of palonosetron.

In some embodiments, the solid composition in the kit comprises 245.3 mg of fosaprepitant dimeglumine and 0.28mg of palonosetron hydrochloride.

In some embodiments, the kit comprises the solid composition comprising 245.3 mg of fosaprepitant dimeglumine equivalent to 150 mg of fosaprepitant free acid and an aqueous solution of human serum albumin with the human serum albumin concentration in the range from 0.1% to 25% (w/v).

In some embodiments, the kit comprises the solid composition comprising 245.3 mg of fosaprepitant dimeglumine and 0.25mg of palonosetron, and an aqueous solution of human serum albumin with the human serum albumin concentration in the range from 0.1% to 25% (w/v).

In some embodiments, the kit comprises the solid composition comprising 245.3 mg of fosaprepitant dimeglumine and 0.28mg of palonosetron hydrochloride, and an aqueous solution of human serum albumin with the human serum albumin concentration in the range from 0.1% to 25% (w/v).

In some embodiments, the kit comprises the solid composition comprising 245.3mg of fosaprepitant dimeglumine equivalent to 150 mg of fosaprepitant free acid and a 10% aqueous solution of human serum albumin (w/v).

In some embodiments, the kit comprises the solid composition comprising 245.3 mg of fosaprepitant dimeglumine and 0.25mg of palonosetron, and a 10% aqueous solution of human serum albumin (w/v).

In some embodiments, the kit comprises the solid composition comprising 245.3 mg of fosaprepitant dimeglumine and 0.28mg of palonosetron hydrochloride, and a 10% aqueous solution of human serum albumin (w/v).

In some embodiments, the kit comprises the solid composition comprising 245.3mg of fosaprepitant dimeglumine equivalent to 150 mg of fosaprepitant free acid and a 10% aqueous solution of human serum albumin (w/v) (10ml).

In some embodiments, the kit comprises the solid composition comprising 245.3 mg of fosaprepitant dimeglumine and 0.25mg of palonosetron, and a 10% aqueous solution of human serum albumin (w/v) (10ml).

In some embodiments, the kit comprises the solid composition comprising 245.3 mg of fosaprepitant dimeglumine and 0.28mg of palonosetron hydrochloride, and a 10% aqueous solution of human serum albumin (w/v) (10ml).

In some embodiments, aqueous solution is a solution in which water is the only solvent.

## Compositions comprising aprepitant and human serum albumin

Also, provided herein is a composition comprising aprepitant and human serum albumin, wherein the aprepitant and the human serum albumin in the composition have a ratio by weight from about 1:80 to about 1:1000. In some embodiments, the composition comprises a non-covalently bound complex comprising aprepitant and human serum albumin.

In some embodiments, the aprepitant and the human serum albumin in the composition have a ratio by weight from about 1:100 to about 1:800. In some embodiments, the aprepitant and the human serum albumin in the composition have a ratio by weight from about 1:120 to about 1:600. In some embodiments, the aprepitant and the human serum albumin in the composition have a ratio by weight from about 1:130 to about 1:400. In some embodiments, the aprepitant and the human serum albumin in the composition have a ratio by weight from about 1:140 to about 1:300. In some embodiments, the aprepitant and the human serum albumin in the composition have a ratio by weight from about 1:150 to about 1:280. In some embodiments, the aprepitant and the human serum albumin in the composition have a ratio by weight from about 1:160 to about 1:260. In some embodiments, the aprepitant and the human serum albumin in the composition have a ratio by weight from about 1:180 to about 1:250. In some embodiments, the aprepitant and the human serum albumin in the composition have a ratio by weight from about 1:200 to about 1:250. In some embodiments, the aprepitant and the human serum albumin in the composition have a ratio by weight of about 1:120, about 1:130, about 1:140, about 1:150, about 1:160, about 1:170, about 1:180, about 1:190, about 1:200, about 1:210, about 1:220, about 1:230, about 1:240, about 1:250, about 1:260, about 1:270, about 1:280, about 1:290, about 1:300, about 1:310, about 1:320, about 1:330, about 1:340, about 1:350, or about 1:400.

In some embodiments, the term "human serum albumin" refers to native and recombinant human serum albumin. Native human serum albumin and other plasma proteins can be precipitated from human plasma by varying the pH and adding ethanol, in what is known as the Cohn fractionation process (Cohn EJ et al., J. Am. Chem. Soc. 1946; 68:459-475). By controlling the pH and ethanol content, semi-purified fractions of plasma proteins can be produced. One of the last proteins to precipitate in the Cohn process is native human serum albumin. After precipitation, a wet paste of crude native human serum albumin is obtained. Subsequent bioprocessing steps (purification, filtration, pasteurization, etc.) can be used to produce a purified, stabilized form of native human serum albumin for commercial use (Lin JJ et al., Pharmaceutical Research 2000; 17:391-6). Recombinant human serum albumin is a highly purified animal-, virus-, and prion-free product as alternative to native human serum albumin, to which it is structurally equivalent (Bosse D et al., J. Clin. Pharmacol. 2005; 45:57-67). Recombinant human serum albumin has been produced by various hosts, both prokaryotic and eukaryotic (Chen Z et al., Biochimica et Biophysica Acta 2013; 1830:5515-5525). A fatty acid free human serum albumin can be prepared by treatment of human serum albumin with charcoal at low pH. Likewise, treatment of human serum albumin with charcoal at low pH can be used to remove fatty acids from human serum albumin (Chen RF, J. Biol. Chem. 1967; 242:173-181). Human serum albumin (HSA) is a highly soluble globular protein of Mr 65K and consists of 585 amino acids. HSA is the most abundant protein in the plasma and accounts for 70-80% of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (see, e.g., Tullis, JAMA, 237, 355-360, 460-463, (1977) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Human serum albumin (HSA) has multiple hydrophobic binding sites (a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (1981), Vorum, Dan. Med. Bull., 46, 379-99 (1999), Kragh-Hansen, Dan. Med Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (1992), and Carter et al., Adv. Protein. Chem., 45, 153-203 (1994)).

In some embodiments, the human serum albumin is a native human serum albumin. In some embodiments, the human serum albumin is a recombinant human serum albumin. In some embodiments, the human serum albumin is a fatty acid free human serum albumin. In some embodiments, the human serum albumin is essentially fatty acid free.

As used herein, the term "essentially fatty acid free" refers to proteins (e.g. serum albumin) that contain less than about 0.02% fatty acid by weight. For example, human serum albumin that is essentially fatty acid free can contain less than 0.02% fatty acid by weight. In some embodiments, the human serum albumin contains no more than 2, 1, 0.5, 0.1, 0.05, 0.01, 0.001, 0.0005, or 0.0001 moles of fatty acids bound to one mole of human serum albumin.

As used herein, the term "fatty acids" refers to non-esterified fatty acids (e.g. linoleic acid, $\alpha$-linoleic acid, $\gamma$-linoleic acid).

Solutions of human serum albumin for infusion are commercially available. Those solutions must be supplemented with stabilizers to allow pasteurization and storage, to avoid the spontaneous polymerization of the albumin. Usually, N-acetyltryptophanate and caprylic acid or their sodium salts are used in alone or in combination.

In some embodiments, the human serum albumin is a commercially available solution of human serum albumin USP for infusion. In some embodiments, the solution of human serum albumin USP for infusion is 5% solution of human serum albumin USP (w/v). In some embodiments, the solution of human serum albumin USP for infusion is 20% solution

of human serum albumin USP (w/v). In some embodiments, the solution of human serum albumin USP for infusion is 25% solution of human serum albumin USP (w/v).

In some embodiments, the composition comprises at least one stabilizer for the human serum albumin. In some embodiments, the composition comprises two stabilizers for the human serum albumin. In some embodiments, the stabilizers are N-acetyltryptophanate and caprylic acid or sodium salt thereof. In some embodiments, the stabilizer is N-acetyltryptophanate or sodium salt thereof. In some embodiments, the stabilizer is caprylic acid or sodium salt thereof.

As used herein the term "aprepitant" is a compound that has the CAS No. 170729-80-3 and the following chemical structure:

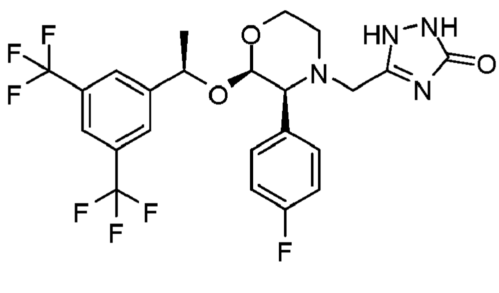

Aprepitant is a white to off-white crystalline solid, with a molecular weight of 534.43. It is practicallyinsoluble in water. Aprepitant is sparingly soluble in ethanol and isopropyl acetate and slightly soluble inacetonitrile.

Aprepitant (EMEND) is a substance P/neurokinin 1 (NK1) receptor antagonist, indicated in combination with other antiemetic for prevention of acute and delayed nausea and vomiting associated with initial and repeat courses of highly emetogenic cancer chemotherapy (HEC) including high-dose cisplatin, and nausea and vomiting associated with initial and repeat courses of moderately emetogenic cancer chemotherapy (MEC). Aprepitant is also indicated for prevention of postoperative nausea and vomiting (PONV) in adults.

In some embodiments, the aprepitant can be a pharmaceutically acceptable salt of fosaprepitant. In some embodiments, the term "pharmaceutically acceptable salts" refers to salts that retain the desired biological activity of the subject compound and exhibit minimal undesired toxicological effects. These pharmaceutically acceptable salts may be prepared in situ during the final isolation and purification of the compound, or by separately reacting the purified compound in its free acid or free base form with a suitable base or acid, respectively. In some embodiments, pharmaceutically acceptable salts may be preferred over the respective free base or free acid because such salts impart greater stability or solubility to the molecule thereby facilitating formulation into a dosage form. Basic compounds are generally capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate),napthalene-2-sulfonate, ethanedisulfonate, hydrogen bisulfide, bitartrate, gluconate, glucuronate, para-bromophenylsulfonate, carbonate, pyrosulfate, sulfite, bisulfite, monohydrogen phosphate, dihydrogen phosphate, metaphosphate, pyrophosphate, chloride, bromide, iodide, decanoate, caprylate, caprate, propiolate, suberate, sebacate, butyne-1,4-dioate, hexyne-1,6-dioate, terephthalate, sulfonate, xylenesulfonate, phenylpropionate, phenylbutyrate, β-hydroxybutyrate, glycolate, propanesulfonate, naphthalene-1-sulfonate, naphthalene-2-sulfonate and 2,5-dihydroxybenzoate. Suitable bases include pharmaceutically acceptable inorganic bases and pharmaceutically acceptable organic bases. Representative pharmaceutically acceptable base addition salts include hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-($C_1$-$C_6$)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; aminosugars such as meglumine, and amino acids such as arginine, lysine, and the like.

In some embodiments, the composition is a solid formulation. For example, the solid formulation can be produced in a uniform manner by lyophilization. A skilled artisan would recognize other methods, such as rotary evaporation, that can also produce solid formulations.

In some embodiments, the aprepitant and the human serum albumin in the solid formulation are bound non-covalently. In some embodiments, the solid formulation comprises a non-covalently bond complex comprising aprepitant and the human serum albumin. In some embodiments, "non-covalent" refers to an interaction between two or more components, wherein the bonds between the components are non-covalent bonds (i.e., no atom of one component shares a pair of electrons with an atom of another component; e.g., weak bonds such as hydrogen bonds, electrostatic effects, $\pi$-effects, hydrophobic effects and Van der Waals forces). Further, human serum albumin (HSA) has multiple hydrophobic binding sites (a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Additionally, after the drug molecule binds to HSA, the drug molecule and HSA form a non-covalently bound drug and protein complex through the binding sites of HSA. This concept is commonly understood by one of ordinary skill in the art to which this disclosure belongs. One example of a non-covalently bound complex is a non-covalently bound complex of HSA and fatty acids, in which the fatty acids bind to HSA through HSA's multiple binding sites.

In some embodiments, the non-covalent interaction between aprepitant and human serum albumin comprises hydrogen bonding. In some embodiments, the non-covalent interaction between aprepitant and human serum albumin comprises electrostatic interaction. In some embodiments, the non-covalent interaction between aprepitant and human serum albumin comprises hydrophobic interaction. In some embodiments, the non-covalent interaction between aprepitant and human serum albumin comprises Van der Waals forces. In some embodiments, the non-covalent interaction between aprepitant and human serum albumin comprises hydrogen bonding, electrostatic interaction, hydrophobic interaction, and Van der Waals forces.

In some embodiments, the composition is an aqueous formulation. In some embodiments, the aqueous formulation is substantially free of solvent other than water. In some embodiments, the aqueous formulation is free of solvent other than water.

As used herein, "substantially free of solvent," in reference to an aqueous solution, refers to an aqueous solution that contains less than 0.5 %, by weight, of any non-water solvent. In some embodiments, the aqueous solution contains less than 0.1%, by weight, of any non-water solvent.

In some embodiments, the aqueous formulation is a clear aqueous solution. For example, the formulation can be a clear and stable aqueous solution reconstituted from a sterile lyophilized powder. In some embodiments, the aqueous formulation is a clear aqueous solution, wherein the aqueous formulation is substantially free of solvent other than water. In some embodiments, the aqueous formulation is a clear aqueous solution, wherein the aqueous formulation is free of solvent other than water.

In some embodiments, the aqueous formulation is a clear aqueous solution reconstituted from the solid formulation (e.g. the sterile lyophilized powder) in water. In some embodiments, the aqueous formulation is a clear aqueous solution reconstituted from the solid formulation (e.g. the sterile lyophilized powder) in 0.9% saline. In some embodiments, the aqueous formulation is a clear aqueous solution reconstituted from the solid formulation (e.g. the sterile lyophilized powder) in 5% Dextrose solution.

In some embodiments, the aqueous formulation is a clear aqueous solution reconstituted from the solid formulation (e.g. the sterile lyophilized powder) in water, wherein the aqueous formulation has pH value from about 5 to about 8. In some embodiments, the aqueous formulation is a clear aqueous solution reconstituted from the solid formulation (e.g. the sterile lyophilized powder) in 0.9% saline, wherein the aqueous formulation has pH value from about 5 to about 8. In some embodiments, the aqueous formulation is a clear aqueous solution reconstituted from the solid formulation (e.g. the sterile lyophilized powder) in 5% Dextrose solution, wherein the aqueous formulation has pH value from about 5 to about 8.

In some embodiments, the aqueous formulation is a clear aqueous solution reconstituted from the solid formulation (e.g. the sterile lyophilized powder) in water, wherein the aqueous formulation has pH value from about 6 to about 7.5. In some embodiments, the aqueous formulation is a clear aqueous solution reconstituted from the solid formulation (e.g. the sterile lyophilized powder) in 0.9% saline, wherein the aqueous formulation has pH value from about 6 to about 7.5. In some embodiments, the aqueous formulation is a clear aqueous solution reconstituted from the solid formulation (e.g. the sterile lyophilized powder) in 5% Dextrose solution, wherein the aqueous formulation has pH value from about 6 to about 7.5.

In some embodiments, the aqueous formulation has pH value from about 5 to about 8. In some embodiments, the aqueous formulation has pH value from about 5.5 to about 7.8. In some embodiments, the aqueous formulation has pH value from about 6 to about 7.5. In some embodiments, the aqueous formulation has pH value from about 6.5 to about 7.5. In some embodiments, the aqueous formulation has pH value from about 6 to about 6.5. In some embodiments, the aqueous formulation has pH value from about 6.5 to about 7. In some embodiments, the aqueous formulation has pH value from about 7 to about 7.5. In some embodiments, the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the aqueous formulation is substantially free of solvent other

than water. In some embodiments, the aqueous formulation is free of solvent other than water.

In some embodiments, the aqueous formulation is a clear aqueous solution, wherein the aqueous formulation has pH value from about 5 to about 8, and wherein the aqueous formulation is substantially free of solvent other than water. In some embodiments, the aqueous formulation is a clear aqueous solution, wherein the aqueous formulation has pH value from about 5 to about 8, and wherein the aqueous formulation is free of solvent other than water. In some embodiments, the aqueous formulation is a clear aqueous solution, wherein the aqueous formulation has pH value from about 5.5 to about 7.8, and wherein the aqueous formulation is substantially free of solvent other than water. In some embodiments, the aqueous formulation is a clear aqueous solution, wherein the aqueous formulation has pH value from about 5.5 to about 7.8, and wherein the aqueous formulation is free of solvent other than water. In some embodiments, the aqueous formulation is a clear aqueous solution, wherein the aqueous formulation has pH value from about 6 to about 7.5, and wherein the aqueous formulation is substantially free of solvent other than water. In some embodiments, the aqueous formulation is a clear aqueous solution, wherein the aqueous formulation has pH value from about 6 to about 7.5, and wherein the aqueous formulation is free of solvent other than water. In some embodiments, the aqueous formulation is a clear aqueous solution, wherein the aqueous formulation has pH value from about 6.5 to about 7.5, and wherein the aqueous formulation is substantially free of solvent other than water. In some embodiments, the aqueous formulation is a clear aqueous solution, wherein the aqueous formulation has pH value from about 6.5 to about 7.5, and wherein the aqueous formulation is free of solvent other than water. In some embodiments, the aqueous formulation is a clear aqueous solution, wherein the aqueous formulation has pH value from about 6 to about 6.5, and wherein the aqueous formulation is substantially free of solvent other than water. In some embodiments, the aqueous formulation is a clear aqueous solution, wherein the aqueous formulation has pH value from about 6 to about 6.5, and wherein the aqueous formulation is free of solvent other than water. In some embodiments, the aqueous formulation is a clear aqueous solution, wherein the aqueous formulation has pH value from about 6.5 to about 7, and wherein the aqueous formulation is substantially free of solvent other than water. In some embodiments, the aqueous formulation is a clear aqueous solution, wherein the aqueous formulation has pH value from about 6.5 to about 7, and wherein the aqueous formulation is free of solvent other than water. In some embodiments, the aqueous formulation is a clear aqueous solution, wherein the aqueous formulation has pH value from about 7 to about 7.5, and wherein the aqueous formulation is substantially free of solvent other than water. In some embodiments, the aqueous formulation is a clear aqueous solution, wherein the aqueous formulation has pH value from about 7 to about 7.5, and wherein the aqueous formulation is free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5, and wherein the aqueous formulation is substantially free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5, and wherein the aqueous formulation is free of solvent other than water.

In some embodiments, the aqueous formulation is a clear aqueous solution. In some embodiments, the aqueous formulation is a clear aqueous solution for at least 1 hour. In some embodiments, the aqueous formulation is a clear aqueous solution for at least 2 hours. In some embodiments, the aqueous formulation is a clear aqueous solution for at least 3 hours. In some embodiments, the aqueous formulation is a clear aqueous solution for at least 4 hours. In some embodiments, the aqueous formulation is a clear aqueous solution for at least 5 hours. In some embodiments, the aqueous formulation is a clear aqueous solution for at least 6 hours. In some embodiments, the aqueous formulation is a clear aqueous solution for at least 8 hours. In some embodiments, the aqueous formulation is a clear aqueous solution for at least 24 hours.

As used herein, the term "clear aqueous solution" refers to an aqueous solution that is transparent and free of visible particles or precipitation upon visual observation.

When visually observed, for example, the term "clear aqueous solution" excludes a milky aqueous solution. Further, the term "clear aqueous solution" excludes a cloudy or hazy aqueous solution.

In some embodiments, the aqueous solution is a clear aqueous solution for at least 1 hour. In some embodiments, the aqueous formulation is a clear aqueous solution for at least 2 hours. In some embodiments, the aqueous formulation is a clear aqueous solution for at least 3 hours. In some embodiments, the aqueous formulation is a clear aqueous solution for at least 6 hours. In some embodiments, the aqueous formulation is a clear aqueous solution for at least 8 hours. In some embodiments, the aqueous formulation is a clear aqueous solution for a period of time selected from 1 hour. 2 hours, 3 hours, 4 hours, 5 hours, 6 hours, 7 hours, 8 hours, 12 hours, and 24 hours. In some embodiments, the aqueous formulation is a clear aqueous solution for at least 24 hours.

In some embodiments, after aqueous formulation is filtered by a 0.22 micron filter, the amount of aprepitant in the filtered aqueous solution is at least 95% of the total amount of aprepitant in the aqueous solution before filtration. In some embodiments, after aqueous formulation is filtered by a 0.22 micron filter, the amount of aprepitant in the filtered aqueous solution is at least 96% of the total amount of aprepitant in the aqueous solution before filtration. In some

embodiments, after aqueous formulation is filtered by a 0.22 micron filter, the amount of aprepitant in the filtered aqueous solution is at least 97% of the total amount of aprepitant in the aqueous solution before filtration. In some embodiments, after aqueous formulation is filtered by a 0.22 micron filter, the amount of aprepitant in the filtered aqueous solution is at least 98% of the total amount of aprepitant in the aqueous solution before filtration. In some embodiments, after aqueous formulation is filtered by a 0.22 micron filter, the amount of aprepitant in the filtered aqueous solution is at least 99% of the total amount of aprepitant in the aqueous solution before filtration. In some embodiments, the aqueous formulation is filtered by a 0.22 micron filter in a period of time selected from 1 hour. 2 hours, 3 hours, 4 hours, 5 hours, 6 hours, 7 hours, 8 hours, 12 hours, and 24 hours.

The amount of the aprepitant in an aqueous solution can be measured by the methods using HPLC. The methods of measuring the amount of the aprepitant in an aqueous solution are illustrated in the experimental examples described herein. The methods are commonly understood by one of ordinary skill in the art to which this disclosure belongs.

As used herein, the term "micron" refers to a unit of measure of one one-thousandth of a millimeter.

Also, provided herein is a pharmaceutical composition comprising the composition comprising the aprepitant and the human serum albumin as described herein, and a pharmaceutically acceptable carrier. In some embodiments, the pharmaceutical composition comprising the composition comprising the aprepitant and the human serum albumin as described herein, and a pharmaceutically acceptable carrier, is a liquid pharmaceutical composition. In some embodiments, the liquid pharmaceutical composition is an aqueous solution. In some embodiments, the liquid pharmaceutical composition is free of solvent other than water. In some embodiments, the liquid pharmaceutical composition is substantially free of solvent other than water. In some embodiments, the liquid pharmaceutical composition is an injectable pharmaceutical formulation. In some embodiments, the liquid pharmaceutical composition is a formulation for infusion (e.g., intravenous infusion).

As used herein, the term "aqueous solution" refers to a solution, wherein at least one solvent is water and the weight % of water in the mixture of solvents is at least 50%, at least 60%, at least 70%, at least 90%, at least 95%, or at least 99%. In some embodiments, aqueous solution is a solution in which water is the only solvent. As used herein, the term "aqueous solvent" refer to a liquid comprising at least 50%, at least 60%, at least 70%, at least 90% or at least 95% water. In some embodiments, aqueous solvent is water.

In some embodiments, the pharmaceutically acceptable carrier is refers to any carrier useful to solubilize and deliver an agent to a subject. A desirable pharmaceutically acceptable carrier is saline. Other pharmaceutically acceptable carrier and their formulation are known to one skilled in the art and described, for example, in Remington's Pharmaceutical Sciences. (20th edition), ed. A. Gennaro, 2003, Lippincon Williams & Wilkins. In some embodiments, the carrier may contain components such as, for example, dextrose, glucose, serum proteins (other than HSA), buffer substances such as phosphates, glycine, sorbic acid, potassium sorbate, salts or electrolytes, such as protamine sulfate, disodium hydrogen phosphate, potassium hydrogen phosphate, sodium chloride, zinc salts, and cellulose-based substances. In some embodiments, the carrier may contain components such as contain anti-oxidants, buffers, bacteriostats and solutes which render the formulation isotonic with the blood of the intended recipient.

In some embodiments, the pharmaceutically acceptable excipient is selected from lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The pharmaceutical compositions may additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The pharmaceutical composition may be formulated so as to provide quick, sustained or delayed release of the active ingredient after administration to the patient by employing procedures known in the art.

In some embodiments, the pharmaceutical composition of the present disclosure may be administered by a syringe or a catheter, or any other means generally known in the art for the delivery of a pharmaceutical agent by injection to the subject in need thereof. The delivery means will vary, as recognized by those skilled in the art, depending on the diseases and conditions treated, the severity of the disease, the sex, age and general health condition of the subject, excipient usage, the possibility of co-usage with other therapeutic treatments such as use of other agents as described herein and the judgment of the treating physician.

The pharmaceutical composition comprising the composition comprising the aprepitant and the human serum albumin as described herein can be administered to a subject via various routes, such as parenterally, including intravenous, intra-arterial, intraperitoneal, intrapulmonary, oral, inhalation, intravascular, intramuscular, intra-tracheal, subcutaneous, intraocular, intrathecal, or transdermal. For example, the composition can be administered by inhalation to treat conditions of the respiratory tract. In some embodiments, the pharmaceutical composition is administrated in a unit dosage form such as capsule or oral suspension (e.g., for pediatric patients 6 months to less than 12 years of age).

In some embodiments, the pharmaceutical composition is free of a surfactant, such as CREMOPHOR® surfactants and Polysorbate 80. In some embodiments, the pharmaceutical composition is substantially free of a surfactant, such as CREMOPHOR® surfactants and Polysorbate 80. As used herein, the term "substantially free of surfactant" refers to a formulation containing less than 0.0005%, less than 0.0003%, or less than 0.0001% of surfactants and/or

less than 0.0005%, less than 0.0003%, or less than 0.0001% of surfactant.

Also, provided herein is a method for the prevention of chemotherapy-induced nausea and vomiting, the method comprising the step of administering to a subject in need thereof of a therapeutically effective amount of a pharmaceutical composition comprising the composition comprising the aprepitant and the human serum albumin as described herein, and a pharmaceutically acceptable carrier. In some embodiments, the methods described herein are for the prevention of acute and delayed nausea and vomiting associated with initial and repeat courses of highly emetogenic cancer chemotherapy (HEC) including high-dose cisplatin. In some embodiments, the methods described herein are for the prevention of nausea and vomiting associated with initial and repeat courses of moderately emetogenic cancer chemotherapy (MEC). In some embodiments, the methods described herein are for the prevention of postoperative nausea and vomiting (PONV).

Also, provided herein is a method of treating chemotherapy-induced nausea and vomiting, the method comprising the step of administering to a subject in need thereof of a therapeutically effective amount of a pharmaceutical composition comprising the composition comprising the aprepitant and the human serum albumin as described herein, and a pharmaceutically acceptable carrier. In some aspects of these embodiments, the chemotherapy-induced nausea and vomiting is acute and delayed nausea and vomiting associated with initial and repeat courses of highly emetogenic cancer chemotherapy (HEC) including high-dose cisplatin. In other aspects of these embodiments, the chemotherapy-induced nausea and vomiting is nausea and vomiting associated with initial and repeat courses of moderately emetogenic cancer chemotherapy (MEC). In yet other aspects of these embodiments, the chemotherapy-induced nausea and vomiting is postoperative nausea and vomiting (PONV) in adults.

In some embodiments, the methods described herein are performed in combination with at least one other antiemetic agents. In some embodiments, the methods described herein are performed in combination with dexamethasone and a 5-$HT_3$ antagonist (e.g., tropisetron, palonosetron, ramosetron, granisetron, ondansetron, dolasetron, or metoclopramide). In some embodiments, the antiemetic agent is selected from tropisetron, palonosetron, ramosetron, granisetron, ondansetron, dolasetron, metoclopramide, domperidone, olanzapine, droperidol, haloperidol, chlorpromazine, prochlorperazine, alizapride, prochlorperazine, metoclopramide, casopitant, aprepitant, cyclizine, diphenhydramine, dimenhydrinate, doxylamine, meclizine, promethazine, hydroxyzine, dronabinol, sativex, midazolam, lorazepam, hyoscine, trimethobenzamide, emetrol, propofol and muscimol.

In some embodiments, provided herein is a method for treating chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC), or postoperative nausea and vomiting (PONV) in adults), the method comprising administering to a subject in need thereof a therapeutically effective amount a pharmaceutical composition comprising the composition comprising the aprepitant and the human serum albumin as described herein, and a therapeutically effective amount of at least one antiemetic agent as described herein.

In some embodiments, provided herein is a method for preventing chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC), or postoperative nausea and vomiting (PONV) in adults), the method comprising administering to a subject in need thereof a therapeutically effective amount a pharmaceutical composition comprising the composition comprising the aprepitant and the human serum albumin as described herein, and a therapeutically effective amount of at least one antiemetic agent as described herein.

In some embodiments, a pharmaceutical composition comprising the composition comprising the aprepitant and the human serum albumin as described herein and an antiemetic agent are administered simultaneously.

In some embodiments, a pharmaceutical composition comprising the composition comprising the aprepitant and the human serum albumin as described herein and an antiemetic agent are administered consecutively.

Also, provided herein is a pharmaceutical composition comprising the composition comprising the aprepitant and the human serum albumin as described herein, at least one antiemetic agent as described herein, and a pharmaceutically acceptable carrier.

In some embodiments, provided herein is a pharmaceutical composition comprising the composition comprising the aprepitant and the human serum albumin as described herein, a 5-$HT_3$ antagonist (e.g., tropisetron, palonosetron, ramosetron, granisetron, ondansetron, dolasetron, or metoclopramide), and a pharmaceutically acceptable carrier.

In some embodiments, provided herein is a pharmaceutical composition comprising the composition comprising the aprepitant and the human serum albumin as described herein, palonosetron, and a pharmaceutically acceptable carrier.

In some embodiments, provided herein is a pharmaceutical composition comprising the composition comprising the aprepitant and the human serum albumin as described herein, 0.5mg of palonosetron, and a pharmaceutically acceptable carrier.

In some embodiments, provided herein is a pharmaceutical composition comprising the composition comprising

the aprepitant and the human serum albumin as described herein, 0.25mg of palonosetron, and a pharmaceutically acceptable carrier.

In some embodiments, provided herein is a pharmaceutical composition comprising the composition comprising the aprepitant and the human serum albumin as described herein, 0.28mg of palonosetron hydrochloride, and a pharmaceutically acceptable carrier.

In some embodiments, provided herein is a pharmaceutical composition comprising the composition comprising the aprepitant and the human serum albumin as described herein, about 0.25mg of palonosetron, and a pharmaceutically acceptable carrier.

In some embodiments, provided herein is a pharmaceutical composition comprising the composition comprising the aprepitant and the human serum albumin as described herein, about 0.28mg of palonosetron hydrochloride, and a pharmaceutically acceptable carrier.

In some embodiments, provided herein is a liquid pharmaceutical formulation for injection comprising the composition comprising the aprepitant and the human serum albumin as described herein, and a pharmaceutically acceptable carrier.

In some embodiments, provided herein is a liquid pharmaceutical formulation for injection comprising the composition comprising the aprepitant and the human serum albumin as described herein, 0.25mg of palonosetron, and a pharmaceutically acceptable carrier.

In some embodiments, provided herein is a liquid pharmaceutical formulation for injection comprising the composition comprising the aprepitant and the human serum albumin as described herein, 0.28mg of palonosetron hydrochloride, and a pharmaceutically acceptable carrier.

In some embodiments, the liquid pharmaceutical formulation for injection is an aqueous solution. In some embodiments, the liquid pharmaceutical formulation for injection is free of solvent other than water. In some embodiments, the liquid pharmaceutical formulation for injection is substantially free of solvent other than water. In some embodiments, the liquid pharmaceutical formulation for injection is a formulation for infusion (e.g., intravenous infusion).

In some embodiments, the liquid pharmaceutical formulation for injection has pH value from about 5 to about 8. In some embodiments, the liquid pharmaceutical formulation for injection has pH value from about 5.5 to about 7.8. In some embodiments, the liquid pharmaceutical formulation for injection has pH value from about 6 to about 7.5. In some embodiments, the liquid pharmaceutical formulation for injection has pH value from about 6.5 to about 7.5. In some embodiments, the liquid pharmaceutical formulation for injection has pH value from about 6 to about 6.5. In some embodiments, the liquid pharmaceutical formulation for injection has pH value from about 6.5 to about 7. In some embodiments, the liquid pharmaceutical formulation for injection has pH value from about 7 to about 7.5. In some embodiments, the liquid pharmaceutical formulation for injection has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the liquid pharmaceutical formulation for injection is substantially free of solvent other than water. In some embodiments, the liquid pharmaceutical formulation for injection is free of solvent other than water.

In some embodiments, provided herein is a pharmaceutical composition comprising the composition comprising the aprepitant and the human serum albumin as described herein, granisetron, and a pharmaceutically acceptable carrier.

In some embodiments, provided herein is a pharmaceutical composition comprising the composition comprising the aprepitant and the human serum albumin as described herein, ondansetron, and a pharmaceutically acceptable carrier.

The methods described herein may be performed alone or in conjunction with another therapy, such as surgery, radiation, chemotherapy, immunotherapy, gene therapy, and the like.

In some embodiments, the amount of aprepitant that is administered to a subject in need thereof with any one of pharmaceutical compositions described herein is from about 50 mg to about 250 mg, from about 60 mg to about 240 mg, from about 70 mg to 230 mg, from about 80 mg to about 220 mg, from about 90 mg to about 210 mg, from about 100 mg to about 200 mg, or from about 100 mg to about 150 mg. In some embodiments, the amount of aprepitant that is administered to a subject in need thereof with any one of pharmaceutical compositions described herein is about 100 mg, about 125 mg, about 150 mg, about 175 mg, or about 200 mg. In some embodiments, the amount of aprepitant that is administered to a subject in need thereof with any one of pharmaceutical compositions described herein is about 125 mg. In some embodiments, the amount of aprepitant is administered as capsules or oral suspensions. In some embodiments, 125 mg of aprepitant is administered on day 1 and 80 mg of aprepitant on days 2 and 3.

As will be understood by those of ordinary skill in the art, the appropriate doses of aprepitant will be approximately those already employed in clinical therapies wherein fosaprepitant is administered alone or in combination with other therapeutic agents. Variation in dosage will likely occur depending on the condition being treated. Appropriate effective doses will also vary, as recognized by those skilled in the art, depending on the severity of the disease, the route of administration, the sex, age and general health condition of the subject, excipient usage, the possibility of co-usage with other therapeutic treatments such as use of other agents, and the judgment of the treating physician. For example,

guidance for selecting an effective dose can be determined by reference to the prescribing information for aprepitant.

**Composition comprising fosaprepitant, or a pharmaceutically acceptable salt thereof, aprepitant and human serum albumin.**

Also, provided herein is a composition comprising fosaprepitant, or a pharmaceutically acceptable salt thereof, aprepitant and human serum albumin. In some embodiments, the fosaprepitant, or a pharmaceutically acceptable salt thereof, and the human serum albumin in the composition have a ratio by weight from about 1:0.1 to about 1:500.

In some embodiments, the aprepitant and the fosaprepitant, or a pharmaceutically acceptable salt thereof, in the composition have a ratio by weight no more than 1:10, no more than 1:20, no more than 1:25, no more than 3:100, no more than 1:50, no more than 1:100, no more than 1:200, no more than 1:500, or no more than 1:1000. In some embodiments, the weight ratio of fosaprepitant, or a pharmaceutically acceptable salt thereof, to aprepitant in the composition is from about 1000:1 to about 20:1, from about 1000:1 to about 50:1, from about 1000:1 to about 70:1, or from about 1000:1 to about 100:1.

In some embodiments, the molar ratio of fosaprepitant, or a pharmaceutically acceptable salt thereof, to aprepitant in the composition is from about 1000:1 to about 10:1, from about 1000 to about 20:1, from about 1000:1 to about 50:1, or from about 1000:1 to about 100:1.

Fosaprepitant easily degrades to aprepitant unless stored at low temperature. Degradation is enhanced by the presence of water. In some embodiments, all or part of aprepitant in the composition is degraded from fosaprepitant, or a pharmaceutically acceptable salt thereof. In some embodiments, all aprepitant in the composition is degraded from fosaprepitant, or a pharmaceutically acceptable salt thereof. In some embodiments, part of aprepitant in the composition is degraded from fosaprepitant, or a pharmaceutically acceptable salt thereof.

In some embodiments, the fosaprepitant, or the pharmaceutically acceptable salt thereof, and the human serum albumin in the composition have a ratio by weight from about 1:0.5 to about 1:300, from about 1:0.8 to about 1:200, from about 1:1 to about 1:150, from about 1:2 to about 1:100, from about 1:2 to about 1:80, from about 1:2 to about 1:50, from about 1:2.5 to about 1:30, from about 1:3 to about 1:20, or from about 2:1 to about 1:20. In some embodiments, the fosaprepitant, or a pharmaceutically acceptable salt thereof, and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, human serum albumin refers to native and recombinant human serum albumin. Native human serum albumin and other plasma proteins can be precipitated from human plasma by varying the pH and adding ethanol, in what is known as the Cohn fractionation process (Cohn EJ et al., J. Am. Chem. Soc. 1946; 68:459-475). By controlling the pH and ethanol content, semi-purified fractions of plasma proteins can be produced. One of the last proteins to precipitate in the Cohn process is native human serum albumin. After precipitation, a wet paste of crude native human serum albumin is obtained. Subsequent bioprocessing steps (purification, filtration, pasteurization, etc.) can be used to produce a purified, stabilized form of native human serum albumin for commercial use (Lin JJ et al., Pharmaceutical Research 2000; 17:391-6). Recombinant human serum albumin is a highly purified animal-, virus-, and prion-free product as alternative to native human serum albumin, to which it is structurally equivalent (Bosse D et al., J. Clin. Pharmacol. 2005; 45:57-67). Recombinant human serum albumin has been produced by various hosts, both prokaryotic and eukaryotic (Chen Z et al., Biochimica et Biophysica Acta 2013; 1830:5515-5525). A fatty acid free human serum albumin can be prepared by treatment of human serum albumin with charcoal at low pH. Likewise, treatment of human serum albumin with charcoal at low pH can be used to remove fatty acids from human serum albumin (Chen RF, J. Biol. Chem. 1967; 242:173-181). Human serum albumin (HSA) is a highly soluble globular protein of Mr 65K and consists of 585 amino acids. HSA is the most abundant protein in the plasma and accounts for 70-80% of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (see, e.g., Tullis, JAMA, 237, 355-360, 460-463, (1977) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Human serum albumin (HSA) has multiple hydrophobic binding sites (a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (1981), Vorum, Dan. Med. Bull., 46, 379-99 (1999), Kragh-Hansen, Dan. Med Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (1992), and Carter et al.,

Adv. Protein. Chem., 45, 153-203 (1994)).

In some embodiments, the human serum albumin is a native human serum albumin. In some embodiments, the human serum albumin is a recombinant human serum albumin. In some embodiments, the human serum albumin is a fatty acid free human serum albumin. In some embodiments, the human serum albumin is essentially fatty acid free.

As used herein, the term "essentially fatty acid free" refers to proteins (e.g. serum albumin) that contain less than about 0.02% fatty acid by weight. For example, human serum albumin that is essentially fatty acid free can contain less than 0.02% fatty acid by weight. In some embodiments, the human serum albumin contains no more than 2, 1, 0.5, 0.1, 0.05, 0.01, 0.001, 0.0005, or 0.0001 moles of fatty acids bound to one mole of human serum albumin.

As used herein, the term "fatty acids" refers to non-esterified fatty acids (e.g. linoleic acid, α-linoleic acid, γ-linoleic acid).

Solutions of human serum albumin for infusion are commercially available. Those solutions must be supplemented with stabilizers to allow pasteurization and storage, to avoid the spontaneous polymerization of the albumin. Usually, N-acetyltryptophan and caprylic acid or their sodium salts are used in alone or in combination.

In some embodiments, the human serum albumin is a commercially available solution of human serum albumin USP for infusion. In some embodiments, the solution of human serum albumin USP for infusion is 5% solution of human serum albumin USP (w/v). In some embodiments, the solution of human serum albumin USP for infusion is 20% solution of human serum albumin USP (w/v). In some embodiments, the solution of human serum albumin USP for infusion is 25% solution of human serum albumin USP (w/v). In some embodiments, the human serum albumin is an aqueous solution prepared by diluting a commercially available solution of human serum albumin USP for infusion with saline. In some embodiments, the human serum albumin is an aqueous solution prepared by diluting a commercially available solution of human serum albumin USP for infusion with water.

In some embodiments, the composition comprises at least one stabilizer for the human serum albumin. In some embodiments, the composition comprises two stabilizers for the human serum albumin. In some embodiments, the stabilizers are N-acetyltryptophan and caprylic acid or sodium salt thereof. In some embodiments, the stabilizer is N-acetyltryptophan or sodium salt thereof. In some embodiments, the stabilizer is caprylic acid or sodium salt thereof.

In some embodiments, the fosaprepitant, or a pharmaceutically acceptable salt thereof, can be fosaprepitant dimeglumine, which is a dimeglumine salt of fosaprepitant (as described, e.g., in the "compositions of fosaprepitant, or a pharmaceutically acceptable salt thereof, and human serum albumin" section).

In some embodiments, aprepitant is as described in "compositions comprising aprepitant and human serum albumin" section.

In some embodiments, the fosaprepitant dimeglumine and the human serum albumin in the composition have a ratio by weight from about 1:0.2 to about 1:300. In some embodiments, the fosaprepitant dimeglumine and the human serum albumin in the composition have a ratio by weight from about 1:0.5 to about 1:150. In some embodiments, the fosaprepitant dimeglumine and the human serum albumin in the composition have a ratio by weight from about 1:0.8 to about 1:120. In some embodiments, the fosaprepitant dimeglumine and the human serum albumin in the composition have a ratio by weight from about 1:1 to about 1:100. In some embodiments, the fosaprepitant dimeglumine and the human serum albumin in the composition have a ratio by weight from about 1:1.2 to about 1:70. In some embodiments, the fosaprepitant dimeglumine and the human serum albumin in the composition have a ratio by weight from about 1:1.5 to about 1:50. In some embodiments, the fosaprepitant dimeglumine and the human serum albumin in the composition have a ratio by weight from about 1:1.8 to about 1:20. In some embodiments, the fosaprepitant dimeglumine and the human serum albumin in the composition have a ratio by weight from about 1:2 to about 1:10. In some embodiments, the fosaprepitant dimeglumine and the human serum albumin in the composition have a ratio by weight from about 2:1 to about 1:20. In some embodiments, the fosaprepitant dimeglumine and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the fosaprepitant and the human serum albumin in the composition are bound non-covalently. In some embodiments, the composition comprises a non-covalently bond complex comprising fosaprepitant and the human serum albumin.

In some embodiments, the aprepitant and the human serum albumin in the composition are bound non-covalently. In some embodiments, the composition comprises a non-covalently bond complex comprising aprepitant and the human serum albumin.

In some embodiments, the fosaprepitant is bound non-covalently to the human serum albumin in the composition. In some embodiments, the aprepitant is bound non-covalently to the human serum albumin in the composition.

As used herein, the term "non-covalent" refers to an interaction between two or more components, wherein the bonds between the components are non-covalent bonds (i.e., no atom of one component shares a pair of electrons with an atom of another component; e.g., weak bonds such as hydrogen bonds, electrostatic effects, π-effects, hydrophobic effects and Van der Waals forces). Further, human serum albumin (HSA) has multiple hydrophobic binding sites

(a total of seven for medium and long-chain fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Additionally, after the drug molecule binds to HSA, the drug molecule and HSA form a non-covalently bound drug and protein complex through the binding sites of HSA. This concept is commonly understood by one of ordinary skill in the art to which this disclosure belongs. One example of a non-covalently bound complex is a non-covalently bound complex of HSA and fatty acids, in which the fatty acids bind to HSA through HSA's multiple binding sites.

In some embodiments, the non-covalent interaction between fosaprepitant and/or aprepitant, and human serum albumin comprises hydrogen bonding. In some embodiments, the non-covalent interaction between fosaprepitant and/or aprepitant, and human serum albumin comprises electrostatic interaction. In some embodiments, the non-covalent interaction between fosaprepitant and/or aprepitant, and human serum albumin comprises hydrophobic interaction. In some embodiments, the non-covalent interaction between fosaprepitant and/or aprepitant, and human serum albumin comprises Van der Waals forces. In some embodiments, the non-covalent interaction between fosaprepitant and/or aprepitant, and human serum albumin comprises hydrogen bonding, electrostatic interaction, hydrophobic interaction, and Van der Waals forces.

In some embodiments, the composition is a solid formulation. For example, the solid formulation can be produced in a uniform manner by lyophilization. A skilled artisan would recognize other methods, such as rotary evaporation, that can also produce solid formulations.

In some embodiments, the composition is an aqueous formulation. In some embodiments, the aqueous formulation is substantially free of solvent other than water. In some embodiments, the aqueous formulation is free of solvent other than water.

As used herein, "substantially free of solvent," in reference to an aqueous solution, refers to an aqueous solution that contains less than 0.5 %, by weight, of any non-water solvent. In some embodiments, the aqueous solution contains less than 0.1%, by weight, of any non-water solvent.

In some embodiments, the aqueous formulation is a clear aqueous solution reconstituted from the solid formulation (e.g. the sterile lyophilized powder) in water. In some embodiments, the aqueous formulation is a clear aqueous solution reconstituted from the solid formulation (e.g. the sterile lyophilized powder) in 0.9% saline. In some embodiments, the aqueous formulation is a clear aqueous solution reconstituted from the solid formulation (e.g. the sterile lyophilized powder) in 5% Dextrose solution.

In some embodiments, the aqueous formulation is a clear aqueous solution reconstituted from the solid formulation (e.g. the sterile lyophilized powder) in water, wherein the aqueous formulation has pH value from about 5 to about 8. In some embodiments, the aqueous formulation is a clear aqueous solution reconstituted from the solid formulation (e.g. the sterile lyophilized powder) in 0.9% saline, wherein the aqueous formulation has pH value from about 5 to about 8. In some embodiments, the aqueous formulation is a clear aqueous solution reconstituted from the solid formulation (e.g. the sterile lyophilized powder) in 5% Dextrose solution, wherein the aqueous formulation has pH value from about 5 to about 8.

In some embodiments, the aqueous formulation is a clear aqueous solution reconstituted from the solid formulation (e.g. the sterile lyophilized powder) in water, wherein the aqueous formulation has pH value from about 6 to about 7.5. In some embodiments, the aqueous formulation is a clear aqueous solution reconstituted from the solid formulation (e.g. the sterile lyophilized powder) in 0.9% saline, wherein the aqueous formulation has pH value from about 6 to about 7.5. In some embodiments, the aqueous formulation is a clear aqueous solution reconstituted from the solid formulation (e.g. the sterile lyophilized powder) in 5% Dextrose solution, wherein the aqueous formulation has pH value from about 6 to about 7.5.

In some embodiments, the aqueous formulation has pH value from about 5 to about 8. In some embodiments, the aqueous formulation has pH value from about 5.5 to about 7.8. In some embodiments, the aqueous formulation has pH value from about 6 to about 7.5. In some embodiments, the aqueous formulation has pH value from about 6.5 to about 7.5. In some embodiments, the aqueous formulation has pH value from about 6 to about 6.5. In some embodiments, the aqueous formulation has pH value from about 6.5 to about 7. In some embodiments, the aqueous formulation has pH value from about 7 to about 7.5. In some embodiments, the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the aqueous formulation is substantially free of solvent other than water. In some embodiments, the aqueous formulation is free of solvent other than water.

In some embodiments, the aqueous formulation is a clear aqueous solution, wherein the aqueous formulation has pH value from about 5 to about 8, and wherein the aqueous formulation is substantially free of solvent other than water. In some embodiments, the aqueous formulation is a clear aqueous solution, wherein the aqueous formulation has pH value from about 5 to about 8, and wherein the aqueous formulation is free of solvent other than water. In some embodiments, the aqueous formulation is a clear aqueous solution, wherein the aqueous formulation has pH value from about 5.5 to about 7.8, and wherein the aqueous formulation is substantially free of solvent other than water. In some

embodiments, the aqueous formulation is a clear aqueous solution, wherein the aqueous formulation has pH value from about 5.5 to about 7.8, and wherein the aqueous formulation is free of solvent other than water. In some embodiments, the aqueous formulation is a clear aqueous solution, wherein the aqueous formulation has pH value from about 6 to about 7.5, and wherein the aqueous formulation is substantially free of solvent other than water. In some embodiments, the aqueous formulation is a clear aqueous solution, wherein the aqueous formulation has pH value from about 6 to about 7.5, and wherein the aqueous formulation is free of solvent other than water. In some embodiments, the aqueous formulation is a clear aqueous solution, wherein the aqueous formulation has pH value from about 6.5 to about 7.5, and wherein the aqueous formulation is substantially free of solvent other than water. In some embodiments, the aqueous formulation is a clear aqueous solution, wherein the aqueous formulation has pH value from about 6.5 to about 7.5, and wherein the aqueous formulation is free of solvent other than water. In some embodiments, the aqueous formulation is a clear aqueous solution, wherein the aqueous formulation has pH value from about 6 to about 6.5, and wherein the aqueous formulation is substantially free of solvent other than water. In some embodiments, the aqueous formulation is a clear aqueous solution, wherein the aqueous formulation has pH value from about 6 to about 6.5, and wherein the aqueous formulation is free of solvent other than water. In some embodiments, the aqueous formulation is a clear aqueous solution, wherein the aqueous formulation has pH value from about 6.5 to about 7, and wherein the aqueous formulation is substantially free of solvent other than water. In some embodiments, the aqueous formulation is a clear aqueous solution, wherein the aqueous formulation has pH value from about 6.5 to about 7, and wherein the aqueous formulation is free of solvent other than water. In some embodiments, the aqueous formulation is a clear aqueous solution, wherein the aqueous formulation has pH value from about 7 to about 7.5, and wherein the aqueous formulation is substantially free of solvent other than water. In some embodiments, the aqueous formulation is a clear aqueous solution, wherein the aqueous formulation has pH value from about 7 to about 7.5, and wherein the aqueous formulation is free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5, and wherein the aqueous formulation is substantially free of solvent other than water. In some embodiments, the aqueous formulation is a clear aqueous solution, wherein the aqueous formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5, and wherein the aqueous formulation is free of solvent other than water.

In some embodiments, the aqueous formulation is a clear aqueous solution. In some embodiments, the solution remains clear for at least about 1 hours, 2 hours, 3 hours, 4 hours, 5 hours, 6 hours, 8 hours, 12 hours, or 24 hours.

As used herein, the term "clear aqueous solution" refers to an aqueous solution that is transparent and free of visible particles, precipitation upon visual observation.

When visually observed, for example, the term "clear aqueous solution" excludes a milky aqueous solution. Further, the term "clear aqueous solution" excludes a cloudy or hazy aqueous solution.

In some embodiments, the aqueous solution is a clear aqueous solution for at least 1 hour. In some embodiments, the aqueous formulation is a clear aqueous solution for at least 2 hours. In some embodiments, the aqueous formulation is a clear aqueous solution for at least 3 hours. In some embodiments, the aqueous formulation is a clear aqueous solution for at least 6 hours. In some embodiments, the aqueous formulation is a clear aqueous solution for at least 8 hours. In some embodiments, the aqueous formulation is a clear aqueous solution for a period of time selected from 1 hour. 2 hours, 3 hours, 4 hours, 5 hours, 6 hours, 7 hours, 8 hours, 12 hours, and 24 hours. In some embodiments, the aqueous formulation is a clear aqueous solution for at least 24 hours.

Also, provided herein is a pharmaceutical composition comprising the composition comprising the fosaprepitant, or a pharmaceutically acceptable salt thereof, the aprepitant and the human serum albumin as described herein, and a pharmaceutically acceptable carrier. In some embodiments, the pharmaceutical composition comprising the composition comprising the fosaprepitant, or a pharmaceutically acceptable salt thereof, and the human serum albumin as described herein, and a pharmaceutically acceptable carrier, is a liquid pharmaceutical composition. In some embodiments, the liquid pharmaceutical composition is an aqueous solution. In some embodiments, the liquid pharmaceutical composition is free of solvent other than water. In some embodiments, the liquid pharmaceutical composition is substantially free of solvent other than water. In some embodiments, the liquid pharmaceutical composition is an injectable pharmaceutical formulation. In some embodiments, the liquid pharmaceutical composition is a formulation for infusion (e.g., intravenous infusion).

As used herein, the term "aqueous solution" refers to a solution, wherein at least one solvent is water and the weight % of water in the mixture of solvents is at least 50%, at least 60%, at least 70%, at least 90%, at least 95%, or at least 99%. In some embodiments, aqueous solution is a solution in which water is the only solvent. As used herein, the term "aqueous solvent" refer to a liquid comprising at least 50%, at least 60%, at least 70%, at least 90% or at least 95% water. In some embodiments, aqueous solvent is water. In some embodiments, aqueous solvent is saline.

In some embodiments, pharmaceutically acceptable carrier refers to any carrier useful to solubilize and deliver an agent to a subject. A desirable pharmaceutically acceptable carrier is saline. Other pharmaceutically acceptable

carrier and their formulation are known to one skilled in the art and described, for example, in Remington's Pharmaceutical Sciences. (20th edition), ed. A. Gennaro, 2003, Lippincon Williams & Wilkins. In some embodiments, the carrier may contain components such as, for example, dextrose, glucose, serum proteins (other than HSA), buffer substances such as phosphates, glycine, sorbic acid, potassium sorbate, salts or electrolytes, such as protamine sulfate, disodium hydrogen phosphate, potassium hydrogen phosphate, sodium chloride, zinc salts, and cellulose-based substances. In some embodiments, the carrier may contain components such as contain anti-oxidants, buffers, bacteriostats and solutes which render the formulation isotonic with the blood of the intended recipient.

In some embodiments, the pharmaceutically acceptable excipient is selected from lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The pharmaceutical compositions may additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The pharmaceutical composition may be formulated so as to provide quick, sustained or delayed release of the active ingredient after administration to the patient by employing procedures known in the art.

In some embodiments, the pharmaceutical composition of the present disclosure may be administered by a syringe or a catheter, or any other means generally known in the art for the delivery of a pharmaceutical agent by injection to the subject in need thereof. The delivery means will vary, as recognized by those skilled in the art, depending on the diseases and conditions treated, the severity of the disease, the sex, age and general health condition of the subject, excipient usage, the possibility of co-usage with other therapeutic treatments such as use of other agents as described herein and the judgment of the treating physician.

The pharmaceutical composition comprising the composition comprising the fosaprepitant, or a pharmaceutically acceptable salt thereof, and the human serum albumin as described herein can be administered to a subject via various routes, such as parenterally, including intravenous, intra-arterial, intraperitoneal, intrapulmonary, oral, inhalation, intravascular, intramuscular, intra-tracheal, subcutaneous, intraocular, intrathecal, or transdermal. For example, the composition can be administered by inhalation to treat conditions of the respiratory tract. In some embodiments, the pharmaceutical composition is administrated intravenously (e.g., as an infusion). In some embodiments, the pharmaceutical composition is administrated orally as a capsule or an oral suspension.

Also, provided herein is a pharmaceutical composition comprising the composition comprising the fosaprepitant dimeglumine, the aprepitant and the human serum albumin as described herein, and a pharmaceutically acceptable carrier.

In some embodiments, the pharmaceutical composition is free of a surfactant, such as CREMOPHOR® surfactants and Polysorbate 80. In some embodiments, the pharmaceutical composition is substantially free of a surfactant, such as CREMOPHOR® surfactants and Polysorbate 80. As used herein, the term "substantially free of surfactant" refers to a formulation containing less than 0.0005%, less than 0.0003%, or less than 0.0001% of surfactants and/or less than 0.0005%, less than 0.0003%, or less than 0.0001% of surfactant.

Also, provided herein is a method for the prevention of chemotherapy-induced nausea and vomiting, the method comprising the step of administering to a subject in need thereof of a therapeutically effective amount of a pharmaceutical composition comprising the composition comprising the fosaprepitant, or a pharmaceutically acceptable salt thereof, the aprepitant and the human serum albumin as described herein, and a pharmaceutically acceptable carrier. In some aspects of these embodiments, the chemotherapy-induced nausea and vomiting is acute and delayed nausea and vomiting associated with initial and repeat courses of highly emetogenic cancer chemotherapy (HEC) including high-dose cisplatin. In other aspects of these embodiments, the chemotherapy-induced nausea and vomiting is nausea and vomiting associated with initial and repeat courses of moderately emetogenic cancer chemotherapy (MEC). In some embodiments, the nausea and vomiting is postoperative nausea and vomiting (PONV) in adults.

Also, provided herein is a method of treating chemotherapy-induced nausea and vomiting, the method comprising the step of administering to a subject in need thereof of a therapeutically effective amount of a pharmaceutical composition comprising the composition comprising the fosaprepitant, or a pharmaceutically acceptable salt thereof, aprepitant and the human serum albumin as described herein, and a pharmaceutically acceptable carrier.

In some aspects of these embodiments, the chemotherapy-induced nausea and vomiting is acute and delayed nausea and vomiting associated with initial and repeat courses of highly emetogenic cancer chemotherapy (HEC) including high-dose cisplatin. In other aspects of these embodiments, the chemotherapy-induced nausea and vomiting is nausea and vomiting associated with initial and repeat courses of moderately emetogenic cancer chemotherapy (MEC). In some embodiments, the nausea and vomiting is postoperative nausea and vomiting (PONV) in adults.

As used herein, an "effective amount," "therapeutically effective amount," or a "pharmaceutically-effective amount" in reference to the compounds or compositions of the instant invention refers to the amount sufficient to induce a desired biological, pharmacological, or therapeutic outcome in a subject. That result can be reduction, prevention, mitigation, delay, shortening the time to resolution of, alleviation of the signs or symptoms of, or exert a medically-beneficial effect upon the underlying pathophysiology or pathogenesis of an expected or observed side-effect, toxicity,

disorder or condition, or any other desired alteration of a biological system.

In some embodiments, the methods described herein are for the prevention of acute and delayed nausea and vomiting associated with initial and repeat courses of highly emetogenic cancer chemotherapy (HEC) including high-dose cisplatin. In some embodiments, the methods described herein are for the prevention of delayed nausea and vomiting associated with initial and repeat courses of moderately emetogenic cancer chemotherapy (MEC). In some embodiments, the methods described herein are for the prevention of postoperative nausea and vomiting (PONV) in adults.

As used herein, the terms "individual", "patient", or "subject" are used interchangeably and refer to any animal, including mammals, preferably mice, rats, other rodents, rabbits, dogs, cats, swine, cattle, sheep, horses, or primates, and most preferably humans.

As used herein the term "treating" or "treatment" refers to 1) inhibiting the disease; for example, inhibiting a disease, condition or disorder in an individual who is experiencing or displaying the pathology or symptomatology of the disease, condition or disorder (i.e., arresting further development of the pathology and/or symptomatology), or 2) ameliorating the disease; for example, ameliorating a disease, condition or disorder in an individual who is experiencing or displaying the pathology or symptomatology of the disease, condition or disorder (i.e., reversing the pathology and/or symptomatology).

As used herein, an "effective amount," "therapeutically effective amount," or a "pharmaceutically-effective amount" in reference to the compounds or compositions of the instant invention refers to the amount sufficient to induce a desired biological, pharmacological, or therapeutic outcome in a subject. That result can be reduction, prevention, mitigation, delay, shortening the time to resolution of, alleviation of the signs or symptoms of, or exert a medically-beneficial effect upon the underlying pathophysiology or pathogenesis of an expected or observed side-effect, toxicity, disorder or condition, or any other desired alteration of a biological system.

As used herein, the term "preventing" (or "prevention") means to completely or almost completely stop an disease or condition (e.g., cancer, metastatic cancer) from occurring, for example when the patient or subject is predisposed to an condition or is at risk of a disease or condition. Preventing can also include inhibiting, i.e., arresting the development, of a condition.

In some embodiments, the methods described herein are performed in combination with at least one other antiemetic agents. In some embodiments, the methods described herein are performed in combination with dexamethasone and a 5-$HT_3$ antagonist (e.g., tropisetron, palonosetron, ramosetron, granisetron, ondansetron, dolasetron, or metoclopramide). In some embodiments, the antiemetic agent is selected from tropisetron, palonosetron, ramosetron, granisetron, ondansetron, dolasetron, metoclopramide, domperidone, olanzapine, droperidol, haloperidol, chlorpromazine, prochlorperazine, alizapride, prochlorperazine, metoclopramide, casopitant, aprepitant, cyclizine, diphenhydramine, dimenhydrinate, doxylamine, meclizine, promethazine, hydroxyzine, dronabinol, sativex, midazolam, lorazepam, hyoscine, trimethobenzamide, emetrol, propofol and muscimol.

In some embodiments, provided herein is a method for treating chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC), or postoperative nausea and vomiting (PONV) in adults), the method comprising administering to a subject in need thereof a therapeutically effective amount a pharmaceutical composition comprising the composition comprising the fosaprepitant, or a pharmaceutically acceptable salt thereof, aprepitant and the human serum albumin as described herein, and a therapeutically effective amount of at least one antiemetic agent as described herein.

In some embodiments, provided herein is a method for preventing chemotherapy-induced nausea and vomiting (e.g., acute and delayed nausea and vomiting associated with initial and repeat courses of highly emetogenic cancer chemotherapy (HEC) including high-dose cisplatin; or of delayed nausea and vomiting associated with initial and repeat courses of moderately emetogenic cancer chemotherapy (MEC), or postoperative nausea and vomiting (PONV) in adults), the method comprising administering to a subject in need thereof a therapeutically effective amount a pharmaceutical composition comprising the composition comprising the fosaprepitant, or a pharmaceutically acceptable salt thereof, aprepitant and the human serum albumin as described herein, and a therapeutically effective amount of at least one antiemetic agent as described herein.

In some embodiments, a pharmaceutical composition comprising the composition comprising the fosaprepitant, or a pharmaceutically acceptable salt thereof, aprepitant and the human serum albumin as described herein and an antiemetic agent are administered simultaneously.

In some embodiments, a pharmaceutical composition comprising the composition comprising the fosaprepitant, or a pharmaceutically acceptable salt thereof, aprepitant and the human serum albumin as described herein and an antiemetic agent are administered consecutively.

Also, provided herein is a pharmaceutical composition comprising the composition comprising the fosaprepitant, or a pharmaceutically acceptable salt thereof, aprepitant and the human serum albumin as described herein, at least

one antiemetic agent as described herein, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, a 5-$HT_3$ antagonist (e.g., tropisetron, palonosetron, ramosetron, granisetron, ondansetron, dolasetron, or metoclopramide), and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, palonosetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, 0.5mg of palonosetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, 0.25mg of palonosetron, and a pharmaceutically acceptable carrier.

In some embodiments, provided herein is a pharmaceutical composition comprising the composition the fosaprepitant, or a pharmaceutically acceptable salt thereof, aprepitant and the human serum albumin as described herein, 0.28mg of palonosetron hydrochloride, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, about 0.25mg of palonosetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, about 0.28mg of palonosetron hydrochloride, and a pharmaceutically acceptable carrier.

In some embodiments, provided herein is a liquid pharmaceutical formulation for injection comprising the composition comprising the fosaprepitant, or a pharmaceutically acceptable salt thereof, aprepitant and the human serum albumin as described herein, 0.25mg of palonosetron, and a pharmaceutically acceptable carrier.

In some embodiments, provided herein is a liquid pharmaceutical formulation for injection comprising the composition comprising the fosaprepitant, or a pharmaceutically acceptable salt thereof, aprepitant and the human serum albumin as described herein, 0.28mg of palonosetron hydrochloride, and a pharmaceutically acceptable carrier.

In some embodiments, the liquid pharmaceutical formulation for injection is an aqueous solution. In some embodiments, the liquid pharmaceutical formulation for injection is free of solvent other than water. In some embodiments, the liquid pharmaceutical formulation for injection is substantially free of solvent other than water. In some embodiments, the liquid pharmaceutical formulation for injection is a formulation for infusion (e.g., intravenous infusion).

In some embodiments, the liquid pharmaceutical formulation for injection has pH value from about 5 to about 8. In some embodiments, the liquid pharmaceutical formulation for injection has pH value from about 5.5 to about 7.8. In some embodiments, the liquid pharmaceutical formulation for injection has pH value from about 6 to about 7.5. In some embodiments, the liquid pharmaceutical formulation for injection has pH value from about 6.5 to about 7.5. In some embodiments, the liquid pharmaceutical formulation for injection has pH value from about 6 to about 6.5. In some embodiments, the liquid pharmaceutical formulation for injection has pH value from about 6.5 to about 7. In some embodiments, the liquid liquid pharmaceutical formulation for injection has pH value from about 7 to about 7.5. In some embodiments, the liquid pharmaceutical formulation for injection has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the liquid pharmaceutical formulation for injection is substantially free of solvent other than water. In some embodiments, the liquid pharmaceutical formulation for injection is free of solvent other than water.

In some embodiments, provided herein is a pharmaceutical composition comprising the composition comprising the fosaprepitant, or a pharmaceutically acceptable salt thereof, aprepitant and the human serum albumin as described herein, granisetron, and a pharmaceutically acceptable carrier.

In some embodiments, provided herein is a pharmaceutical composition comprising the composition comprising the fosaprepitant, or a pharmaceutically acceptable salt thereof, aprepitant and the human serum albumin as described herein, ondansetron, and a pharmaceutically acceptable carrier.

The methods described herein may be performed alone or in conjunction with another therapy, such as surgery, radiation, chemotherapy, immunotherapy, gene therapy, and the like.

In some embodiments, the amount of fosaprepitant, or a pharmaceutically acceptable salt thereof, that is administered to a subject in need thereof with any one of pharmaceutical compositions described herein is from about 50 mg to about 250 mg, from about 60 mg to about 240 mg, from about 70 mg to 230 mg, from about 80 mg to about 220 mg, from about 90 mg to about 210 mg, from about 100 mg to about 200 mg, or from about 125 mg to about 175 mg. In some embodiments, the amount of fosaprepitant, or a pharmaceutically acceptable salt thereof, that is administered

to a subject in need thereof with any one of pharmaceutical compositions described herein is about 100 mg, about 125 mg, about 150 mg, about 175 mg, or about 200 mg. In some embodiments, the amount of fosaprepitant, or a pharmaceutically acceptable salt thereof, that is administered to a subject in need thereof with any one of pharmaceutical compositions described herein is about 150 mg. In some embodiments, the amount of fosaprepitant, or a pharmaceutically acceptable salt thereof, is administered as an intravenous infusion over 20 to 30 minutes approximately 30 minutes prior to chemotherapy.

As will be understood by those of ordinary skill in the art, the appropriate doses of fosaprepitant will be approximately those already employed in clinical therapies wherein fosaprepitant is administered alone or in combination with other therapeutic agents. Variation in dosage will likely occur depending on the condition being treated. Appropriate effective doses will also vary, as recognized by those skilled in the art, depending on the severity of the disease, the route of administration, the sex, age and general health condition of the subject, excipient usage, the possibility of co-usage with other therapeutic treatments such as use of other agents, and the judgment of the treating physician. For example, guidance for selecting an effective dose can be determined by reference to the prescribing information for fosaprepitant.

Also, provided herein is a liquid pharmaceutical composition comprising the composition comprising the fosaprepitant, or a pharmaceutically acceptable salt thereof, the aprepitant and the human serum albumin as described herein, and a pharmaceutically acceptable carrier.

In some embodiments, provided herein is a liquid pharmaceutical composition comprising the composition comprising the fosaprepitant, or a pharmaceutically acceptable salt thereof, the aprepitant and the human serum albumin as described herein, palonosetron, and a pharmaceutically acceptable carrier.

In some embodiments, the liquid pharmaceutical composition comprises 0.25mg of palonosetron.

In some embodiments, the liquid pharmaceutical composition comprises 0.28mg of palonosetron hydrochloride.

In some embodiments, the liquid pharmaceutical composition is a reconstituted solution, reconstituted from the solid composition comprising the fosaprepitant, or a pharmaceutically acceptable salt thereof, the aprepitant and the human serum albumin as described herein.

In some embodiments, the liquid pharmaceutical composition is a reconstituted solution, reconstituted from the solid composition comprising the fosaprepitant, or a pharmaceutically acceptable salt thereof, the aprepitant and the human serum albumin as described herein and palonosetron.

In some embodiments, the liquid pharmaceutical composition is an aqueous solution. In some embodiments, the liquid pharmaceutical composition is an aqueous solution substantially free of solvent other than water. In some embodiments, the liquid pharmaceutical composition is an aqueous solution free of solvent other than water.

In some embodiments, the liquid pharmaceutical composition has pH value from about 5 to about 8. In some embodiments, the liquid pharmaceutical composition has pH value from about 5.5 to about 7.8. In some embodiments, the liquid pharmaceutical composition has pH value from about 6 to about 7.5. In some embodiments, the liquid pharmaceutical composition has pH value from about 6.5 to about 7.5. In some embodiments, the liquid pharmaceutical composition has pH value from about 6 to about 6.5. In some embodiments, the liquid pharmaceutical composition has pH value from about 6.5 to about 7. In some embodiments, the liquid pharmaceutical composition has pH value from about 7 to about 7.5. In some embodiments, the liquid pharmaceutical composition has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the liquid pharmaceutical composition is substantially free of solvent other than water. In some embodiments, the liquid pharmaceutical composition is free of solvent other than water.

In some embodiments, the liquid pharmaceutical composition is an aqueous reconstituted solution, reconstituted in a parenterally acceptable aqueous pharmaceutical diluent. In some embodiments, the liquid pharmaceutical composition is an aqueous reconstituted solution, reconstituted in an aqueous infusion fluid.

In some embodiments, the liquid pharmaceutical composition is an aqueous reconstituted solution, prepared by reconstituting a solid composition comprising the fosaprepitant, or a pharmaceutically acceptable salt thereof, and the aprepitant in a parenterally acceptable aqueous pharmaceutical diluents comprising human serum albumin.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a parenterally acceptable aqueous pharmaceutical diluents comprising human serum albumin.

In some embodiments, the liquid pharmaceutical composition is an aqueous reconstituted solution, prepared by reconstituting a solid composition comprising the fosaprepitant, or a pharmaceutically acceptable salt thereof, and the aprepitant in a parenterally acceptable aqueous pharmaceutical diluents comprising a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a parenterally acceptable aqueous pharmaceutical diluents comprising a

commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution comprising a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution comprising a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution prepared from diluting a commercially available solution of human serum albumin USP for infusion with saline.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution prepared from diluting a commercially available solution of human serum albumin USP for infusion with saline.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution prepared from diluting a commercially available solution of human serum albumin USP for infusion with water.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution prepared from diluting a commercially available solution of human serum albumin USP for infusion with water.

In some embodiments, the liquid pharmaceutical composition is an aqueous reconstituted solution, prepared by reconstituting a solid composition comprising the fosaprepitant, or a pharmaceutically acceptable salt thereof, and the aprepitant in a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a commercially available solution of human serum albumin USP for infusion.

In some embodiments, the liquid pharmaceutical composition is an aqueous reconstituted solution, prepared by reconstituting a solid composition comprising the fosaprepitant, or a pharmaceutically acceptable salt thereof, and the aprepitant in an aqueous solution of human serum albumin, in which the concentration in weight of human serum albumin in the solution is in the range from 0.1% to 25% (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution of human serum albumin, in which the concentration in weight of human serum albumin in the solution is in the range from 0.1% to 25% (w/v).

In some embodiments, the liquid pharmaceutical composition is an aqueous reconstituted solution, prepared by reconstituting a solid composition comprising the fosaprepitant, or a pharmaceutically acceptable salt thereof, and the aprepitant in a 5% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 5% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, and the aprepitant in a 10% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 10% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, and the aprepitant in a 20% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 20% solution of human serum albumin USP for infusion (w/v).

In some embodiments, the liquid pharmaceutical composition is an aqueous reconstituted solution, prepared

by reconstituting a solid composition comprising the fosaprepitant, or a pharmaceutically acceptable salt thereof, and the aprepitant in a 25% solution of human serum albumin USP for infusion (w/v).

In some embodiments, the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 25% solution of human serum albumin USP for infusion (w/v).

In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:20, no more than 1:25, no more than 3:100, no more than 1:50, no more than 1:100, no more than 1:200, no more than 1:500, or no more than 1:1000.

In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by molar no more than 1:20, no more than 1:25, no more than 3:100, no more than 1:50, no more than 1:100, no more than 1:200, no more than 1:500, or no more than 1:1000.

In some embodiments, the aprepitant and the fosaprepitant, or a pharmaceutically acceptable salt thereof, in the said solid composition comprising the fosaprepitant, or a pharmaceutically acceptable salt thereof, the aprepitant, and 0.25mg of palonosetron have a ratio by weight no more than 1:20, no more than 1:25, no more than 3:100, no more than 1:50, no more than 1:100, no more than 1:200, no more than 1:500, or no more than 1:1000.

In some embodiments, the aprepitant and the fosaprepitant, or a pharmaceutically acceptable salt thereof, in the said solid composition comprising the fosaprepitant, or a pharmaceutically acceptable salt thereof, the aprepitant, and 0.25mg of palonosetron have a ratio by molar no more than 1:20, no more than 1:25, no more than 3:100, no more than 1:50, no more than 1:100, no more than 1:200, no more than 1:500, or no more than 1:1000.

Fosaprepitant easily degrades to aprepitant unless stored at low temperature. Degradation is enhanced by the presence of water. In some embodiments, all or part of aprepitant in the solid composition is degraded from fosaprepitant, or a pharmaceutically acceptable salt thereof. In some embodiments, all aprepitant in the solid composition is degraded from fosaprepitant, or a pharmaceutically acceptable salt thereof. In some embodiments, part of aprepitant in the solid composition is degraded from fosaprepitant, or a pharmaceutically acceptable salt thereof.

In some embodiments, the liquid pharmaceutical composition contains from about 100 mg to about 150 mg of the fosaprepitant, or a pharmaceutically acceptable salt thereof. In some embodiments, the liquid pharmaceutical composition contains from about 145 mg to about 150 mg of the fosaprepitant, or a pharmaceutically acceptable salt thereof. In some embodiments, the liquid pharmaceutical composition contains about 150 mg of the fosaprepitant, or a pharmaceutically acceptable salt thereof. In some embodiments, the liquid pharmaceutical composition contains 150 mg of the fosaprepitant, or a pharmaceutically acceptable salt thereof. In some embodiments, the liquid pharmaceutical composition contains 245.3 mg of the fosaprepitant dimeglumine equivalent to 150 mg of fosaprepitant free acid.

In some embodiments, the fosaprepitant, or a pharmaceutically acceptable salt thereof, and the human serum albumin in the liquid pharmaceutical composition have a ratio by weight from about 1:0.5 to about 1:300. In some embodiments, the fosaprepitant, or a pharmaceutically acceptable salt thereof, and the human serum albumin in the liquid pharmaceutical composition have a ratio by weight from about 1:0.8 to about 1:200. In some embodiments, the fosaprepitant, or a pharmaceutically acceptable salt thereof, and the human serum albumin in the liquid pharmaceutical composition have a ratio by weight from about 1:1 to about 1:150. In some embodiments, the fosaprepitant, or a pharmaceutically acceptable salt thereof, and the human serum albumin in the liquid pharmaceutical composition have a ratio by weight from about 1:2 to about 1:100. In some embodiments, the fosaprepitant, or a pharmaceutically acceptable salt thereof, and the human serum albumin in the liquid pharmaceutical composition have a ratio by weight from about 1:2 to about 1:80. In some embodiments, the fosaprepitant, or a pharmaceutically acceptable salt thereof, and the human serum albumin in the liquid pharmaceutical composition have a ratio by weight from about 1:2 to about 1:50. In some embodiments, the fosaprepitant, or a pharmaceutically acceptable salt thereof, and the human serum albumin in the liquid pharmaceutical composition have a ratio by weight from about 1:2.5 to about 1:30. In some embodiments, the fosaprepitant, or a pharmaceutically acceptable salt thereof, and the human serum albumin in the liquid pharmaceutical composition have a ratio by weight from about 1:3 to about 1:20. In some embodiments, the fosaprepitant, or a pharmaceutically acceptable salt thereof, and the human serum albumin in the liquid pharmaceutical composition have a ratio by weight from about 1:3.5 to about 1:15. In some embodiments, the fosaprepitant, or a pharmaceutically acceptable salt thereof, and the human serum albumin in the liquid pharmaceutical composition have a ratio by weight from about 2:1 to about 1:20. In some embodiments, the fosaprepitant, or a pharmaceutically acceptable salt thereof, and the human serum albumin in the liquid pharmaceutical composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the liquid pharmaceutical composition is a clear aqueous solution. In some embodiments, the liquid pharmaceutical composition is a clear aqueous solution for at least 1 hour. In some embodiments, the liquid

pharmaceutical composition is a clear aqueous solution for at least 2 hours. In some embodiments, the liquid pharmaceutical composition is a clear aqueous solution for at least 3 hours. In some embodiments, the liquid pharmaceutical composition is a clear aqueous solution for at least 4 hours. In some embodiments, the liquid pharmaceutical composition is a clear aqueous solution for at least 5 hours. In some embodiments, the liquid pharmaceutical composition is a clear aqueous solution for at least 6 hours. In some embodiments, the liquid pharmaceutical composition is a clear aqueous solution for at least 24 hours.

In some embodiments, the liquid pharmaceutical composition is an injectable pharmaceutical formulation.

In some embodiments, a unit dosage form for injection comprising the liquid pharmaceutical composition contains about 150mg of the fosaprepitant. In some embodiments, a unit dosage form for injection comprising the liquid pharmaceutical composition contains 150mg of the fosaprepitant. In some embodiments, a unit dosage form for injection comprising the liquid pharmaceutical composition contains 245.3 mg of the fosaprepitant dimeglumine equivalent to 150 mg of the fosaprepitant (fosaprepitant free acid).

In some embodiments, a unit dosage form for injection comprising the liquid pharmaceutical composition contains about 150mg of fosaprepitant and 0.25mg of palonosetron. In some embodiments, a unit dosage form for injection comprising the liquid pharmaceutical composition contains 150mg of fosaprepitant and 0.25mg of palonosetron.In some embodiments, a unit dosage form for injection comprising the liquid pharmaceutical composition contains 245.3 mg of fosaprepitant dimeglumine and 0.28mg of palonosetron hydrochloride.

In some embodiments, the injectable pharmaceutical formulation is free of solvent other than water. In some embodiments, the injectable pharmaceutical formulation is substantially free of solvent other than water.

In some embodiments, the injectable pharmaceutical formulation has pH value from about 5 to about 8. In some embodiments, the injectable pharmaceutical formulation has pH value from about 5.5 to about 7.8. In some embodiments, the injectable pharmaceutical formulation has pH value from about 6 to about 7.5. In some embodiments, the injectable pharmaceutical formulation has pH value from about 6.5 to about 7.5. In some embodiments, the injectable pharmaceutical formulation has pH value from about 6 to about 6.5. In some embodiments, the injectable pharmaceutical formulation has pH value from about 6.5 to about 7. In some embodiments, the injectable pharmaceutical formulation has pH value from about 7 to about 7.5. In some embodiments, the injectable pharmaceutical formulation has pH value about 6, about 6.1, about 6.2, about 6.3, about 6.4, about 6.5, about 6.6, about 6.7, about 6.8, about 6.9, about 7.0, about 7.1, about 7.2, about 7.3, about 7.4, or about 7.5. In some embodiments, the injectable pharmaceutical formulation is substantially free of solvent other than water. In some embodiments, the injectable pharmaceutical formulation is free of solvent other than water.

In some embodiments, the injectable pharmaceutical formulation is a reconstituted solution, reconstituted from the composition comprising the fosaprepitant, or a pharmaceutically acceptable salt thereof, the aprepitant and the human serum albumin as described herein. In some embodiments, the injectable pharmaceutical formulation is a reconstituted solution, reconstituted in an aqueous infusion fluid. In some embodiments, the aqueous infusion fluid is normal saline. In some embodiments, the aqueous infusion fluid is a dextrose solution.

In some embodiments, the injectable pharmaceutical formulation is a clear aqueous solution. In some embodiments, the injectable pharmaceutical formulation is a clear aqueous solution for at least 1 hour. In some embodiments, the injectable pharmaceutical formulation is a clear aqueous solution for at least 2 hours. In some embodiments, the injectable pharmaceutical formulation is a clear aqueous solution for at least 3 hours. In some embodiments, the injectable pharmaceutical formulation is a clear aqueous solution for at least 4 hours. In some embodiments, the injectable pharmaceutical formulation is a clear aqueous solution for at least 5 hours. In some embodiments, the injectable pharmaceutical formulation is a clear aqueous solution for at least 6 hours.

In some embodiments, the injectable pharmaceutical formulation is a clear aqueous solution for at least 1 hour at a temperature from about 0 °C to about 10 °C. In some embodiments, the injectable pharmaceutical formulation is a clear aqueous solution for at least 2 hours at a temperature from about 0 °C to about 10 °C. In some embodiments, the injectable pharmaceutical formulation is a clear aqueous solution for at least 3 hours at a temperature from about 0 °C to about 10 °C. In some embodiments, the injectable pharmaceutical formulation is a clear aqueous solution for at least 6 hours at a temperature from about 0 °C to about 10 °C. In some embodiments, the injectable pharmaceutical formulation is a clear aqueous solution for at least 8 hours at a temperature from about 0 °C to about 10 °C. In some embodiments, the injectable pharmaceutical formulation is a clear aqueous solution for at least 24 hours at a temperature from about 0 °C to about 10 °C.

Also, provided herein is a kit comprising a solid composition comprising the fosaprepitant, or a pharmaceutically acceptable salt thereof, and the aprepitant , and an aqueous solution of human serum albumin.

In some embodiments, the said solid composition in the kit further comprises palonosetron. In some embodiments, the said solid composition in the kit further comprises 0.25mg of palonosetron. In some embodiments, the said solid composition in the kit further comprises 0.28mg of palonosetron hydrochloride.

In some embodiments, the aprepitant and the fosaprepitant, or a pharmaceutically acceptable salt thereof, in the said solid composition comprising the fosaprepitant, or a pharmaceutically acceptable salt thereof, and the aprepitant

have a ratio by weight no more than 1:20. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:25. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 3:100. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:50. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:100. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:200. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:500. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:1000. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:2000. In some embodiments, the aprepitant and the fosaprepitant, or a pharmaceutically acceptable salt thereof, in the said solid composition comprising the fosaprepitant, or a pharmaceutically acceptable salt thereof, and the aprepitant have a ratio by weight no more than 1:5000.

Fosaprepitant easily degrades to aprepitant unless stored at low temperature. Degradation is enhanced by the presence of water. In some embodiments, all or part of aprepitant in the solid composition is degraded from fosaprepitant, or a pharmaceutically acceptable salt thereof. In some embodiments, all aprepitant in the solid composition is degraded from fosaprepitant, or a pharmaceutically acceptable salt thereof. In some embodiments, part of aprepitant in the solid composition is degraded from fosaprepitant, or a pharmaceutically acceptable salt thereof.

In some embodiments, the fosaprepitant, or a pharmaceutically acceptable salt thereof, and the human serum albumin in the kit have a ratio by weight from about 1:0.5 to about 1:300. In some embodiments, the fosaprepitant, or a pharmaceutically acceptable salt thereof,and the human serum albumin in the kit have a ratio by weight from about 1:0.8 to about 1:200. In some embodiments, the fosaprepitant, or a pharmaceutically acceptable salt thereof, and the human serum albumin in the kit have a ratio by weight from about 1:1 to about 1:150. In some embodiments, the fosaprepitant, or a pharmaceutically acceptable salt thereof, and the human serum albumin in the kit have a ratio by weight from about 1:2 to about 1:100. In some embodiments, the fosaprepitant, or a pharmaceutically acceptable salt thereof, and the human serum albumin in the kit have a ratio by weight from about 1:2 to about 1:80. In some embodiments, the fosaprepitant, or a pharmaceutically acceptable salt thereof, and the human serum albumin in the kit have a ratio by weight from about 1:2 to about 1:50. In some embodiments, the fosaprepitant, or a pharmaceutically acceptable salt thereof, and the human serum albumin in the kit have a ratio by weight from about 1:2.5 to about 1:30. In some embodiments, the fosaprepitant, or a pharmaceutically acceptable salt thereof, and the human serum albumin in the kit have a ratio by weight from about 1:3 to about 1:20. In some embodiments, the fosaprepitant, or a pharmaceutically acceptable salt thereof, and the human serum albumin in the kit have a ratio by weight from about 1:3.5 to about 1:15. In some embodiments, the fosaprepitant, or a pharmaceutically acceptable salt thereof, and the human serum albumin in the kit have a ratio by weight from about 2:1 to about 1:20. In some embodiments, the fosaprepitant, or a pharmaceutically acceptable salt thereof, and the human serum albumin in the kit have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the solid composition in the kit comprises the fosaprepitant dimeglumine and the aprepitant.

In some embodiments, the solid composition in the kit comprises the fosaprepitant dimeglumine, the aprepitant, and palonosetron.

In some embodiments, the solid composition in the kit comprises the fosaprepitant dimeglumine, the aprepitant, and 0.25mg of palonosetron.

In some embodiments, the solid composition in the kit comprises the fosaprepitant dimeglumine, the aprepitant, and 0.28mg of palonosetron hydrochloride.

In some embodiments, the solid composition in the kit comprises 245.3mg of fosaprepitant dimeglumine and 0.28mg of palonosetron hydrochloride.

In some embodiments, the fosaprepitant dimeglumine and the human serum albumin in the kit have a ratio by

weight from about 1:0.2 to about 1:300. In some embodiments, the fosaprepitant dimeglumine and the human serum albumin in the kit have a ratio by weight from about 1:0.5 to about 1:150. In some embodiments, the fosaprepitant dimeglumine and the human serum albumin in the kit have a ratio by weight from about 1:0.8 to about 1:120. In some embodiments, the fosaprepitant dimeglumine and the human serum albumin in the kit have a ratio by weight from about 1:1 to about 1:100. In some embodiments, the fosaprepitant dimeglumine and the human serum albumin in the kit have a ratio by weight from about 1:1.2 to about 1:70. In some embodiments, the fosaprepitant dimeglumine and the human serum albumin in the kit have a ratio by weight from about 1:1.5 to about 1:50. In some embodiments, the fosaprepitant dimeglumine and the human serum albumin in the kit have a ratio by weight from about 1:1.8 to about 1:20. In some embodiments, the fosaprepitant dimeglumine and the human serum albumin in the kit have a ratio by weight from about 1:2 to about 1:10. In some embodiments, the fosaprepitant dimeglumine and the human serum albumin in the kit have a ratio by weight from about 2:1 to about 1:20. In some embodiments, the fosaprepitant dimeglumine and the human serum albumin in the kit have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the aqueous solution of human serum albumin in the kit comprises a commercially available solution of human serum albumin USP for infusion.

In some embodiments, the aqueous solution of human serum albumin in the kit has concentration of human serum albumin in the range from 0.1% to 25% (w/v).

In some embodiments, the solid composition in the kit comprises 245.3mg of fosaprepitant dimeglumine equivalent to 150 mg of fosaprepitant free acid.

In some embodiments, the solid composition in the kit comprises about 245.3mg of fosaprepitant dimeglumine.

In some embodiments, the kit comprises the solid composition comprising 245.3mg of fosaprepitant dimeglumine equivalent to 150 mg of fosaprepitant free acid and an aqueous solution of human serum albumin with the human serum albumin in the range from 0.1% to 25% (w/v).

In some embodiments, the kit comprises the solid composition comprising 245.3mg of fosaprepitant dimeglumine equivalent to 150 mg of fosaprepitant free acid and a 10% aqueous solution of human serum albumin (w/v).

In some embodiments, the kit comprises the solid composition comprising 245.3mg of fosaprepitant dimeglumine equivalent to 150 mg of fosaprepitant free acid and a 10% aqueous solution of human serum albumin (w/v) (10ml).

In some embodiments, the solid composition in the kit comprises 245.3mg of fosaprepitant dimeglumine equivalent to 150 mg of fosaprepitant free acid and and 0.28mg of palonosetron hydrochloride.

In some embodiments, the kit comprises the solid composition comprising 245.3mg of fosaprepitant dimeglumine equivalent to 150 mg of fosaprepitant free acid and 0.28mg of palonosetron hydrochloride, and an aqueous solution of human serum albumin with the human serum albumin in the range from 0.1% to 25% (w/v).

In some embodiments, the kit comprises the solid composition comprising 245.3mg of fosaprepitant dimeglumine equivalent to 150 mg of fosaprepitant free acid and 0.28mg of palonosetron hydrochloride, and a 10% aqueous solution of human serum albumin (w/v).

In some embodiments, the kit comprises the solid composition comprising 245.3mg of fosaprepitant dimeglumine equivalent to 150 mg of fosaprepitant free acid and 0.28mg of palonosetron hydrochloride, and a 10% aqueous solution of human serum albumin (w/v) (10ml).

## Methods of making

Also, provided herein are several methods to prepare a composition comprising fosaprepitant, or a pharmaceutically acceptable salt thereof, and human serum albumin as described herein, a composition comprising aprepitant and human serum albumin as described herein, or a composition comprising fosaprepitant, or a pharmaceutically acceptable salt thereof, aprepitant and human serum albumin as described herein.

### I. Compositions comprising fosaprepitant

In some embodiments, the present disclosure provides a method of preparing a composition comprising fosaprepitant, or a pharmaceutically acceptable salt thereof, and human serum albumin as described herein.

In some embodiments, the method of preparing a composition comprising fosaprepitant, or a pharmaceutically acceptable salt thereof, and human serum albumin as described herein comprise the steps of:

(i) obtaining a first solution comprising fosaprepitant, or a pharmaceutically acceptable salt thereof;
(ii) obtaining a second aqueous solution comprising human serum albumin (HSA); and
(iii) mixing the first solution of step (i) and the second aqueous solution of step (ii) to obtain a third aqueous solution

comprising the composition comprising fosaprepitant and human serum albumin.

In some embodiments, the first solution is an aqueous solution. In other embodiments, the first solution is a solution in a water-miscible non-aqueous solvent.
In some embodiments, the fosaprepitant, or a pharmaceutically acceptable salt thereof, is fosaprepitant dimeglumine.
In some embodiments, the composition is solid.
In some embodiments, the composition comprises a non-covalently bond complex comprising fosaprepitant and the human serum albumin.
In some embodiments, the mixing step is followed by removal of aqueous solvent from the third aqueous solution to yield the solid composition comprising fosaprepitant and human serum albumin. In some embodiments, the removal of aqueous solvent is conducted by rotary evaporation. In some embodiments, the removal of aqueous solvent is conducted by lyophilization.
In some embodiments, the fosaprepitant, or a pharmaceutically acceptable salt thereof, and the human serum albumin in the composition have a ratio by weight from about 1:0.5 to about 1:300, from about 1:0.8 to about 1:200, from about 1:1 to about 1:150, from about 1:2 to about 1:100, from about 1:2 to about 1:80, from about 1:2 to about 1:50, from about 1:2 to about 1:40, from about 1:2.5 to about 1:30, or from about 1:3 to about 1:20. In some embodiments, the fosaprepitant, or a pharmaceutically acceptable salt thereof, and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70. In some embodiments, the fosaprepitant, or a pharmaceutically acceptable salt thereof, and the human serum albumin in the composition have a molar ratio from about 200:1 to about 1:1, from about 150:1 to about 1:1, from about 100:1 to about 1:1, from about 80:1 to about 1:1, from about 60:1 to about 1:1, from about 60:1 to about 3:1, from about 55:1 to about 1:1, from about 55:1 to about 3:1, from about 50:1 to about 1:1, from about 50:1 to about 3:1, from about 40:1 to about 1:1, from about 30:1 to about 1:1, from about 30:1 to about 3:1, from about 20:1 to about 1:1, from about 20:1 to about 3:1, from about 10:1 to about 1:1, from about 5:1 to about 1:1, from about 5:1 to about 3:1, or from about 3:1 to about 1:1. In some embodiments, the fosaprepitant, or a pharmaceutically acceptable salt thereof, and the human serum albumin in the composition have a molar ratio of about 1:1, about 3:1, about 5:1, about 7:1, about 10:1, about 15;1, about 20:1, about 25;1, about 30:1, about 40:1, about 45;1, about 50:1, about 55:1, about 60:1, about 80:1, or about 100:1.
In some embodiments, the present disclosure provides a composition comprising fosaprepitant, or a pharmaceutically acceptable salt thereof, and human serum albumin prepared by any one of the processes described herein.
A non-limiting embodiments of the method are as follows.

*Formation of the first solution*

In some embodiments, a defined amount of fosaprepitant, or a pharmaceutically acceptable salt thereof, is dissolved in a solvent to obtain the first solution comprising fosaprepitant, or a pharmaceutically acceptable salt thereof. In some embodiments, the solvent is an aqueous solvent (e.g., water). In other embodiments, the solvent is a water-miscible non-aqueous solvent (e.g., an alcohol such as methanol).
In some embodiments, the solvent is aqueous solvent (e.g., water) and the amount of solvent is from about 0.01 mL to about 1 mL, from about 0.01 mL to about 0.5 mL, from about 0.01 mL to about 0.1 mL, from about 0.01 mL to about 0.05 mL, or from about 0.02 mL to about 0.04 mL per 1 mg of fosaprepitant, or a pharmaceutically acceptable salt thereof. In some embodiments, the amount of solvent is about 0.01 mL, about 0.02 mL, about 0.03 mL, about 0.04 mL, about 0.05 mL, about 0.1 mL, about 0.15 mL, about 0.2 mL, about 0.3 mL, about 0.4 mL, or about 0.5 mL per 1 mg of fosaprepitant, or a pharmaceutically acceptable salt thereof.

*Formation of the second aqueous solution*

In some embodiments, a defined amount of human serum albumin is dissolved in an amount of aqueous solvent to form a second aqueous solution. In some aspects of these embodiments, the aqueous solvent is water. In some aspects of these embodiments, the aqueous solvent is saline.
In some embodiments, the amount of the aqueous solvent in the first aqueous solution is from about 0.001mL to about 10 mL per 1 mg of HSA. In some embodiments, the amount of the aqueous solvent in the first aqueous solution per 1 mg of HSA is from about 0.004mL to about 5 mL, from about 0.005 mL to about 1 mL, from about 0.008 mL to about 0.5 mL, 0.01 mL to about 0.2 mL, or from 0.01 mL to about 0.15 mL. In some embodiments, the amount of the aqueous solvent in the first aqueous solution per 1 mg of HSA is from about 0.0005 mL to about 0.025 mL, from about 0.0005 mL to about 1 mL, from about 0.0005 mL to about 0.05 mL, or from about 0.005 mL to about 0.05 mL. In some

embodiments, the amount of the aqueous solvent in the first aqueous solution per 1 mg of HSA is about 0.0005 mL, about 0.005 mL, about 0.01 mL, about 0.02 mL, or about 0.025 mL.

In some embodiments, a commercially available solution of human serum albumin USP for infusion can be used to form a second aqueous solution.

In some embodiments, a commercially available solution of human serum albumin USP for infusion can be used to form a second aqueous solution with or without being diluted with water or saline.In some embodiments, a commercially available solution of human serum albumin USP for infusion can be used to form a second aqueous solution when diluted with water.

In some embodiments, a commercially available solution of human serum albumin USP for infusion can be used to form a second aqueous solution when diluted with saline.

5%, 20%, and 25% solution of human serum albumin (w/v) USP for infusion are commercially available.

In some embodiments, a solution of human serum albumin USP for infusion with or without dilution to form a second aqueous solution has the concentration in weight of human serum albumin in the range from about 0.1% to about 25% (w/v).

In some embodiments, a solution of human serum albumin USP for infusion with or without dilution to form a second aqueous solution has the concentration in weight of human serum albumin in the range from about 1% to about 20% (w/v).

In some embodiments, a solution of human serum albumin USP for infusion with or without dilution to form a second aqueous solution has the concentration in weight of human serum albumin (w/v) in about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%.

In some embodiments, a 10% solution of human serum albumin (w/v) USP for infusion is used to form a second aqueous solution. In some embodiments, a 5% solution of human serum albumin (w/v) USP for infusion is used to form a second aqueous solution. In some embodiments, a 12.5% solution of human serum albumin (w/v) USP for infusion is used to form a second aqueous solution.

In some embodiments, the preparation of the first solution and the preparation of the second aqueous solution are performed concurrently. In some embodiments, the preparation of the first solution and the preparation of the second aqueous solution are performed sequentially. In some embodiments, the preparation of the first solution is performed before the preparation of the second aqueous solution. In some embodiments, the preparation of the second aqueous solution is performed before the preparation of the first solution.

*Formation of the third aqueous solution*

In some embodiments, the first solution comprising fosaprepitant, or a pharmaceutically acceptable salt thereof, is mixed with the second aqueous solution comprising HSA. In some embodiments, the third aqueous solution is a clear aqueous solution.

In some embodiments, the volume ratio of the amount of solvent (e.g., water) in the first solution and to the amount of aqueous solvent (e.g., water) in the second aqueous solution to form the third aqueous solution is from about 1:10 to about 10:1, from about 1:5 to about 5:1, from about 1:2 to about 5:1, from about 1:1 to about 5:1, or from about 1:1 to about 3:1. In some embodiments, the volume ratio of the amount of solvent (e.g., water) in the first solution and to the amount of aqueous solvent (e.g., water) in the second aqueous solution is about 1:1, about 2:1, about 3:1, about 4:1, or about 5:1.

In some embodiments, the mixing is carried out by adding the first solution to the second aqueous solution to form the third aqueous solution. In some embodiments, the mixing is varied out by adding the second aqueous solution to the first solution to form the third aqueous solution. In some embodiments, the addition is dropwise. In some embodiments, the mixing is performed with agitation, stirring or shaking. In some embodiments, the mixing is carried out at the temperature from about 0 °C to about 30°C. In some embodiments, the mixing is carried out at the temperature from about 0 °C to about 20 °C. In some embodiments, the mixing is carried out at the temperature from about 0 °C to about 10 °C. In some embodiments, the mixing is carried out at the temperature from about 0 °C to about 5 °C. In some embodiments, the mixing is carried out at the temperature about 0 °C. In some embodiments, the mixing is carried out at the temperature about 5 °C. In some embodiments, the mixing is carried out at the temperature about 10 °C. In some embodiments, the time of mixing is in a range from about 0.1 min to about 24 hours. In some embodiments, the time of mixing is in a range from about 1 min to about 2 hour. In some embodiments, the time of mixing is in a range from about 1 min to about 1 hour. In some embodiments, the time of mixing is in a range from about 5 min to about 30 min.

*Optionally removal of water from the third aqueous solution*

In some embodiments, the water can be removed from the third aqueous solution to provide a solid composition.

In some embodiments, the third aqueous solution is filtered before removal of water. For example, the third aqueous solution can be filtered by a 0.22 micron filter before removal of water. As used herein, the term "micron" refers to a unit of measure of one one-thousandth of a millimeter.
In some embodiments, the water is removed under a vacuum. In some embodiments, the water is removed using rotary evaporation. In some embodiments, the water is removed by lyophilization.

*Optionally reconstitution of the solid*

In some embodiments the solid composition comprising the fosaprepitant, or a pharmaceutically acceptable salt thereof, and the human serum albumin is mixed with a pharmaceutical carrier which is acceptable for an injection formulation or an infusion formulation (water solution). In some embodiments, the water solution is a saline solution. In some embodiments, the water solution is a 5% Dextrose water solution. In some embodiments, the mixing is the addition of the water solution to the solid. In some embodiments, the mixing is the addition of the solid to the water solution. In some embodiments, the mixing reconstitutes the solid. In some embodiments, the mixing yields a clear aqueous solution.

II. **Compositions comprising fosaprepitant and aprepitant**

In some embodiments, the present disclosure provides a method of preparing a composition comprising fosaprepitant, or a pharmaceutically acceptable salt thereof, aprepitant, and human serum albumin as described herein.
In some embodiments, the method of preparing a composition comprising fosaprepitant, or a pharmaceutically acceptable salt thereof, aprepitant and human serum albumin as described herein comprise the steps of:

(i) obtaining an organic solution comprising fosaprepitant, or a pharmaceutically acceptable salt thereof, and aprepitant in polar water- miscible organic solvent;
(ii) obtaining a first aqueous solution comprising human serum albumin (HSA); and
(iii) mixing the organic solution of step (i) and the first aqueous solution of step (ii) to obtain a second aqueous solution comprising the composition comprising fosaprepitant, aprepitant and human serum albumin.

In some embodiments, the fosaprepitant, or a pharmaceutically acceptable salt thereof, is fosaprepitant dimeglumine.
In some embodiments, the composition is solid.
In some embodiments, the composition comprises a non-covalently bond complex comprising fosaprepitant, aprepitant and the human serum albumin.
In some embodiments, the mixing step is followed by removal of solvents from the second aqueous solution to yield the solid composition comprising fosaprepitant, aprepitant and human serum albumin. In some embodiments, the removal of aqueous solvent is conducted by rotary evaporation. In some embodiments, the removal of aqueous solvent is conducted by lyophilization.
In some embodiments, the weight ratio of fosaprepitant, or a pharmaceutically acceptable salt thereof, to aprepitant in the composition is from about 200:1 to about 1:1, from about 150:1 to about 10:1, or from about 100:1 to about 30:1. In some embodiments, the weight ratio of fosaprepitant, or a pharmaceutically acceptable salt thereof, to aprepitant in the composition is about 200:1, about 100:1, about 100:2, about 100:3, about 100:4 or about 100:5.
In some embodiments, the molar ratio of fosaprepitant, or a pharmaceutically acceptable salt thereof, to aprepitant in the composition is from about 200:1 to about 5:1, from about 150 to about 10:1, from about 100:1 to about 20:1, or from about 90:1 to about 30:1.
In some embodiments, the weight ratio of aprepitant to human serum albumin in the composition is from about 1:1 to about 1:200, from about 1:5 to about 1:150, or from about 1:10 to about 1:100. In some embodiments, the weight ratio of aprepitant to human serum albumin in the composition is about 1:5, about 1:10, about 1:13, about 1:15, about 1:20, about 1:30, about 1:33, about 1:40, about 1:50, about 1:75, or about 1:100.
In some embodiments, the fosaprepitant, or the pharmaceutically acceptable salt thereof, and the human serum albumin in the composition have a ratio by weight from about 1:0.5 to about 1:300, from about 1:0.8 to about 1:200, from about 1:1 to about 1:150, from about 1:2 to about 1:100, from about 1:2 to about 1:80, from about 1:2 to about 1:50, from about 1:2.5 to about 1:30, from about 1:3 to about 1:20, from about 1:1 to about 3:1, or from about 1:1 to about 2.5:1. In some embodiments, the weight ratio of fosaprepitant to the human serum albumin in the composition is about 1:1, about 1.5:1, about 2:1, about 2.5:1, or about 3:1. In some embodiments, the fosaprepitant and the human serum albumin in the composition have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 1:70.

In some embodiments, the present disclosure provides a composition comprising fosaprepitant, or a pharmaceutically acceptable salt thereof, aprepitant, and human serum albumin prepared by any one of the processes described herein.
A non-limiting embodiments of the method are as follows.

*Formation of the organic solution*

As used herein, the term "organic solution" refers to a solution wherein at least one solvent is a non-aqueous solvent and the weight % of the non-aqueous solvent in the mixture of solvents is at least 50%, at least 60%, at least 70%, at least 90%, at least 95%, or at least 99%. In some embodiments, organic solution is a solution in which does not comprise water as a solvent.
In some embodiments, the terms "organic solvent" and "non-aqueous solvent" are used interchangeably and refer to a liquid comprising is at least 50%, at least 60%, at least 70%, at least 90%, at least 95%, or at least 99% of a solvent other than water. In some embodiments, the organic solvent does not comprise water.
In some embodiments, fosaprepitant, or a pharmaceutically acceptable salt thereof, and aprepitant are dissolved in a polar organic solvent (e.g., an alcohol such as methanol, ethanol, isopropanol, and/or n-butanol; THF, $CH_3CN$; DMF; or mixtures thereof) to form an organic solution. The polar organic solvent is miscible in water. In some embodiments, the polar organic solvent is an alcohol. In some embodiments, the polar organic solvent is ethanol or methanol, or mixtures thereof. For example, the polar organic solvent can be ethanol. In some embodiments, the polar organic solvent is methanol.
In some embodiments, the amount of polar organic solvent (e.g., methanol) is from about 0.005 mL to about 5 mL per 1 mg of solid mixture of aprepitant and fosaprepitant, or a pharmaceutically acceptable salt thereof. In some embodiments, the amount of polar organic solvent is from about 0.01 mL to about 3 mL per 1 mg of solid mixture of aprepitant and fosaprepitant, or a pharmaceutically acceptable salt thereof. In some embodiments, the amount of polar organic solvent is from about 0.01 mL to about 2 mL per 1 mg of solid mixture of aprepitant and fosaprepitant, or a pharmaceutically acceptable salt thereof. In some embodiments, the amount of polar organic solvent is from about 0.01 mL to about 1 mL per 1 mg of solid mixture of aprepitant and fosaprepitant, or a pharmaceutically acceptable salt thereof. In some embodiments, the amount of polar organic solvent is about 0.01 mL, about 0.011 mL, about 0.012 mL, about 0.013 mL, about 0.015 mL, or about 0.02 mL per 1 mg of solid mixture of aprepitant and fosaprepitant, or a pharmaceutically acceptable salt thereof

*Formation of the first aqueous solution*

In some embodiments, a defined amount of human serum albumin is dissolved in an amount of aqueous solvent to form a first aqueous solution. In some aspects of these embodiments, the aqueous solvent is water. In some aspects of these embodiments, the aqueous solvent is saline.
In some embodiments, the amount of the aqueous solvent in the first aqueous solution is from about 0.001mL to about 10 mL per 1 mg of HSA. In some embodiments, the amount of the aqueous solvent in the first aqueous solution per 1 mg of HSA is from about 0.004mL to about 5 mL, from about 0.005 mL to about 1 mL, from about 0.008 mL to about 0.5 mL, 0.01 mL to about 0.2 mL, or from 0.01 mL to about 0.15 mL. In some embodiments, the amount of the aqueous solvent in the first aqueous solution per 1 mg of HSA is from about 0.0005 mL to about 0.025 mL, from about 0.0005 mL to about 1 mL, from about 0.0005 mL to about 0.05 mL, or from about 0.005 mL to about 0.05 mL. In some embodiments, the amount of the aqueous solvent in the first aqueous solution per 1 mg of HSA is about 0.0005 mL, about 0.005 mL, about 0.01 mL, about 0.02 mL, or about 0.025 mL.
In some embodiments, a commercially available solution of human serum albumin USP for infusion can be used to form a first aqueous solution.
In some embodiments, a commercially available solution of human serum albumin USP for infusion can be used to form a first aqueous solution when diluted with water.
In some embodiments, a commercially available solution of human serum albumin USP for infusion can be used to form a first aqueous solution when diluted with saline.
5%, 20%, and 25% solution of human serum albumin (w/v) USP for infusion are commercially available.
In some embodiments, a solution of human serum albumin USP for infusion with or without dilution to form a first aqueous solution has the concentration in weight of human serum albumin in the range from about 0.1% to about 25% (w/v).
In some embodiments, a solution of human serum albumin USP for infusion with or without dilution to form a first aqueous solution has the concentration in weight of human serum albumin in the range from about 1% to about 20% (w/v).
In some embodiments, a solution of human serum albumin USP for infusion with or without dilution to form a

first aqueous solution has the concentration in weight of human serum albumin (w/v) in about 1%, 2%, 3%, 4%, 5%, 6%, 7%, 8%, 9%, 10%, 11%, 12%, 13%, 14%, 15%, 16%, 17%, 18%, 19%, or 20%.

In some embodiments, a 10% solution of human serum albumin (w/v) USP for infusion is used to form a first aqueous solution.

In some embodiments, a 5% solution of human serum albumin (w/v) USP for infusion is used to form a first aqueous solution.

In some embodiments, a 12.5% solution of human serum albumin (w/v) USP for infusion is used to form a first aqueous solution.

In some embodiments, the preparation of the organic solution and the preparation of the first aqueous solution are performed concurrently. In some embodiments, the preparation of the organic solution and the preparation of the first aqueous solution are performed sequentially. In some embodiments, the preparation of the organic solution is performed before the preparation of the first aqueous solution. In some embodiments, the preparation of the first aqueous solution is performed before the preparation of the organic solution.

*Formation of the second aqueous solution*

In some embodiments, the first aqueous solution of human serum albumin is mixed with a solid composition comprising fosaprepitant and aprepitant to form a second aqueous solution. In some embodiments, the first aqueous solution of human serum albumin is mixed with a solid composition comprising fosaprepitant dimeglumine and aprepitant to form a second aqueous solution. In some embodiments, the second aqueous solution is a clear aqueous solution.

In some embodiments, the aprepitant and the fosaprepitant in the said solid composition comprising the fosaprepitant and the aprepitant have a ratio by weight no more than 1:20. In some embodiments, the aprepitant and the fosaprepitant in the said solid composition comprising the fosaprepitant and the aprepitant have a ratio by weight no more than 1:25. In some embodiments, the aprepitant and the fosaprepitant in the said solid composition comprising the fosaprepitant and the aprepitant have a ratio by weight no more than 3:100. In some embodiments, the aprepitant and the fosaprepitant in the said solid composition comprising the fosaprepitant and the aprepitant have a ratio by weight no more than 1:50. In some embodiments, the aprepitant and the fosaprepitant in the said solid composition comprising the fosaprepitant and the aprepitant have a ratio by weight no more than 1:100. In some embodiments, the aprepitant and the fosaprepitant in the said solid composition comprising the fosaprepitant and the aprepitant have a ratio by weight no more than 1:200. In some embodiments, the aprepitant and the fosaprepitant in the said solid composition comprising the fosaprepitant and the aprepitant have a ratio by weight no more than 1:500.

In some embodiments, the fosaprepitant and the human serum albumin in the second aqueous solution have a ratio by weight from about 1:0.5 to about 1:300. In some embodiments, the fosaprepitant and the human serum albumin in the second aqueous solution have a ratio by weight from about 1:0.8 to about 1:200. In some embodiments, the fosaprepitant and the human serum albumin in the second aqueous solution have a ratio by weight from about 1:1 to about 1:150. In some embodiments, the fosaprepitant and the human serum albumin in the second aqueous solution have a ratio by weight from about 1:2 to about 1:100. In some embodiments, the fosaprepitant and the human serum albumin in the second aqueous solution have a ratio by weight from about 1:2 to about 1:80. In some embodiments, the fosaprepitant and the human serum albumin in the second aqueous solution have a ratio by weight from about 1:2 to about 1:50. In some embodiments, the fosaprepitant and the human serum albumin in the second aqueous solution have a ratio by weight from about 1:2.5 to about 1:30. In some embodiments, the fosaprepitant and the human serum albumin in the second aqueous solution have a ratio by weight from about 1:3 to about 1:20. In some embodiments, the fosaprepitant and the human serum albumin in the second aqueous solution have a ratio by weight from about 1:3.5 to about 1:15. In some embodiments, the fosaprepitant and the human serum albumin in the second aqueous solution have a ratio by weight of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:50, about 1:60, or about 70.

In some embodiments, the solid composition is added to the first aqueous solution to form a second aqueous solution. In some embodiments, the first aqueous solution is added to the solid composition to form a second aqueous solution. In some embodiments, the mixing is performed with agitation. In some embodiments, the mixing is performed with stirring. In some embodiments, the mixing is performed with shaking.

In some embodiments, the second aqueous can be further diluted by saline to a desired concentration for infusion.

In some embodiments, organic solution comprising fosaprepitant, or a pharmaceutically acceptable salt thereof, and aprepitant is mixed with the first aqueous solution of human serum albumin to form a second aqueous solution. In some embodiments, the second aqueous solution is a clear aqueous solution. In some embodiments, the mixing is carried out in a reaction vessel comprising aqueous solvent (e.g., water), and the volume ratio of the aqueous solvent in the reaction vessel to the volume ration of the aqueous solvent in the first aqueous solution is from about 4 to about 10.

In some embodiments, the volume ratio of the amount of the aqueous solvent (e.g., water or saline) to the amount of the polar organic solvent in the second aqueous solution is in a range from about 50:1 to about 1:50, from about 25:1 to about 1:1, from about 20:1 to about 1:1, from about 15:1 to about 1:1, from about 10:1 to about 1:1, from about 5:1 to about 1:1, from about 2.5:1 to about 1:1, from about 2.2:1 to about 1:1, from about 2:1 to about 1:1, from about 1.8:1 to about 1:1, from about 1.7:1 to about 1:1, or from about 1.5:1 to about 1:1. In some embodiments, the volume ratio of the amount of the aqueous solvent (e.g., water or saline) to the amount of the polar organic solvent in the second aqueous solution is about 1.5:1, about 1.7:1, about 1.8:1, about 2:1, about 2.2:1, or about 2.5:1.

In some embodiments, the mixing is carried out by adding the organic solution to the first aqueous solution to form the second aqueous solution. In some embodiments, the mixing is carried out by adding the organic solution to the reaction vessel comprising aqueous solvent and the first aqueous solution to form the second aqueous solution. In some embodiments, the mixing is varied out by adding the first aqueous solution to the organic solution to form the second aqueous solution. In some embodiments, the addition is dropwise. In some embodiments, the mixing is performed with agitation, stirring or shaking. In some embodiments, the mixing is carried out at the temperature from about 0 °C to about 30 °C. In some embodiments, the mixing is carried out at the temperature from about 0 °C to about 20 °C. In some embodiments, the mixing is carried out at the temperature from about 0 °C to about 10 °C. In some embodiments, the mixing is carried out at the temperature from about 0 °C to about 5 °C. In some embodiments, the mixing is carried out at the temperature about 0 °C. In some embodiments, the mixing is carried out at the temperature about 5 °C. In some embodiments, the mixing is carried out at the temperature about 10 °C.

In some embodiments, the time of mixing is in a range from about 0.1 min to about 24 hours. In some embodiments, the time of mixing is in a range from about 1 min to about 2 hour. In some embodiments, the time of mixing is in a range from about 1 min to about 1 hour. In some embodiments, the time of mixing is in a range from about 5 min to about 30 min.

*Optionally removal of water from the second aqueous solution*

In some embodiments, the water can be removed from the second aqueous solution to provide a solid composition.

In some embodiments, the second aqueous solution is filtered before removal of water. For example, the second aqueous solution can be filtered by a 0.22 micron filter before removal of water.

As used herein, the term "micron" refers to a unit of measure of one one-thousandth of a millimeter.

In some embodiments, the water is removed under a vacuum. In some embodiments, the water is removed using rotary evaporation. In some embodiments, the water is removed by lyophilization.

*Optionally reconstitution of the solid compostion*

In some embodiments the solid composition comprising the fosaprepitant, the aprepitant, and the human serum albumin is mixed with a water solution. In some embodiments, the water solution is a saline solution. In some embodiments, the water solution is a 5% Dextrose water solution. In some embodiments, the mixing is the addition of the water solution to the solid. In some embodiments, the mixing is the addition of the solid to the water solution. In some embodiments, the mixing reconstitutes the solid. In some embodiments, the mixing yields a clear aqueous solution.

## III. Compositions comprising aprepitant

Also, provided herein is a method of preparing a composition comprising aprepitant and human serum albumin as described herein, comprising the steps of:

(i) obtaining an organic solution of aprepitant in polar water- miscible organic solvent;
(ii) obtaining a first aqueous solution comprising human serum albumin (HSA); and
(iii) mixing the organic solution comprising aprepitant and the first aqueous solution comprising human serum albumin to obtain a second aqueous solution comprising the composition comprising aprepitant and human serum albumin.

In some embodiments, the composition is solid.

In some embodiments, the composition comprises a non-covalently bond complex comprising aprepitant and the human serum albumin.

In some embodiments, the mixing step is followed by removal of solvents from the second aqueous solution to yield the solid composition comprising fosaprepitant, aprepitant and human serum albumin. In some embodiments, the removal of aqueous solvent is conducted by rotary evaporation. In some embodiments, the removal of aqueous solvent is conducted by lyophilization.

In some embodiments, the aprepitant and the human serum albumin in the composition have a ratio by weight from about 1:80 to about 1:1000. In some embodiments, the aprepitant and the human serum albumin in the composition have a ratio by weight from about 1:100 to about 1:800. In some embodiments, the aprepitant and the human serum albumin in the composition have a ratio by weight from about 1:120 to about 1:600. In some embodiments, the aprepitant and the human serum albumin in the composition have a ratio by weight from about 1:130 to about 1:400. In some embodiments, the aprepitant and the human serum albumin in the composition have a ratio by weight from about 1:140 to about 1:300. In some embodiments, the aprepitant and the human serum albumin in the composition have a ratio by weight from about 1:150 to about 1:280. In some embodiments, the aprepitant and the human serum albumin in the composition have a ratio by weight from about 1:160 to about 1:260. In some embodiments, the aprepitant and the human serum albumin in the composition have a ratio by weight from about 1:180 to about 1:250. In some embodiments, the aprepitant and the human serum albumin in the composition have a ratio by weight from about 1:200 to about 1:250. In some embodiments, the aprepitant and the human serum albumin in the composition have a ratio by weight of about 1:120, about 1:130, about 1:140, about 1:150, about 1:160, about 1:170, about 1:180, about 1:190, about 1:200, about 1:210, about 1:220, about 1:230, about 1:240, about 1:250, about 1:260, about 1:270, about 1:280, about 1:290, about 1:300, about 1:310, about 1:320, about 1:330, about 1:340, about 1:350, or about 1:400.

In some embodiments, the present disclosure provides a composition comprising aprepitant and human serum albumin prepared by any one of the methods described herein.

Non-limiting embodiments of these methods are as follows.

*Formation of the organic solution*

As used herein, the term "organic solution" refers to a solution wherein at least one solvent is a non-aqueous solvent and the weight % of the non-aqueous solvent in the mixture of solvents is at least 50%, at least 60%, at least 70%, at least 90%, at least 95%, or at least 99%. In some embodiments, organic solution is a solution in which does not comprise water as a solvent.

In some embodiments, the terms "organic solvent" and "non-aqueous solvent" are used interchangeably and refer to a liquid comprising is at least 50%, at least 60%, at least 70%, at least 90%, at least 95%, or at least 99% of a solvent other than water. In some embodiments, the organic solvent does not comprise water.

In some embodiments, aprepitant is dissolved in a polar organic solvent (e.g., an alcohol such as methanol, ethanol, isopropanol, and/or n-butanol; THF, $CH_3CN$; DMF; or mixtures thereof) to form an organic solution.

The polar organic solvent is miscible in water. In some embodiments, the polar organic solvent is an alcohol. In some embodiments, the polar organic solvent is ethanol or methanol, or mixtures thereof. For example, the polar organic solvent can be ethanol. In some embodiments, the polar organic solvent is methanol.

In some embodiments, the amount of polar organic solvent (e.g., methanol) is from about 0.5 mL to about 5 mL, from about 1.0 mL to about 4 mL, or from about 1.3 mL to about 3.5 mL per 1 mg of aprepitant. In some embodiments, the amount of polar organic solvent is about 0.5 mL, about 1 mL, about 1.3 mL, about 1.5 mL, about 1.7 mL, about 2 mL, about 2.5 mL, about 3 mL, about 3.5 mL, about 4 mL, or about 5 mL per 1 mg of aprepitant.

*Formation of the first aqueous solution*

In some embodiments, a defined amount of human serum albumin is dissolved in an amount of aqueous solvent to form a first aqueous solution. In some aspects of these embodiments, the aqueous solvent is water.

In some embodiments, the amount of the aqueous solvent in the first aqueous solution is from about 0.005mL to about 10 mL per 1 mg of HSA. In some embodiments, the amount of the aqueous solvent in the first aqueous solution per 1 mg of HSA is from about 0.005mL to about 5 mL, about 0.01 mL to about 1 mL, from about 0.01 mL to about 0.5 mL, 0.01 mL to about 0.1 mL, 0.01 mL to about 0.02 mL, 0.01 mL to about 0.025 mL or 0.01 mL to about 0.05 mL. In some embodiments, the amount of the aqueous solvent in the first aqueous solution per 1 mg of HSA is about 0.01 mL, about 0.015 mL, about 0.018 mL, about 0.02 mL, about 0.025 mL, about 0.03 mL, about 0.033 mL, about 0.04 mL, or about 0.05 mL. In some embodiments, the resulting composition comprising the aprepitant and the human serum albumin can have any ratio by weight of the aprepitant to the human serum albumin as described herein. In some embodiments, the human serum albumin is a fatty acid free human serum albumin. In some embodiments, the human serum albumin is essentially fatty acid free.

In some embodiments, a commercially available solution of human serum albumin USP for infusion can be used to form a first aqueous solution with or without diluted with water.

In some embodiments, the preparation of the organic solution and the preparation of the first aqueous solution are performed concurrently.

In some embodiments, the preparation of the organic solution and the preparation of the first aqueous solution are performed sequentially. In some embodiments, the preparation of the organic solution is performed before the

preparation of the first aqueous solution. In some embodiments, the preparation of the first aqueous solution is performed before the preparation of the organic solution.

In some embodiments, a defined amount of human serum albumin is dissolved in an amount of aqueous solvent to form a first aqueous solution. In some aspects of these embodiments, the aqueous solvent is water. In some aspects of these embodiments, the aqueous solvent is saline.

In some embodiments, the amount of the aqueous solvent in the first aqueous solution is from about 0.001mL to about 10 mL per 1 mg of HSA. In some embodiments, the amount of the aqueous solvent in the first aqueous solution per 1 mg of HSA is from about 0.004mL to about 5 mL, from about 0.005 mL to about 1 mL, from about 0.008 mL to about 0.5 mL, 0.01 mL to about 0.2 mL, or from 0.01 mL to about 0.15 mL. In some embodiments, the amount of the aqueous solvent in the first aqueous solution per 1 mg of HSA is from about 0.0005 mL to about 0.025 mL, from about 0.0005 mL to about 1 mL, from about 0.0005 mL to about 0.05 mL, or from about 0.005 mL to about 0.05 mL. In some embodiments, the amount of the aqueous solvent in the first aqueous solution per 1 mg of HSA is about 0.0005 mL, about 0.005 mL, about 0.01 mL, about 0.02 mL, or about 0.025 mL.

In some embodiments, a commercially available solution of human serum albumin USP for infusion can be used to form a first aqueous solution. In some embodiments, a commercially available solution of human serum albumin USP for infusion can be used to form a first aqueous solution when diluted with water. In some embodiments, a commercially available solution of human serum albumin USP for infusion can be used to form a first aqueous solution when diluted with saline. 5%, 20%, and 25% solution of human serum albumin (w/v) USP for infusion are commercially available.

In some embodiments, a solution of human serum albumin USP for infusion with or without dilution to form a first aqueous solution has the concentration in weight of human serum albumin in the range from about 0.1% to about 25% (w/v).

In some embodiments, a solution of human serum albumin USP for infusion with or without dilution to form a first aqueous solution has the concentration in weight of human serum albumin in the range from about 1% to about 20% (w/v).

In some embodiments, a solution of human serum albumin USP for infusion with or without dilution to form a first aqueous solution has the concentration in weight of human serum albumin (w/v) in about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%.

In some embodiments, a 10% solution of human serum albumin (w/v) USP for infusion is used to form a first aqueous solution. In some embodiments, a 5% solution of human serum albumin (w/v) USP for infusion is used to form a first aqueous solution. In some embodiments, a 12.5% solution of human serum albumin (w/v) USP for infusion is used to form a first aqueous solution.

In some embodiments, the preparation of the organic solution and the preparation of the first aqueous solution are performed concurrently. In some embodiments, the preparation of the organic solution and the preparation of the first aqueous solution are performed sequentially. In some embodiments, the preparation of the organic solution is performed before the preparation of the first aqueous solution. In some embodiments, the preparation of the first aqueous solution is performed before the preparation of the organic solution.

*Formation of the second aqueous solution*

In some embodiments, the organic solution of aprepitant is mixed with the first aqueous solution of human serum albumin to form a second aqueous solution. In some embodiments, the second aqueous solution is a clear aqueous solution.

In some embodiments, the volume ratio of the amount of water to the amount of the polar organic solvent is in a range from about 1:1 to about 1000:1. In some embodiments, the volume ratio of the amount of water to the amount of the polar organic solvent is in a range from about 1.5:1 to about 100:1. In some embodiments, the volume ratio of the amount of water to the amount of the polar organic solvent is in a range from about 2:1 to about 100:1. In some embodiments, the volume ratio of the amount of water to the amount of the polar organic solvent is in a range from about 4:1 to about 50:1. In some embodiments, the volume ratio of the amount of water to the amount of the polar organic solvent is in a range from about 6:1 to about 25:1. In some embodiments, the volume ratio of the amount of water to the amount of the polar organic solvent is about 1:1, about 1.2:1, about 1.3:1, about 1.4:1, about 1.5:1, about 1.6:1, about 1.7:1, about 1.8:1, about 1.9:1, about 2:1, about 2.1:1, about 2.2:1, about 2.3:1, about 2.4:1, about 2.5:1, about 3:1, about 4:1, about 5:1, about 6:1, about 7:1, about 8:1, about 9:1, about 10:1, about 15:1, about 15:1, about 18:1, about 20:1, about 25:1, about 30:1, about 35:1, about 40:1, about 45:1, about 50:1, about 55:1, about 60:1, about 65:1, about 70:1, about 75:1, about 80:1, about 90:1, about 100:1, about 200:1, about 300:1, about 400:1, about 500:1, about 600:1, about 700:1, about 800:1, about 900:1, or about 1000:1.

In some embodiments, the organic solution is added to the first aqueous solution to form a second aqueous solution. In some embodiments, the organic solution is added dropwise to the first aqueous solution to form a second

aqueous solution. In some embodiments, the first aqueous solution is added to the organic solution to form a second aqueous solution. In some embodiments, the addition is dropwise. In some embodiments, the mixing is performed with agitation. In some embodiments, the mixing is performed with stirring. In some embodiments, the mixing is performed with shaking. In some embodiments, the mixing is carried out such that the Reynolds number in the resultant fluid forming the second aqueous solution is from about 1 to about 10000, from about 5 to about 9000, from about 10 to about 8000, from about 20 to about 7000, from about 30 to about 6000, from about 40 to about 5000, from about 40 to about 4000, from about 40 to about 3000, from about 40 to about 2000, from about 40 to about 1000, from about 1000 to about 10000, from about 2000 to about 9000, from about 2000 to about 8000, from about 2000 to about 7000, from about 2000 to about 6000, or from about 2000 to about 5000. In some embodiments, the mixing is carried out such that the Reynolds number in the resultant fluid of the second aqueous solution is about 20, about 40, about 100, about 500, about 1000, about 1500, about 2000, about 2500, about 3000, about 3500, about 4000, about 4500, about 5000, about 6000, about 7000, about 8000, or about 10000.

In some embodiments, the addition is done at the temperature from about 0 °C to about 30 °C. In some embodiments, the addition is done at the temperature from about 0 °C to about 20 °C. In some embodiments, the addition is done at the temperature from about 0 °C to about 10 °C. In some embodiments, the addition is done at the temperature from about 0 °C to about 5 °C. In some embodiments, the addition is done at the temperature about 0 °C. In some embodiments, the addition is done at the temperature about 5 °C. In some embodiments, the addition is done at the temperature about 10 °C.

In some embodiments, the time of addition is in a range from about 0.1 min to about 24 hours. In some embodiments, the time of addition is in a range from about 1 min to about 2 hour. In some embodiments, the time of addition is in a range from about 1 min to about 1 hour. In some embodiments, the time of addition is in a range from about 5 min to about 30 min.

*Removal of organic solvent and water from the second aqueous solution*

In some embodiments, upon completion of mixing of the organic solution with the first aqueous solution to form the second aqueous solution, the polar organic solvent and water are removed from the second aqueous solution to provide a solid. In some embodiments, the solvents are removed by lyophilization. In some embodiments, the solvents are removed under a vacuum. In some embodiments, the solvents are removed using rotary evaporation.

In some embodiments, the second aqueous solution was filtered before removal of the solvents. For example, the second aqueous solution can be filtered by a 0.22 micron filter before removal of the solvents.

As used herein, the term "micron" refers to a unit of measure of one one-thousandth of a millimeter.

In some embodiments, the polar organic solvent is removed under reduced pressure. In some embodiments, the polar organic solvent is removed using rotary evaporation. In some embodiments, the polar organic solvent is removed under a vacuum. In some embodiments, the removal of the polar organic solvent yields a clear aqueous solution. In some embodiments, water is removed from the aqueous under a vacuum. In some embodiments, water is removed from the aqueous solution using rotary evaporation. In some embodiments, water is removed from the aqueous solution by lyophilization.

In some embodiments, the solvents including both water and organic solvent are removed from the second aqueous solution simultaneously to provide a solid composition. In some embodiments, the solvents are removed under a vacuum. In some embodiments, the solvents are removed using rotary evaporation. In some embodiments, the solvents are removed by lyophilization. In some embodiments, the second aqueous solution was filtered before removal of the solvents.

*Reconstitution of the solid*

In some embodiments, the solid comprising the aprepitant and the human serum albumin is mixed with a water solution. In some embodiments, the water solution is a saline solution. In some embodiments, the water solution is a 5% Dextrose water solution. In some embodiments, the mixing is the addition of the water solution to the solid. In some embodiments, the mixing is the addition of the solid to the water solution. In some embodiments, the mixing reconstitutes the solid. In some embodiments, the mixing yields a clear aqueous solution.

Unless otherwise defined, all technical and scientific terms used herein have the same meaning as commonly understood by one of ordinary skill in the art to which this disclosure belongs. Methods and materials are described herein for use in the present disclosure; other suitable methods and materials known in the art can also be used. The materials, methods, and examples are illustrative only and not intended to be limiting. All publications, patent applications, patents, and other references mentioned herein are incorporated by reference in their entirety. In case of conflict, the present specification, including definitions, will control.

## EXAMPLES

### Materials and methods

**HPLC** analysis: The HPLC system used herein is a SHIMADZU LC-10AT vp series system, which consists of a SHIMADZU LC-10AT vp pump, a manual injector, a SHIMADZU CTO-10AS vp column oven, a SHIMADZU SPD-10A vp wavelength detector, and a SHIMADZU LC solution workstation. Agilent Zorbax XDB-C18 column (4.6 mm x 50 mm, 5 μm) is used as an analytical HPLC column. Mobile phases A and B consist of water with 0.1% trifluoroacetic acid (TFA) and methanol with 0.1% TFA, respectively. The mobile phases were delivered at a programmed linear gradient. Separation was pumped at a flow rate of 1 ml/minute, and initiated and maintained at a mobile phase ratio of 85:15 (A:B) for 1minute. The ratio was changed to 10:90 (A:B) over a period of 2 minutes using a linear curve and then maintained at 10:90 (A:B) over a period of 4.5 minutes. The mobile phase was subsequently changed back to 85:15 (A:B) over a period of 1 minute and this ratio was maintained for 1.5 minutes before the next sample was injected. The effluent is detected at a wavelength of 254 nm using a UV detector. The sample injection amount is 20 μl.

### Example 1: Composition comprising fosaprepitant and human serum albumin (HSA).

The ratio by weight of fosaprepitant dimeglumine to HSA prepared was about 1:40.

Fosaprepitant dimeglumine (10 mg) was dissolved in water (2 ml) in a vial to give a clear solution. A solution of HSA (400 mg, 2 ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

### Example 2: Composition comprising fosaprepitant and human serum albumin (HSA).

The ratio by weight of fosaprepitant dimeglumine to HSA prepared was about 1:20.

Fosaprepitant dimeglumine (10 mg) was dissolved in water (1 ml) in a vial to give a clear solution. A solution of HSA (200 mg, 1 ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

### Example 3: Composition comprising fosaprepitant and human serum albumin (HSA).

The ratio by weight of fosaprepitant dimeglumine to HSA prepared was about 1:4.

Fosaprepitant dimeglumine (25 mg) was dissolved in water (1.5 ml) in a vial to give a clear solution. A solution of HSA (100 mg, 0.5ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

### Example 4: Composition comprising fosaprepitant and human serum albumin (HSA).

The ratio by weight of fosaprepitant dimeglumine to HSA prepared was about 1:2.

Fosaprepitant dimeglumine (50 mg) was dissolved in water (1.5 ml) in a vial to give a clear solution. A solution of HSA (100 mg, 0.5ml) (20% human serum albumin solution for infusion (product name: AlbuRx) from CSL Behring) was added into the vial of the fosaprepitant dimeglumine water solution at 0 °C. Upon completion of the addition, a clear solution was obtained. The resulting clear aqueous solution was lyophilized overnight to give a white solid.

A sample of 100 mg of the lyophilized solid was reconstituted by adding 2 mL water to give a clear aqueous solution.

### Example 5: Composition comprising fosaprepitant and human serum albumin (HSA).

The ratio by weight of fosaprepitant dimeglumine to HSA prepared was about 1:4.

Fosaprepitant dimeglumine (50 mg) was dissolved in water (1ml) in a vial to give a clear solution. A solution of HSA (200 mg, 1ml) (20% human serum albumin solution for infusion (product name: AlbuRx) from CSL Behring) was added into the vial of the fosaprepitant dimeglumine water solution at 0 °C. Upon completion of the addition and warmed up to room temperature, a clear solution was obtained. 0.2ml of the resulting clear aqueous solution was added into a saline solution (2ml) to give a clear aqueous solution.

**Example 6: The stability of fosaprepitant in a human serum albumin (HSA) solution.**

To 3 different vials containing 25 mg of fosaprepitant dimeglumine each were added 1ml of 10% HSA solution (w/v), 1ml of 5% HSA solution (w/v), and 1ml of 2% HSA solution (w/v), respectively, at 25 °C. A clear aqueous solution was obtained initially for all 3 vials. 10% HSA solution (w/v), 5% HSA solution (w/v), and 2% HSA solution (w/v) are obtained by diluting (20% human serum albumin solution for infusion (product name: AlbuRx) with saline. The clear aqueous solutions in the 3 vials stayed clear after 1 hour, 2 hours, 3 hours, 4 hours, 6 hours, 8 hours, and 24 hours at 25 °C.

**Example 7: Composition comprising aprepitant and human serum albumin (HSA).**

The ratio by weight of aprepitant to HSA prepared was about 1:130.

Aprepitant (2 mg) was dissolved in methanol (2.6 ml) in a vial to give a clear solution. HSA (260 mg) (native fatty acid free human serum albumin purchased from SeraCare Life Sciences, product code: HS-455-80, which contains fatty acids < 0.2 mg/gm) as a powder was dissolved in 6 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution. After 1 hour at room temperature, the solution became cloudy from precipitation.

**Example 8: Composition comprising aprepitant and human serum albumin (HSA).**

The ratio by weight of aprepitant to HSA prepared was about 1:140.

Aprepitant (2 mg) was dissolved in methanol (2.6 ml) in a vial to give a clear solution. HSA (280 mg) (native fatty acid free human serum albumin purchased from SeraCare Life Sciences, product code: HS-455-80, which contains fatty acids < 0.2 mg/gm) as a powder was dissolved in 6 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution. After 1 hour at room temperature, the solution became cloudy from precipitation.

**Example 9: Composition comprising aprepitant and human serum albumin (HSA).**

The ratio by weight of aprepitant to HSA prepared was about 1:150.

Aprepitant (2 mg) was dissolved in methanol (2.6 ml) in a vial to give a clear solution. HSA (300 mg) (native fatty acid free human serum albumin purchased from SeraCare Life Sciences, product code: HS-455-80, which contains fatty acids < 0.2mg/gm) as a powder was dissolved in 6 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution. The clear aqueous solution stayed clear after 1 hour at room temperature. After 1.5 hours at room temperature, the solution became cloudy from precipitation.

**Example 10: Composition comprising aprepitant and human serum albumin (HSA).**

The ratio by weight of aprepitant to HSA prepared was about 1:200.

Aprepitant (2 mg) was dissolved in methanol (3.4 ml) in a vial to give a clear solution. HSA (400 mg) (native

fatty acid free human serum albumin purchased from SeraCare Life Sciences, product code: HS-455-80, which contains fatty acids < 0.2 mg/gm) as a powder was dissolved in 8 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution. The clear aqueous solution stayed clear after 1.5 hours at room temperature. After 2 hours at room temperature, the solution became cloudy from precipitation.

**Example 11: Composition comprising aprepitant and human serum albumin (HSA).**

The ratio by weight of aprepitant to HSA prepared was about 1:400.

Aprepitant (2 mg) was dissolved in methanol (6.9 ml) in a vial to give a clear solution. HSA (800 mg) (native fatty acid free human serum albumin purchased from SeraCare Life Sciences, product code: HS-455-80, which contains fatty acids < 0.2 mg/gm) as a powder was dissolved in 16 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution.

**Example 12: Composition comprising aprepitant and human serum albumin (HSA).**

The ratio by weight of aprepitant to HSA prepared was about 1:220.

Aprepitant (2 mg) was dissolved in methanol (3.9 ml) in a vial to give a clear solution. HSA (440 mg) (native fatty acid free human serum albumin purchased from SeraCare Life Sciences, product code: HS-455-80, which contains fatty acids < 0.2 mg/gm) as a powder was dissolved in 9 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution.

**Example 13: Composition comprising aprepitant and human serum albumin (HSA).**

The ratio by weight of aprepitant to HSA prepared was about 1:230.

Aprepitant (2 mg) was dissolved in methanol (3.9 ml) in a vial to give a clear solution. HSA (460 mg) (native fatty acid free human serum albumin purchased from SeraCare Life Sciences, product code: HS-455-80, which contains fatty acids < 0.2 mg/gm) as a powder was dissolved in 9 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron aqueous phase filter. The resulting clear aqueous solution was lyophilized overnight to give a white solid.

A sample of 100 mg of the lyophilized solid was reconstituted by adding 2 mL water to give a clear aqueous solution.

**Example 14: Composition comprising aprepitant and human serum albumin (HSA).**

The ratio by weight of aprepitant to HSA prepared was about 1:250.

Aprepitant (10 mg) was dissolved in methanol (10.7 ml) in a vial to give a clear solution. HSA (2500 mg) (native fatty acid free human serum albumin purchased from

SeraCare Life Sciences, product code: HS-455-80, which contains fatty acids < 0.2 mg/gm) as a powder was dissolved in 25 ml of water in a round bottom flask. The methanol solution of aprepitant was added slowly dropwise into the flask of the HSA solution with stirring at 0 °C. Upon completion of the addition, a clear solution was obtained. The methanol was removed under vacuum to give a clear solution. The clear water solution was filtered by a 0.22 micron

aqueous phase filter. The resulting clear aqueous solution was lyophilized overnight to give a white solid.

A sample of 100 mg of the lyophilized solid was reconstituted by adding 2 mL water to give a clear aqueous solution.

**Example 15: Measure the correlation between HPLC peak area and the aprepitant concentration.**

Methanol solutions of aprepitant in 6 different concentrations, 0.025 mg/ml, 0.0375 mg/ml, 0.05 mg/ml, 0.075 mg/ml, 0.1 mg/ml, and 0.15 mg/ml, were prepared. The 6 aprepitant methanol solutions were analyzed in HPLC. The peak area and concentration of aprepitant were correlated using linear regression. The linear regression data is shown as below.

$$Y \text{ (peak area)} = 3508 + 1.67218E6 * X \text{ (concentration)}, R = 0.99994, P < 0.0001.$$

**Example 16: Measure the aprepitant concentrations in the clear aqueous solutions before and after the filtration at 0 hour, and after the filtration at 1, 2, 3, 4, 5, and 6 hours.**

2300 mg of the lyophilized solid of the composition comprising aprepitant and HSA from Example 14 (The ratio by weight of aprepitant to HSA is about 1:250) was dissolved in 23 ml of water to give a clear aqueous solution, which was kept at about 25 °C. Immediately after the lyophilized solid was dissolved in water, 4 ml of the clear aqueous solution was taken out from the 23 ml solution. Then 1 ml of the solution was taken out from the 4 ml clear aqueous solution to give the solution AP-0-0h, and the remaining 3 ml of the solution was filtered by the same 0.22 micron aqueous phase filter at 1ml at a time to give the solutions AP-1-0h, AP-2-0h, and AP-3-0h. To 300 μl of the solutions AP-0-0h and AP-3-0h were added 700 μl of acetonitrile separately. The mixtures were vortexed for seconds and then centrifuged at 4,000 g for 5 minutes. The supernatants were removed and collected followed by injection on HPLC. The same procedure was repeated one more time for each of solutions AP-0-0h and AP-3-0h. Based on the HPLC data and the measurement data of Example 15, the aprepitant concentrations of the solutions of AP-0-0h and AP-3-0h have been calculated and shown in the Table 1. At 0 hour, the aprepitant concentration of the clear aqueous solution after the filtration was about 98.7% of the aprepitant concentration of the clear aqueous solution before the filtration.

**Table 1**

| Solution Number | Aprepitant Concentration (mg/ml) | Average aprepitant Concentration (mg/ml) |
|---|---|---|
| AP-0-0h-1 | 0.3623 | 0.3637 |
| AP-0-0h-2 | 0.3650 | |
| AP-3-0h-1 | 0.3637 | 0.3590 |
| AP-3-0h-2 | 0.3543 | |

At 1 hour, 3 ml of the clear aqueous solution was taken out from the remaining 19 ml of the aqueous solution. Then 1 ml of the solution was taken out from the 3 ml clear aqueous solution and filtered by a 0.22 micron aqueous phase filter to give the solution AP-1-1h, and the remaining 2 ml of the solution was filtered by the same 0.22 micron aqueous phase filter at 1 ml at a time to give the solutions AP-2-1h and AP-3-1h. To 300 μl of the solution AP-3-1h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-1h. Based on the HPLC data and the measurement data of Example 15, the aprepitant concentrations of the solution AP-3-1h have been calculated and shown in the Table 2. At 1 hour, the aprepitant concentration of the clear aqueous solution after the filtration was about 97.8% of the aprepitant concentration of the clear aqueous solution at 0 hour before the filtration.

**Table 2**

| Solution Number | Aprepitant Concentration (mg/ml) | Average aprepitant Concentration (mg/ml) |
|---|---|---|
| AP-3-1h -1 | 0.3563 | 0.3557 |
| AP-3-1h -2 | 0.3550 | |

At 2 hours, 3 ml of the clear aqueous solution was taken out from the remaining 16 ml of the aqueous solution. Then 1 ml of the solution was taken out from the 3 ml clear aqueous solution and filtered by a 0.22 micron aqueous phase filter to give the solution AP-1-2h, and the remaining 2 ml of the solution was filtered by the same 0.22 micron aqueous phase filter at 1 ml at a time to give the solutions AP-2-2h and AP-3-2h. To 300 μl of the solution AP-3-2h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-2h. Based on the HPLC data and the measurement data of sample 15, the aprepitant concentrations of the solution AP-3-2h have been calculated and shown in the Table 3. At 2 hours, the aprepitant concentration of the clear aqueous solution after the filtration was about 98.7% of the aprepitant concentration of the clear aqueous solution at 0 hour before the filtration.

**Table 3**

| Solution Number | Aprepitant Concentration (mg/ml) | Average aprepitant Concentration (mg/ml) |
|---|---|---|
| AP-3-2h -1 | 0.3587 | 0.3589 |
| AP-3-2h -2 | 0.3590 | |

At 3 hours, 3 ml of the clear aqueous solution was taken out from the remaining 13 ml of the aqueous solution. Then 1 ml of the solution was taken out from the 3 ml clear aqueous solution and filtered by a 0.22 micron aqueous phase filter to give the solution AP-1-3h, and the remaining 2 ml of the solution was filtered by the same 0.22 micron aqueous phase filter at 1 ml at a time to give the solutions AP-2-3h and AP-3-3h. To 300 μl of the solution AP-3-3h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-3h. Based on the HPLC data and the measurement data of sample 15, the aprepitant concentrations of the solution AP-3-3h have been calculated and shown in the Table 4. At 3 hours, the aprepitant concentration of the clear aqueous solution after the filtration was about 98.5% of the aprepitant concentration of the clear aqueous solution at 0 hour before the filtration.

**Table 4**

| Solution Number | Aprepitant Concentration (mg/ml) | Average aprepitant Concentration (mg/ml) |
|---|---|---|
| AP-3-3h -1 | 0.3590 | 0.3582 |
| AP-3-3h -2 | 0.3573 | |

At 4 hours, 3 ml of the clear aqueous solution was taken out from the remaining 10 ml of the aqueous solution. Then 1 ml of the solution was taken out from the 3 ml clear aqueous solution and filtered by a 0.22 micron aqueous phase filter to give the solution AP-1-4h, and the remaining 2 ml of the solution was filtered by the same 0.22 micron aqueous phase filter at 1 ml at a time to give the solutions AP-2-4h and AP-3-4h. To 300 μl of the solution AP-3-4h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-4h. Based on the HPLC data and the measurement data of sample 15, the aprepitant concentrations of the solution AP-3-4h have been calculated and shown in the Table 5. At 4 hours, the aprepitant concentration of the clear aqueous solution after the filtration was about 98.1% of the aprepitant concentration of the clear aqueous solution at 0 hour before the filtration.

**Table 5**

| Solution Number | Aprepitant Concentration (mg/ml) | Average aprepitant Concentration (mg/ml) |
|---|---|---|
| AP-3-4h -1 | 0.3573 | 0.3567 |
| AP-3-4h -2 | 0.3560 | |

At 5 hours, 3 ml of the clear aqueous solution was taken out from the remaining 7 ml of the aqueous solution. Then 1 ml of the solution was taken out from the 3 ml clear aqueous solution and filtered by a 0.22 micron aqueous phase filter to give the solution AP-1-5h, and the remaining 2 ml of the solution was filtered by the same 0.22 micron aqueous phase filter at 1 ml at a time to give the solutions AP-2-5h and AP-3-5h. To 300 μl of the solution AP-3-5h was

added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-5h. Based on the HPLC data and the measurement data of sample 15, the aprepitant concentrations of the solution AP-3-5h have been calculated and shown in the Table 6. At 5 hours, the aprepitant concentration of the clear aqueous solution after the filtration was about 97.8% of the aprepitant concentration of the clear aqueous solution at 0 hour before the filtration.

**Table 6**

| Solution Number | Aprepitant Concentration (mg/ml) | Average aprepitant Concentration (mg/ml) |
|---|---|---|
| AP-3-5h -1 | 0.3540 | 0.3557 |
| AP-3-5h -2 | 0.3573 | |

At 6 hours, 3 ml of the clear aqueous solution was taken out from the remaining 4 ml of the aqueous solution. Then 1 ml of the solution was taken out from the 3 ml clear aqueous solution and filtered by a 0.22 micron aqueous phase filter to give the solution AP-1-6h, and the remaining 2 ml of the solution was filtered by the same 0.22 micron aqueous phase filter at 1 ml at a time to give the solutions AP-2-6h and AP-3-6h. To 300 μl of the solution AP-3-6h was added 700 μl of acetonitrile. The mixture was vortexed for seconds and then centrifuged at 4,000 g for 5 minutes. The supernatant was removed and collected followed by injection on HPLC. The same procedure was repeated one more time for the solution AP-3-6h. Based on the HPLC data and the measurement data of sample 15, the aprepitant concentrations of the solution AP-3-6h have been calculated and shown in the Table 7. At 6 hours, the aprepitant concentration of the clear aqueous solution after the filtration was about 98.3% of the aprepitant concentration of the clear aqueous solution at 0 hour before the filtration.

**Table 7**

| Solution Number | Aprepitant Concentration (mg/ml) | Average aprepitant Concentration (mg/ml) |
|---|---|---|
| AP-3-6h -1 | 0.3577 | 0.3575 |
| AP-3-6h -2 | 0.3573 | |

**Example 17: Measure the stability of the solid composition comprising fosaprepitant and aprepitant in an aqueous solution with or without HSA.**

The ratio by weight of fosaprepitant dimeglumine to aprepitant in the solid composition was about 100:6.

Aprepitant (6 mg) and fosaprepitant dimeglumine (100 mg) was dissolved in 1ml of methanol to give a clear solution. Then methanol in the solution was removed to give a white solid. 20 mg each of the white solid obtained was added into each vial of the 3 vials. Water (1ml), 10% HSA solution (w/v) (1ml), 20% HSA solution (w/v) (1ml) were added into each vial with 20mg of the white solid of fosaprepitant dimeglumine and aprepitant separately. 20% HSA (w/v) solution is a 20% human serum albumin solution for infusion (product name: AlbuRx) from CSL Behring. 10% HSA solution (w/v) was obtained by diluting from the 20% HSA (w/v) solution with water. The white solids of the 3 vials were all dissolved to give a clear solution initially. The stability of the 3 solutions of fosaprepitant dimeglumine and aprepitant was shown in the table 8. In the table 8, "clear" means a clear solution, and "precipitation" means white precipitation formed in the solution.

**Table 8**

| t (minutes) | 0 | 5 | 15 | 30 |
|---|---|---|---|---|
| water (1ml) | clear | precipitation | precipitation | precipitation |
| 10% HSA (w/v) (1ml) | clear | clear | precipitation | precipitation |
| 20% HSA (w/v) (1ml) | clear | clear | clear | precipitation |

**Example 18: Measure the stability of the solid composition comprising fosaprepitant and aprepitant in an aqueous solution with or without HSA.**

The ratio by weight of fosaprepitant dimeglumine to aprepitant in the solid composition was about 100:5.

Aprepitant (5 mg) and fosaprepitant dimeglumine (100 mg) was dissolved in 1ml of methanol to give a clear solution. Then methanol in the solution was removed to give a white solid. 25 mg each of the white solid obtained was added into each vial of the 3 vials. Water (1ml), 10% HSA solution (w/v) (1ml), 20% HSA solution (w/v) (1ml) were added into each vial with 25 mg of the white solid of fosaprepitant dimeglumine and aprepitant separately. 20% has (w/v) solution is a 20% human serum albumin solution for infusion (product name: AlbuRx) from CSL Behring. 10% HSA solution (w/v) was obtained by diluting the 20% HSA (w/v) solution with water. The white solids of the 3 vials were all dissolved to give a clear solution initially. The stability of the 3 solutions of fosaprepitant dimeglumine and aprepitant was shown in the table 9. In the table 9, "clear" means a clear solution, and "precipitation" means white precipitation formed in the solution.

**Table 9**

| t (minutes) | 0 | 5 | 15 | 30 |
|---|---|---|---|---|
| water (1ml) | clear | precipitation | precipitation | precipitation |
| 10% HSA (w/v) (1ml) | clear | clear | clear | precipitation |
| 20% HSA (w/v) (1ml) | clear | clear | clear | precipitation |

**Example 19: Measure the stability of the solid composition comprising fosaprepitant and aprepitant in an aqueous solution with or without HSA.**

The ratio by weight of fosaprepitant dimeglumine to aprepitant in the solid composition was about 100:4.

Aprepitant (4 mg) and fosaprepitant dimeglumine (100 mg) was dissolved in 1ml of methanol to give a clear solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 10% HSA solution (w/v) was obtained by diluting the 20% HSA (w/v) solution with water. The white solids of the 3 vials were all dissolved to give a clear solution initially. The stability of the 3 solutions of fosaprepitant dimeglumine and aprepitant was shown in the table 10. In the table 10, "clear" means a clear solution, and "precipitation" means white precipitation formed in the solution.

**Table 10**

| t (minutes) | 0 | 5 | 15 | 30 | 60 | 120 | 180 |
|---|---|---|---|---|---|---|---|
| water (1ml) | clear | precipitation | precipitation | precipitation | precipitation | precipitation | precipitation |
| 10% HSA (w/v) (1ml) | clear | clear | clear | clear | precipitation | precipitation | precipitation |
| 20% HSA (w/v) (1ml) | clear | clear | clear | clear | clear | precipitation | precipitation |
| 20% HSA (w/v) (2ml) | clear | clear | clear | clear | clear | clear | precipitation |

**Example 20: Measure the stability of the solid composition comprising fosaprepitant and aprepitant in an aqueous solution with or without HSA.**

The ratio by weight of fosaprepitant dimeglumine to aprepitant in the solid composition was about 100:3.

Aprepitant (3 mg) and fosaprepitant dimeglumine (100 mg) was dissolved in 1ml of methanol to give a clear solution. Then methanol in the solution was removed to give a white solid. 25 mg each of the white solid obtained was added into each vial of the 3 vials. Water (1ml), 10% HSA solution (w/v) (1ml), 20% HSA solution (w/v) (1ml) were added into each vial with 25 mg of the white solid of fosaprepitant dimeglumine and aprepitant separately. 20% HSA (w/v) solution is a 20% human serum albumin solution for infusion (product name: AlbuRx) from CSL Behring. 5% HSA solution (w/v) and 10% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 11. In the table 11, "clear" means a clear solution, and "precipitation" means white precipitation formed in the solution.

**Table 11**

| t (minutes) | 0 | 5 | 10 | 120 | 180 | 300 | overnight |
|---|---|---|---|---|---|---|---|
| water (1ml) | clear | clear | precipitation | precipitation | precipitation | precipitation | precipitation |
| 5% HSA (w/v) (1ml) | clear | clear | clear | clear | precipitation | precipitation | precipitation |
| 10% HSA (w/v) (1ml) | clear | clear | clear | clear | clear | clear | precipitation |

**Example 21: Measure the stability of the solid composition comprising fosaprepitant and aprepitant in an aqueous solution with or without HSA.**

The ratio by weight of fosaprepitant dimeglumine to aprepitant in the solid composition was about 100:2.
Aprepitant (2 mg) and fosaprepitant dimeglumine (100 mg) was dissolved in 1ml of methanol to give a clear solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 5% HSA solution (w/v) and 10% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 12. In the table 12, "clear" means a clear solution, and "precipitation" means white precipitations formed in the solution.

**Table 12**

| t (minutes) | 0 | 5 | 10 | 60 | 180 | 300 | overnight |
|---|---|---|---|---|---|---|---|
| water (1ml) | clear | clear | precipitation | precipitation | precipitation | precipitation | precipitation |
| 5% HSA (w/v) (1ml) | clear | clear | clear | clear | clear | clear | clear |
| 10% HSA (w/v) (1ml) | clear | clear | clear | clear | clear | clear | clear |

**Example 22: Measure the stability of the solid composition comprising fosaprepitant and aprepitant in an aqueous solution with or without HSA.**

The ratio by weight of fosaprepitant dimeglumine to aprepitant in the solid composition was about 100:1.
Aprepitant (1 mg) and fosaprepitant dimeglumine (100 mg) was dissolved in 1ml of methanol to give a clear solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 5% HSA solution (w/v) and 10% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 13. In the table 13, "clear" means a clear solution, and "precipitation" means white precipitations formed in the solution.

**Table 13**

| t (minutes) | 0 | 5 | 10 | 60 | 180 | 300 | overnight |
|---|---|---|---|---|---|---|---|
| water (1ml) | clear | clear | precipitation | precipitation | precipitation | precipitation | precipitation |
| 5% HSA (w/v) (1ml) | clear | clear | clear | clear | clear | clear | clear |
| 10% HSA (w/v) (1ml) | clear | clear | clear | clear | clear | clear | clear |

**Example 23: Measure the stability of the solid composition comprising fosaprepitant and aprepitant in an aqueous solution with or without HSA.**

The ratio by weight of fosaprepitant dimeglumine to aprepitant in the solid composition was about 100:0.5.
Aprepitant (0.5 mg) and fosaprepitant dimeglumine (100 mg) was dissolved in 1ml of methanol to give a clear

solution. Then methanol in the solution was removed to give a white solid. 25mg each of the white solid obtained was added into each vial of the 3 vials. Water (1ml), 10% HSA solution (w/v) (1ml), 20% HSA solution (w/v) (1ml) were added into each vial with 25mg of the white solid of fosaprepitant dimeglumine and aprepitant separately. 20% HSA (w/v) solution is a 20% human serum albumin solution for infusion (product name: AlbuRx) from CSL Behring. 2% HSA solution (w/v) and 3% HSA solution (w/v) were obtained by diluting the 20% HSA (w/v) solution with water. The white solids of the 3 vials were all dissolved to give a clear solution initially. The stability of the 3 solutions of fosaprepitant dimeglumine and aprepitant was shown in the table 14. In the table 14, "clear" means a clear solution, and "precipitation" means white precipitations formed in the solution.

**Table 14**

| t (minutes) | 0 | 5 | 10 | 60 | 180 | 300 | overnight |
|---|---|---|---|---|---|---|---|
| water (1ml) | clear | clear | precipitation | precipitation | precipitation | precipitation | precipitation |
| 2% HSA (w/v) (1ml) | clear | clear | clear | clear | clear | clear | precipitation |
| 3% HSA (w/v) (1ml) | clear | clear | clear | clear | clear | clear | precipitation |

**Example 24: Measure the stability of the solid composition comprising fosaprepitant and aprepitant in an aqueous solution with or without HSA.**

The ratio by weight of fosaprepitant dimeglumine to aprepitant in the solid composition was about 100:0.3.

Aprepitant (0.3 mg) and fosaprepitant dimeglumine (100 mg) was dissolved in 1 ml of methanol to give a clear solution. Then methanol in the solution was removed to give a white solid. 25 mg each of the white solid obtained was added into each vial of the 3 vials. Water (1 ml), 10% HSA solution (w/v) (1 ml), 20% HSA solution (w/v) (1 ml) were added into each vial with 25 mg of the white solid of fosaprepitant dimeglumine and aprepitant separately. 20% HSA (w/v) solution is a 20% human serum albumin solution for infusion (product name: AlbuRx) from CSL Behring. 2% HSA solution (w/v) was obtained by diluting the 20% HSA (w/v) solution with water. The white solids of the 3 vials were all dissolved to give a clear solution initially. The stability of the 3 solutions of fosaprepitant dimeglumine and aprepitant was shown in the table 15. In the table 15, "clear" means a clear solution, and "precipitation" means white precipitations formed in the solution.

**Table 15**

| t (minutes) | 0 | 5 | 10 | 60 | 180 | 300 | overnight |
|---|---|---|---|---|---|---|---|
| water (1ml) | clear | clear | clear | clear | clear | clear | precipitation |
| 2% HSA (w/v) (1ml) | clear | clear | clear | clear | clear | clear | clear |

**Example 25: Composition comprising fosaprepitant, aprepitant, and human serum albumin (HSA)**.

The ratio by weight of fosaprepitant dimeglumine to aprepitant was about 100:1, and the ratio by weight of fosaprepitant dimeglumine to HSA prepared was about 1:1.

Fosaprepitant dimeglumine (100 mg) and aprepitant (1 mg) were dissolved in methanol (1.1 ml) in a vial to give a clear solution. A solution of HSA (100 mg, 0.5 ml) (20% human serum albumin solution for infusion (product name: AlbuRx) from CSL Behring) was added into 2 ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was shown in the table 16. In the table 16, "clear" means a clear solution, and "precipitation" means white precipitations formed in the solution.

**Table 16**

| t (hours) | 1 | 2 | 3 | 4 |
|---|---|---|---|---|
| water (2ml) | clear | clear | clear | clear |

**Example 26: Composition comprising fosaprepitant, aprepitant, and human serum albumin (HSA).**

The ratio by weight of fosaprepitant dimeglumine to aprepitant was about 100:2, and the ratio by weight of fosaprepitant dimeglumine to HSA prepared was about 1:1.

Fosaprepitant dimeglumine (100 mg) and aprepitant (2mg) were dissolved in methanol (1.2 ml) in a vial to give a clear solution. A solution of HSA (100 mg, 0.5ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was shown in the table 17. In the table 17, "clear" means a clear solution, and "precipitation" means white precipitations formed in the solution.

**Table 17**

| t (hours) | 1 | 2 | 3 | 4 |
|---|---|---|---|---|
| water (2ml) | clear | clear | precipitation | precipitation |

**Example 27: Composition comprising fosaprepitant, aprepitant, and human serum albumin (HSA).**

The ratio by weight of fosaprepitant dimeglumine to aprepitant was about 100:3, and the ratio by weight of fosaprepitant dimeglumine to HSA prepared was about 1:1.

Fosaprepitant dimeglumine (100 mg) and aprepitant (3mg) were dissolved in methanol (1.3 ml) in a vial to give a clear solution. A solution of HSA (100 mg, 0.5ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was studied. The white precipitation was formed in the solution in about one hour.

**Example 28: Composition comprising fosaprepitant, aprepitant, and human serum albumin (HSA).**

The ratio by weight of fosaprepitant dimeglumine to aprepitant was about 100:1, and the ratio by weight of fosaprepitant dimeglumine to HSA prepared was about 2.5:1.

Fosaprepitant dimeglumine (100 mg) and aprepitant (1mg) were dissolved in methanol (1.1 ml) in a vial to give a clear solution. A solution of HSA (40 mg, 0.2ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was shown in the table 18. In the table 18, "clear" means a clear solution, and "precipitation" means white precipitations formed in the solution.

**Table 18**

| t (hours) | 1 | 2 | 3 | 4 |
|---|---|---|---|---|
| water (2ml) | clear | clear | clear | clear |

**Example 29: Composition comprising fosaprepitant, aprepitant, and human serum albumin (HSA).**

The ratio by weight of fosaprepitant dimeglumine to aprepitant was about 100:2, and the ratio by weight of fosaprepitant dimeglumine to HSA prepared was about 2.5:1.

Fosaprepitant dimeglumine (100 mg) and aprepitant (2 mg) were dissolved in methanol (1.2 ml) in a vial to give a clear solution. A solution of HSA (40 mg, 0.2 ml) (20% human serum albumin solution for infusion (product name:

AlbuRx) from CSL Behring) was added into 2 ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was studied. The white precipitation was formed in the solution in about 40 minutes.

**Example 30: Composition comprising fosaprepitant, aprepitant, and human serum albumin (HSA).**

The ratio by weight of fosaprepitant dimeglumine to aprepitant was about 100:3, and the ratio by weight of fosaprepitant dimeglumine to HSA prepared was about 2.5:1.

Fosaprepitant dimeglumine (100 mg) and aprepitant (3mg) were dissolved in methanol (1.3 ml) in a vial to give a clear solution. A solution of HSA (40 mg, 0.2ml) (20% human serum albumin solution for infusion (product name: AlbuRx) from CSL Behring) was added into 2ml of water in a round bottom flask. The methanol solution of fosaprepitant dimeglumine and aprepitant was added slowly dropwise into the flask of the HSA solution with stirring at 0 °C. Upon completion of the addition, a clear solution was obtained. Then, the methanol in the solution was removed under vacuum to give a clear solution. The resulting clear aqueous solution was lyophilized overnight to give a white solid.

A sample of 100 mg of the lyophilized solid was reconstituted by adding 2 mL water to give a clear aqueous solution. The stability of the clear solution was studied. The white precipitation was formed in the solution in about 30 minutes.

**Example 31: Measuring pH value of the aqueous solution comprising fosaprepitant dimeglumine with or without human serum albumin (HSA)**

24.5mg of fosaprepitant dimeglumine was dissolved in 15ml of 0.9% saline solution to give a clear aqueous solution, which was kept at about 25 °C and measured for pH value. The pH value of the clear aqueous solution is 7.70 (3 measurement points: 7.70, 7.70, and 7.70).

24.5mg of fosaprepitant dimeglumine was dissolved in 15ml of a HSA solution, which was prepared by adding 0.5ml of 20% human serum albumin solution for infusion (product name: AlbuRx) from CSL Behring into 14.5ml of 0.9% saline solution, to give a clear aqueous solution. The resulting clear aqueous solution was kept at about 25 °C and measured for pH value. The pH value of the clear aqueous solution is 7.15 (3 measurement points: 7.15, 7.15, and 7.16).

**Example 32: Measuring pH value of the aqueous solution comprising fosaprepitant dimeglumine and palonosetron hydrochloride with human serum albumin (HSA)**

245.3mg of fosaprepitant dimeglumine and 0.28mg of palonosetron hydrochloride were dissolved in methanol, and then methanol was removed from the solution to give a white solid. 24.5mg of the resulting white solid was dissolved in 15ml of a HSA solution, which was prepared by adding 0.5ml of 20% human serum albumin solution for infusion (product name: AlbuRx) from CSL Behring into 14.5ml of 0.9% saline solution, to give a clear aqueous solution. The resulting clear aqueous solution was kept at about 25 °C and measured for pH value. The pH value of the clear aqueous solution is 7.09 (3 measurement points: 7.09, 7.10, and 7.09).

**OTHER EMBODIMENTS**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**EMBODIMENTS**

Although the present invention is defined in the claims, it should be understood that the present invention can also (alternatively) be defined in accordance with the following embodiments:

1. A composition comprising fosaprepitant, or a pharmaceutically acceptable salt thereof, and human serum albumin, wherein the fosaprepitant, or a pharmaceutically acceptable salt thereof, and the human serum albumin in the composition have a ratio by weight from about 1:0.1 to about 1:500.

2. The composition of embodiment 1, wherein the fosaprepitant, or a pharmaceutically acceptable salt thereof, and the human serum albumin in the composition have a ratio by weight from about 1:1 to about 1:150.

3. The composition of any one of embodiments 1-2, wherein the fosaprepitant, or a pharmaceutically acceptable salt thereof, and the human serum albumin in the composition have a ratio by weight from about 1:2 to about 1:50.

4. The composition of anyone of embodiments 1-3, wherein the fosaprepitant, or a pharmaceutically acceptable salt thereof, can be fosaprepitant dimeglumine.

5. The composition of anyone of embodiments 1-4, wherein the composition comprises at least one stabilizer for the human serum albumin.

6. The composition of anyone of embodiments 1-5, wherein the composition is a solid formulation.

7. The composition of anyone of embodiments 1-5, wherein the composition is an aqueous formulation.

8. The composition of embodiments 7, wherein the aqueous formulation is substantially free of solvent other than water.

9. The composition of any one of embodiments 7-8, wherein the aqueous formulation is a clear aqueous solution.

10. A pharmaceutical composition comprising the composition of any one of embodiments 1-9, and a pharmaceutically acceptable carrier.

11. A method for the prevention of chemotherapy-induced nausea and vomiting, the method comprising the step of administering to a subject in need thereof a therapeutically effective amount of a pharmaceutical composition of embodiment 10.

12. The method of embodiment 11, wherein the method is for the prevention of acute and delayed nausea and vomiting associated with initial and repeat courses of highly emetogenic cancer chemotherapy (HEC) including high-dose cisplatin.

13. The method of embodiment 11, wherein the method is for the prevention of delayed nausea and vomiting associated with initial and repeat courses of moderately emetogenic cancer chemotherapy (MEC).

14. The pharmaceutical composition of embodiment 10, wherein the composition comprises palonosetron.

15. The pharmaceutical composition of embodiment 14, wherein the composition comprises 150mg of fosaprepitant, the human serum albumin, 0.25mg of palonosetron, and a pharmaceutically acceptable carrier.

16. A composition comprising aprepitant and human serum albumin, wherein the aprepitant and the human serum albumin in the composition have a ratio by weight from about 1:80 to about 1:1000.

17. The composition of embodiment 16, wherein the aprepitant and the human serum albumin in the composition have a ratio by weight from about 1:130 to about 1:400.

18. The composition of any one of embodiments 16-17, wherein the aprepitant and the human serum albumin in the composition have a ratio by weight from about 1:160 to about 1:260.

19. The composition of any one of embodiments 16-18, wherein the human serum albumin is essentially fatty acid free.

20. The composition of any one of embodiments 16-19, wherein the composition is a solid formulation.

21. The composition of any one of embodiments 16-19, wherein the composition is an aqueous formulation.

22. The composition of embodiment 21, wherein the aqueous formulation is substantially free of solvent other than water.

23. The composition of any one of embodiments 21-22, wherein the aqueous formulation is a clear aqueous solution.

24. The composition of any one of embodiments 21-23, wherein the aqueous formulation is a clear aqueous solution for at least 6 hours.

25. A pharmaceutical composition comprising the composition of any one of embodiments 16-24, and a pharmaceutically acceptable carrier.

26. The pharmaceutical composition of embodiment 25, wherein the composition is free of a surfactant selected from CREMOPHOR® surfactants and Polysorbate 80.

27. A method for the prevention of chemotherapy-induced nausea and vomiting, the method comprising the step of administering to a subject in need thereof a therapeutically effective amount of a pharmaceutical composition of anyone of embodiments 25-26.

28. A composition comprising fosaprepitant, or a pharmaceutically acceptable salt thereof, aprepitant and human serum albumin, wherein the fosaprepitant, or a pharmaceutically acceptable salt thereof, and the human serum albumin in the composition have a ratio by weight from about 1:0.1 to about 1:500.

29. The composition of embodiment 28, wherein the aprepitant and the fosaprepitant, or a pharmaceutically acceptable salt thereof, in the composition have a ratio by weight no more than 1:50.

30. The composition of any one of embodiments 28-29, wherein the aprepitant and the fosaprepitant, or a pharmaceutically acceptable salt thereof, in the composition have a ratio by weight no more than 1:200.

31. The composition of any one of embodiments 28-30, wherein the aprepitant and the fosaprepitant, or a pharmaceutically acceptable salt thereof, in the composition have a ratio by weight no more than 1:500.

32. The composition of any one of embodiments 28-31, wherein the aprepitant and the fosaprepitant, or a pharmaceutically acceptable salt thereof, in the composition have a ratio by weight no more than 1:1000.

33. The composition of any one of embodiments 28-32, wherein the fosaprepitant, or a pharmaceutically acceptable salt thereof, and the human serum albumin in the composition have a ratio by weight from about 1:1 to about 1:150.

34. The composition of any one of embodiments 28-33, wherein the fosaprepitant, or a pharmaceutically acceptable salt thereof, and the human serum albumin in the composition have a ratio by weight from about 1:2 to about 1:50.

35. The composition of anyone of embodiments 28-34, wherein the fosaprepitant, or a pharmaceutically acceptable salt thereof, can be fosaprepitant dimeglumine.

36. The composition of anyone of embodiments 28-35, wherein the composition comprises at least one stabilizer for the human serum albumin.

37. The composition of anyone of embodiments 28-36, wherein the composition is a solid formulation.

38. The composition of anyone of embodiments 28-37, wherein the composition is an aqueous formulation.

39. The composition of embodiments 38, wherein the aqueous formulation is substantially free of solvent other than water.

40. The composition of any one of embodiments 38-39, wherein the aqueous formulation is a clear aqueous solution.

41. A pharmaceutical composition comprising the composition of any one of embodiments 28-40, and a pharmaceutically acceptable carrier.

42. A method for the prevention of chemotherapy-induced nausea and vomiting, the method comprising the step of administering to a subject in need thereof a therapeutically effective amount of a pharmaceutical composition of embodiment 41.

43. The method of embodiment 42, wherein the method is for the prevention of acute and delayed nausea and vomiting associated with initial and repeat courses of highly emetogenic cancer chemotherapy (HEC) including high-dose cisplatin.

44. The method of embodiment 42, wherein the method is for the prevention of delayed nausea and vomiting associated with initial and repeat courses of moderately emetogenic cancer chemotherapy (MEC).

45. A liquid pharmaceutical composition comprising the composition of any one of embodiments 28-40, and a pharmaceutically acceptable carrier.

46. The liquid pharmaceutical composition of embodiment 45, wherein the liquid pharmaceutical composition is an aqueous solution substantially free of solvent other than water.

47. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, and the aprepitant in a parenterally acceptable aqueous pharmaceutical diluents comprising human serum albumin.

48. The liquid pharmaceutical composition of any one of embodiments 45-47, wherein the liquid pharmaceutical composition is an aqueous reconstituted solution, prepared by reconstituting a solid composition comprising the fosaprepitant, or a pharmaceutically acceptable salt thereof, and the aprepitant in a parenterally acceptable aqueous pharmaceutical diluents comprising a commercially available solution of human serum albumin USP for infusion.

49. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, and the aprepitant in an aqueous solution comprising a commercially available solution of human serum albumin USP for infusion.

50. The liquid pharmaceutical composition of any one of embodiments 45-46 and 49, wherein the liquid pharmaceutical composition is an aqueous reconstituted solution, prepared by reconstituting a solid composition comprising the fosaprepitant, or a pharmaceutically acceptable salt thereof, and the aprepitant in an aqueous solution prepared from diluting a commercially available solution of human serum albumin USP for infusion with water or saline.

51. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, and the aprepitant in a commercially available solution of human serum albumin USP for infusion.

52. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, and the aprepitant in an aqueous solution of human serum albumin, in which the concentration in weight of human serum albumin in the solution is in the range from 0.1% to 25% (w/v).

53. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a parenterally acceptable aqueous pharmaceutical diluents comprising human serum albumin.

54. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution comprising a commercially available solution of human serum albumin USP for infusion.

55. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a commercially available solution of human serum albumin USP for infusion.

56. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in an aqueous solution of human serum albumin, in which the concentration in weight of human serum albumin in the solution is in the range from 0.1% to 25% (w/v).

57. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 5% solution of human serum albumin USP for infusion.

58. The liquid pharmaceutical composition of any one of embodiments 45-46, wherein the liquid pharmaceutical composition is an aqueous reconstituted solution, prepared by reconstituting a solid composition comprising the fosaprepitant, or a pharmaceutically acceptable salt thereof, the aprepitant, and 0.25mg of palonosetron in a 10% solution of human serum albumin USP for infusion.

59. The liquid pharmaceutical composition of any one of embodiments 47-58, wherein the liquid pharmaceutical composition is a clear aqueous solution.

60. The liquid pharmaceutical composition of any one of embodiments 47-59, wherein the liquid pharmaceutical composition is a clear aqueous solution for at least 3 hours.

61. The liquid pharmaceutical composition of any one of embodiments 47-60, wherein the liquid pharmaceutical composition is a clear aqueous solution for at least 6 hours.

62. A unit dosage form for injection comprising the liquid pharmaceutical composition of any one of embodiments 47-61.

63. A kit comprising a solid composition comprising the fosaprepitant, or a pharmaceutically acceptable salt thereof, and the aprepitant, and an aqueous solution of human serum albumin.

64. The kit of embodiment 63, wherein the solid composition in the kit further comprises 0.25mg of palonosetron.

65. The kit of any one of embodiments 63-64, wherein the solid composition in the kit comprises about 245.3mg of fosaprepitant dimeglumine.

66. The kit of any one of embodiments 63-65, wherein the aqueous solution of human serum albumin in the kit comprises a commercially available solution of human serum albumin USP for infusion.

67. The kit of any one of embodiments 63-66, wherein the aqueous solution of human serum albumin in the kit has concentration of human serum albumin in the range from 0.1% to 25% (w/v).

68. The kit of any one of embodiments 63-67, wherein the solid composition in the kit comprises 245.3mg of fosaprepitant dimeglumine equivalent to 150 mg of fosaprepitant free acid and and 0.28mg of palonosetron hydrochloride.

## Claims

1. A pharmaceutical composition comprising fosaprepitant, human serum albumin, a 5-HT3 antagonist, and a pharmaceutically acceptable carrier.

2. The composition of claim 1, wherein the 5-HT3 antagonist is palonosetron.

3. The composition of claim 2, wherein the composition comprises fosaprepitant dimeglumine, human serum albumin, palonosetron hydrochloride, and a pharmaceutically acceptable carrier.

4. The composition of claim 2, wherein the composition comprises 150mg fosaprepitant and 0.25mg palonosetron.

5. The composition of claim 3, wherein the composition comprises 245.3mg fosaprepitant dimeglumine and 0.28mg palonosetron hydrochloride.

6. The composition of claims 1-5, wherein the fosaprepitant, or fosaprepitant dimeglumine, and the human serum albumin in the composition have a ratio by weight from about 1:0.1 to about 1:500.

7. The composition of claim 6, wherein fosaprepitant dimeglumine and the human serum albumin in the composition have a ratio by weight from about 1:0.2 to about 1:300.

8. The composition of claims 1-7, wherein the pharmaceutically acceptable carrier is saline.

9. A liquid pharmaceutical formulation for injection comprising fosaprepitant, 0.25mg palonosetron, human serum albumin, and a pharmaceutically acceptable carrier.

10. The formulation of claim 9, wherein the formulation comprises 150mg fosaprepitant.

11. The formulation of claim 9, wherein the formulation comprises 245.3mg fosaprepitant dimeglumine, 0.28 palonosetron hydrochloride, human serum albumin, and a pharmaceutically acceptable carrier.

12. The formulation of claim 11, wherein fosaprepitant dimeglumine and the human serum albumin in the composition have a ratio by weight from about 1:0.2 to about 1:300.

13. The formulation of claims 9-12, wherein the human serum albumin concentration in the formulation is in the range from 0.1% to 25% (w/v).

14. The formulation of claims 9-13, wherein the pharmaceutically acceptable carrier is saline.

15. The formulation of claims 9-14, wherein the formulation is a formulation for infusion (e.g., intravenous infusion).

Europäisches Patentamt
European Patent Office
Office européen des brevets

# EUROPEAN SEARCH REPORT

Application Number
EP 22 15 6237

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PATRICK LANGFORD ET AL: "Fosaprepitant and aprepitant: an update of the evidence for their place in the prevention of chemotherapy-induced nausea and vomiting", CORE EVIDENCE, vol. 5, 24 September 2009 (2009-09-24), pages 77-90, XP055570918, DOI: 10.2147/CE.S6012 * pages 77-90 * | 1-15 | INV. A61K31/675 A61K31/5377 A61K38/38 A61K47/42 ADD. A61K9/08 A61K9/19 |
| X | WO 2014/093907 A1 (A P PHARMA INC [US]) 19 June 2014 (2014-06-19) * paragraphs [0006], [0010]; examples 1-8 * | 1-15 | |
| X | WO 2016/059587 A1 (PIRAMAL ENTPR LTD [IN]) 21 April 2016 (2016-04-21) * pages 2-3; claim 4; examples 1-14 * | 1-15 | |
| X | US 2015/165045 A1 (KARAVAS EVANGELOS [GR] ET AL) 18 June 2015 (2015-06-18) * page 1 - page 3; table 1 * | 1-15 | TECHNICAL FIELDS SEARCHED (IPC) |
| X | WO 2011/019911 A1 (OPKO HEALTH INC [US]; WAN JIANSHENG [US] ET AL.) 17 February 2011 (2011-02-17) * page 1 - page 8 * | 1-15 | A61K |
| A | SUSANA GARCIA-RECIO ET AL: "Biological and Pharmacological Aspects of the NK1-Receptor", BIOMED RESEARCH INTERNATIONAL, vol. 2015, 1 January 2015 (2015-01-01), pages 1-14, XP055601325, ISSN: 2314-6133, DOI: 10.1155/2015/495704 * the whole document * | 1-15 | |

-/--

The present search report has been drawn up for all claims

1

| Place of search | Date of completion of the search | Examiner |
|---|---|---|
| Munich | 11 May 2022 | Seranski, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 6237

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | T. L. NG ET AL: "Chemotherapy-Induced Nausea and Vomiting: Time for More Emphasis on Nausea?", THE ONCOLOGIST, vol. 20, no. 6, 6 May 2015 (2015-05-06), pages 576-583, XP055601329, US ISSN: 1083-7159, DOI: 10.1634/theoncologist.2014-0438 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 11 May 2022 | Seranski, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO.

EP 22 15 6237

This annex lists the patent family members relating to the patent documents cited in the above-mentioned European search report. The members are as contained in the European Patent Office EDP file on
The European Patent Office is in no way liable for these particulars which are merely given for the purpose of information.

11-05-2022

| Patent document cited in search report | Publication date | Patent family member(s) | Publication date |
|---|---|---|---|
| WO 2014093907 A1 | 19-06-2014 | EP 2931253 A1 | 21-10-2015 |
| | | US 2015320866 A1 | 12-11-2015 |
| | | US 2017232107 A1 | 17-08-2017 |
| | | US 2021338820 A1 | 04-11-2021 |
| | | WO 2014093907 A1 | 19-06-2014 |
| WO 2016059587 A1 | 21-04-2016 | EP 3206706 A1 | 23-08-2017 |
| | | US 2017239335 A1 | 24-08-2017 |
| | | WO 2016059587 A1 | 21-04-2016 |
| US 2015165045 A1 | 18-06-2015 | DK 2866797 T3 | 03-08-2020 |
| | | EP 2866797 A1 | 06-05-2015 |
| | | ES 2798103 T3 | 09-12-2020 |
| | | HR P20201138 T1 | 30-10-2020 |
| | | HU E051147 T2 | 01-03-2021 |
| | | LT 2866797 T | 25-06-2020 |
| | | PL 2866797 T3 | 02-11-2020 |
| | | PT 2866797 T | 22-07-2020 |
| | | SI 2866797 T1 | 31-08-2020 |
| | | US 2015165045 A1 | 18-06-2015 |
| | | US 2020237920 A1 | 30-07-2020 |
| | | WO 2014005606 A1 | 09-01-2014 |
| WO 2011019911 A1 | 17-02-2011 | AU 2010282483 A1 | 01-03-2012 |
| | | BR 112012003263 A2 | 22-09-2015 |
| | | CA 2770403 A1 | 17-02-2011 |
| | | CN 102573475 A | 11-07-2012 |
| | | CN 105503870 A | 20-04-2016 |
| | | EP 2464230 A1 | 20-06-2012 |
| | | EP 3143996 A1 | 22-03-2017 |
| | | ES 2609640 T3 | 21-04-2017 |
| | | JP 5860399 B2 | 16-02-2016 |
| | | JP 6308991 B2 | 11-04-2018 |
| | | JP 2013501806 A | 17-01-2013 |
| | | JP 2016041734 A | 31-03-2016 |
| | | KR 20120060215 A | 11-06-2012 |
| | | MX 336071 B | 06-01-2016 |
| | | RU 2012109405 A | 20-09-2013 |
| | | RU 2017145628 A | 19-02-2019 |
| | | SG 178403 A1 | 29-03-2012 |
| | | SG 10201407538W A | 29-01-2015 |
| | | TW 201111382 A | 01-04-2011 |
| | | TW 201604195 A | 01-02-2016 |
| | | US 2011038925 A1 | 17-02-2011 |
| | | US 2016024092 A1 | 28-01-2016 |
| | | WO 2011019911 A1 | 17-02-2011 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO.

EP 22 15 6237

This annex lists the patent family members relating to the patent documents cited in the above-mentioned European search report. The members are as contained in the European Patent Office EDP file on
The European Patent Office is in no way liable for these particulars which are merely given for the purpose of information.

11-05-2022

| Patent document cited in search report | Publication date | Patent family member(s) | Publication date |
|---|---|---|---|

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62370453
- US 62412085


### Non-patent literature cited in the description


- **LEAL AD et al.** *Support Care Cancer,* 2014, vol. 22, 1313-1317
- **COHN EJ et al.** *J. Am. Chem. Soc.,* 1946, vol. 68, 459-475
- **LIN JJ et al.** *Pharmaceutical Research,* 2000, vol. 17, 391-6
- **BOSSE D et al.** *J. Clin. Pharmacol.,* 2005, vol. 45, 57-67
- **CHEN Z et al.** *Biochimica etBiophysica Acta,* 2013, vol. 1830, 5515-5525
- **CHEN RF.** *J. Biol. Chem.,* 1967, vol. 242, 173-181
- **TULLIS.** *JAMA,* 1977, vol. 237, 355-360, 460-463
- **HOUSER et al.** *Surgery, Gynecology and Obstetrics,* 1980, vol. 150, 811-816
- **FINLAYSON.** *Seminars in Thrombosis and Hemostasis,* 1980, vol. 6, 85-120
- **GOODMAN et al.** The Pharmacological Basis of Therapeutics. McGraw-Hill, 1996
- **FEHSKE et al.** *Biochem. Pharmcol.,* 1981, vol. 30, 687-92
- **VORUM.** *Dan. Med. Bull.,* 1999, vol. 46, 379-99
- **KRAGH-HANSEN.** *Dan. Med Bull.,* 1990, vol. 1441, 131-40
- **CURRY et al.** *Nat. Struct. Biol.,* 1998, vol. 5, 827-35
- **SUGIO et al.** *Protein. Eng.,* 1999, vol. 12, 439-46
- **HE et al.** *Nature,* 1992, vol. 358, 209-15
- **CARTER et al.** *Adv. Protein. Chem.,* 1994, vol. 45, 153-203
- *CHEMICAL ABSTRACTS,* 172673-20-0
- *CHEMICAL ABSTRACTS,* 265121-04-8
- Remington's Pharmaceutical Sciences. Lippincon Williams & Wilkins, 2003
- *CHEMICAL ABSTRACTS,* 135729-61-2
- *CHEMICAL ABSTRACTS,* 135729-62-3
- **CHEN Z et al.** *Biochimica et Biophysica Acta,* 2013, vol. 1830, 5515-5525
- **FEHSKE et al.** *Biochem. Pharmcol.,* 1981, vol. 30, 687-92
- *CHEMICAL ABSTRACTS,* 170729-80-3